# EUROPÄISCHE PATENTSCHRIFT

(11) **EP 1 987 021 B1**
(45) Veröffentlichungstag und Bekanntmachung des Hinweises auf die Patenterteilung: **22.01.2014**
(21) Anmeldenummer: 07704562.3
(22) Anmeldetag: 13.02.2007
(51) Int. Cl.: C07D 401/12, A61K 31/55, A61P 7/02, C07D 403/12, C07D 401/14

(54) **SUBSTITUIERTE PROLINAMIDE, DEREN HERSTELLUNG UND DEREN VERWENDUNG ALS ARZNEIMITTEL**
SUBSTITUTED PROLINE AMIDES, PREPARATION AND USE AS MEDICAMENTS THEREOF
PROLINE-AMIDES SUBSTITUÉES, LEUR PRÉPARATION ET LEUR UTILISATION COMME MÉDICAMENTS

(30) Priorität: 14.02.2006 EP 06101653
(43) Veröffentlichungstag der Anmeldung: 05.11.2008
(73) Patentinhaber: BOEHRINGER INGELHEIM INTERNATIONAL GMBH, 55216 Ingelheim (DE); Boehringer Ingelheim Pharma GmbH & Co. KG, 55216 Ingelheim (DE)
(72) Erfinder: PRIEPKE, Henning, 88447 Warthausen (DE); DAHMANN, Georg, 88448 Attenweiler (DE); GERLACH, Kai, 88441 Mittelbiberach (DE); NAR, Herbert, 88416 Ochsenhausen (DE); PFAU, Roland, 88400 Biberach (DE); SCHULER-METZ, Annette, 89081 Ulm (DE); WIENEN, Wolfgang, 88400 Biberach (DE)
(74) Vertreter: Simon, Elke Anna Maria
(86) Internationale Anmeldenummer: PCT/EP2007/051390
(87) Internationale Veröffentlichungsnummer: WO 2007/093595

(56) Entgegenhaltungen:
- WO-A-2004/087695
- WO-A-2004/110433
- WO-A-2005/058817
- WO-A-2005/092849

## Beschreibung

Gegenstand der vorliegenden Erfindung sind neue substituierte Prolinamide der allgemeinen Formel (I) 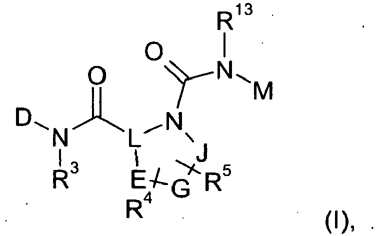 deren Tautomere, deren Enantiomere, deren Diastereomere, deren Gemische und deren Salze, insbesondere deren physiologisch verträgliche Salze mit anorganischen oder organischen Säuren oder Basen, welche wertvolle Eigenschaften aufweisen.

Die Verbindungen der obigen allgemeinen Formel (I) sowie deren Tautomere, deren Enantiomere, deren Diastereomere, deren Gemische und deren Salze, insbesondere deren physiologisch verträgliche Salze mit anorganischen oder organischen Säuren oder Basen, und deren Stereoisomere weisen wertvolle pharmakologische Eigenschaften auf, insbesondere eine antithrombotische Wirkung und eine Faktor Xa-inhibierende Wirkung.

Gegenstand der vorliegenden Anmeldung sind neue Verbindungen der obigen allgemeinen Formel (I), deren Herstellung, die die pharmakologisch wirksamen Verbindungen enthaltenden Arzneimittel, deren Herstellung und Verwendung.

Eine 1. Ausführungsform der vorliegenden Erfindung umfasst diejenigen Verbindungen der allgemeinen Formel (I), in denen
- D: ein substituiertes bicyclisches Ringsystem der Formel (II) 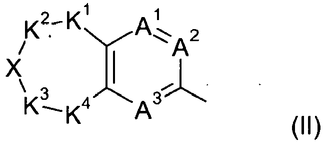 darstellt, in dem
K¹ und K⁴ jeweils unabhängig voneinander eine -CH₂-, -CHR^{7a}-, -CR^{7b}R^{7c}-, -C(=CH2)- oder eine -C(O)-Gruppe bedeuten, und wobei
R^{7a}/R^{7b}/R^{7c} jeweils unabhängig voneinander ein Fluoratom, eine Hydroxy-, C₁₅-Alkyloxy-, Amino-, C₁₋₅-Alkylamino-, Di-(C₁₋₅-alkyl)-amino-, C₃₋₅-Cycloalkylenimino-, C₁₋₅-Alkylcarbonylaminogruppe, eine C₃₋₅-Cycloalkyl- oder eine C₁₋₅-Alkylgruppe, die durch 1-3 Fluoratome substituiert sein kann, eine Hydroxy-C₁₋₅-alkyl-, C₁₋₅-Alkyloxy-C₁₋₅-alkyl-, Amino-C₁₋₅-alkyl-, C₁₋₅-Alkylamino-C₁₋₅-alkyl-, Di-(C₁₋₅-alkyl)-amino-C₁₋₅-alkyl-, C₄₋₇-Cycloalkylenimino-C₁₋₅-alkyl-, Carboxy-C₀₋₅-alkyl-, C₁₋₅-Alkyloxycarbonyl-C₀₋₅-alkyl-, Aminocarbonyl-C₀₋₅-alkyl-, C₁₋₅-Alkylaminocarbonyl-C₀₋₅-alkyl-, Di-(C₁₋₅-alkyl)-aminocarbonyl-C₀₋₅-alkyl- oder eine C₄₋₇-Cycloalkyleniminocarbonyl-C₀₋₅-alkyl-gruppe bedeutet,
wobei nicht gleichzeitig beide Reste R^{7b}/R^{7c} über ein Heteroatom an das Ringkohlenstoffatom gebunden sein können, außer -C(R^{7b}R^{7c})- entspricht einer -CF₂-Gruppe, oder
R^{7a} eine durch Fluor-, Chlor- , Brom-, Methyl-, Methoxy-, Amino-oder Nitro-substituierte Phenyl- oder monocyclische Heteroarylgruppe bedeutet, oder zwei Reste R^{7b}/R^{7c} zusammen mit dem Ringkohlenstoffatom einen 3-, 4-, 5-, 6- oder 7-gliedrigen gesättigten Carbocyclus oder einen Cyclopenten-, Cyclohexen-, Oxetan-, Azetidin-, Thietan-, Tetrahydrofuran-, Pyrrolidin-, Tetrahydrothiophen-, Tetrahydropyran-, Piperidin-, Pentamethylensulfid-, Hexamethylenimin-, 1,3-Dioxolan-, 1,4-Dioxan-, Hexahydropyridazin-, Piperazin-, Thiomorpholin-, Morpholin-, 2-Imidazolidinon-, 2-Oxazolidinon-, Tetrahydro-2(1H)-pyrimidinon-oder [1,3]Oxazinan-2-on-ring bilden können,
wobei dessen Methylengruppen durch 1-2 C₁₋₃-Alkyl- oder CF₃-gruppen substituiert sein können, und/oder dessen Methylengruppen, sofern sie nicht an ein Heteroatom gebunden sind, durch 1-2 Fluoratome substituiert sein können, und/oder bei dem eine -CH₂-Gruppe neben einem N-Atom durch eine -CO-Gruppe ersetzt sein kann, und/oder dessen Iminogruppen jeweils durch eine C₁₋₃-Alkyl- oder C₁₃-Alkylcarbonyl-gruppe substituiert sein können, und/oder
bei dem das Schwefelatom zu einer Sulfoxid oder Sulfongruppe oxidiert sein kann,

K² und K³ jeweils unabhängig voneinander eine -CH₂-, -CHR^{8a}-, -CR^{8b}R^{8c}-oder eine -C(O)-Gruppe bedeuten, wobei
R^{8a}/R^{8b}/R^{8c} jeweils unabhängig voneinander eine C₃₋₅-Cycloalkyl- oder eine C₁₋₅-Alkylgruppe, die durch 1-3 Fluoratome substituiert sein kann, eine Hydroxy-C₁₋₅-alkyl-, C₁₋₅-Alkyloxy-C₁₋₅-alkyl-, Amino-C₁₋₅-alkyl-, C₁₋₅-Alkylamino-C₁₋₅-alkyl-, Di-(C₁₋₅-alkyl)-amino-C₁₋₅-alkyl-, C₄₋₇-Cycloalkylenimino-C₁₋₅-alkyl-, Carboxy-C₀₋₅-alkyl-, C₁₋₅-Alkyloxycarbonyl-C₀₋₅-alkyl-, Aminocarbonyl-C₀₋₅-alkyl-, C₁₋₅-Alkylaminocarbonyl-C₀₋₅-alkyl-, Di-(C₁₋₅-alkyl)-aminocarbonyl-C₀₋₅-alkyl- oder eine C₄₋₇-Cycloalkyleniminocarbonyl-C₀₋₅-alkyl-gruppe bedeutet,
oder zwei Reste R^{8b}/R^{8c} zusammen mit dem Ringkohlenstoffatom einen 3-, 4-, 5-, 6- oder 7-gliedrigen gesättigten Carbocyclus oder einen Cyclopenten-, Cyclohexen-, Oxetan-, Azetidin-, Thietan-, Tetrahydrofuran-, Pyrrolidin-, Tetrahydrothiophen-, Tetrahydropyran-, Piperidin-, Pentamethylensulfid-, Hexamethylenimin-, Hexahydropyridazin-, Tetrahydro-2(1H)-pyrimidinon-, [1,3]Oxazinan-2-on-ring bilden können,
wobei dessen Methylengruppen durch 1-2 C₁₋₃-Alkyl- oder CF₃-gruppen substituiert sein können, und/oder dessen Methylengruppen, sofern sie nicht an ein Heteroatom gebunden sind, durch 1-2 Fluoratome substituiert sein können, und/oder bei dem eine -CH₂-Gruppe neben einem Stickstoffatom durch eine -CO-Gruppe ersetzt sein kann, und/oder dessen Iminogruppen jeweils durch eine C₁₋₃-Alkyl- oder C₁₃-Alkylcarbonyl-gruppe substituiert sein können, und/oder
bei dem das Schwefelatom zu einer Sulfoxid- oder Sulfongruppe oxidiert sein kann,
mit der Massgabe, dass ein durch R^{8b} oder R^{8c} eingebrachtes Heteroatom nicht durch nur ein Kohlenstoffatom von X in Formel (I) entfernt sein darf, und
insgesamt in Formel (II) maximal vier Reste ausgewählt aus R^{7a}, R^{7b}, R^{7c}, R^{8a}, R^{8b} und R^{8c} vorhanden sein dürfen, und
X ein Sauerstoff- oder Schwefelatom, eine CF₂-, Surfen-, Sulfon- oder eine NR¹-Gruppe bedeutet, in der
R¹ ein Wasserstoffatom oder eine Hydroxy-, C₁₋₃-Alkyloxy-, Amino-, C₁₋₃-Alkylamino-, Di-(C₁₋₃-alkyl)-amino-, eine C₁₋₅-Alkyl-, C₂₋₅-Alkenyl-CH₂-, C₂₋₅-Alkinyl-CH₂-, C₃₋₆-Cycloalkyl-, C₄₋₆-Cycloalkenyl-, Oxetan-3-yl-, Tetrahydrofuran-3-yl-, Benzyl-, C₁₋₅-Alkyl-carbonyl-, Trifluormethylcarbonyl-, C₃₋₆-Cycloalkyl-carbonyl-, C₁₋₅-Alkyl-sulfonyl-, C₃₋₆-Cycloalkyl-sulfonyl-, Aminocarbonyl-, C₁₋₅-Alkylaminocarbonyl-, Di-(C₁₋₅-alkyl)-aminocarbonyl-, C₁₋₅-Alkyloxycarbonyl-, C₄₋₇-Cycloalkyleniminocarbonyl-gruppe bedeutet,
wobei die in den voranstehend genannten Gruppen befindlichen Methylen- und Methylgruppen zusätzlich durch eine C₁₋₃Alkyl-, Carboxyl, C₁₋₅-Alkoxycarbonyl-gruppe substituiert sein können, oder durch eine Hydroxy-, C₁₋₅-Alkyloxy-, Amino-, C₁₋₅-Alkylamino-, C₁₅-Dialkylamino- oder C₄₋₇-Cycloalkyleniminogruppe substituiert sein können, sofern die Methylen- oder Methylgruppen nicht direkt an ein Heteroatom aus der Gruppe O, N oder S gebunden sind, und/oder ein bis drei Wasserstoffatome durch Fluoratome ersetzt sein können, sofern die Methylen- oder Methylgruppen nicht direkt an ein Heteroatom aus der Gruppe O, N oder S gebunden sind,
und in dem
- A¹: entweder N oder CR¹⁰ bedeutet,
- A²: entweder N oder CR¹¹ bedeutet,
- A³: entweder N oder CR¹² bedeutet,

wobei R¹⁰, R¹¹ und R¹² jeweils unabhängig voneinander
ein Wasserstoff-, Fluor-, Chlor-, Brom- oder lodatom, oder eine C₁₅-Alkyl-, CF₃-, C₂₋₅ -Alkenyl-, C₂₋₅-Alkinyl-, eine Cyano-, Carboxy-, C₁₋₅-Alkyloxycarbonyl-, Hydroxy-, C₁₋₃-Alkyloxy-, CF₃O-, CHF₂O-, CH₂FO-, Amino-, C₁₋₅-Alkylamino-, Di-(C₁₋₅-Alkyl)-amino- oder C₄₇-Cycloalkylenimino-gruppe bedeuten, oder
- D: eine der vier Gruppen (II-1), (II-2), (II-3), (II-4), (II-5) oder (II-6) darstellt 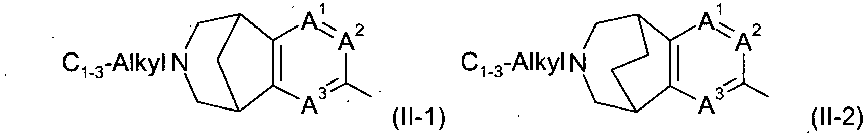 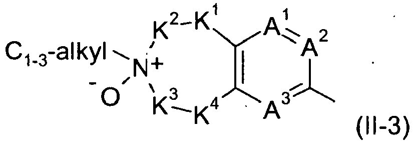 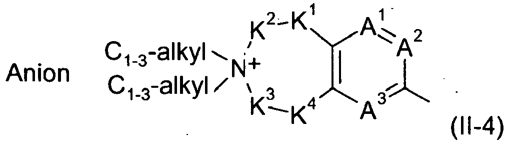 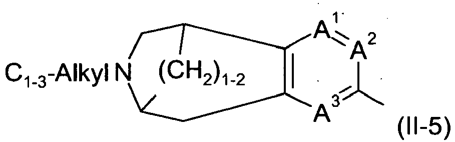 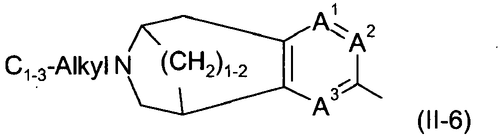 in der die Reste A1, A2, A3, K1, K2, K3, K4 wie oben definiert sind, und das Anion in (II-4) ein Fluorid, Chlorid, Bromid, Iodid, Sulfat, Hydrogensulfat, Phosphat, Hexafluorphosphat, Hydrogenphosphat, Benzoat, Salicylat, Succinat, Citrat oder Tartrat bedeutet,
- R³: ein Wasserstoffatom oder eine C₁₋₃-Alkylgruppe bedeutet, und
- -L-E-G-J-: eine -C-C-C-C- oder -C-C=C-C-Gruppe bedeutet, die durch R⁴ und R⁵ substituiert sein kann, und
- R⁴: ein Wasserstoffatom oder
eine geradkettige oder verzweigte C₁₋₆-Alkyl-, C₂₋₆-Alkenyl- oder C₂₋₆-Alkinylgruppe bedeutet,
wobei die Wasserstoffatome der Methylen- und/oder Methylfragmente der geradkettigen oder verzweigten C₁₋₆-Alkyl-, C₂₋₆-Alkenyl- oder C₂₋₆-Alkinylgruppe gegebenenfalls ganz oder teilweise durch Fluoratome ersetzt sein können, und/oder
wobei die Wasserstoffatome der Methylen- und/oder Methylfragmente der geradkettigen oder verzweigten C₁₋₆-Alkyl-, C₂₋₆-Alkenyl- oder C₂₋₆-Alkinylgruppe gegebenenfalls jeweils unabhängig voneinander durch ein bis zwei Substituenten ausgewählt aus einer C₃₋₅-Cycloalkylgruppe, einer Nitril-, Hydroxy- oder C₁₋₅-Alkyloxygruppe, wobei die Wasserstoffatome der C₁₋₅-Alkyloxygruppe gegebenenfalls ganz oder teilweise durch Fluoratome ersetzt sein können, einer Allyloxy-, Propargyloxy-, Benzyloxy-, C₁₋₅-Alkylcarbonyloxy-, C₁₋₅-Alkyloxycarbonyloxy-, Carboxy-C₁₋₅-alkyloxy-, C₁₋₅-Alkyloxycarbonyl-C₁₋₅-alkyloxy-, Mercapto-, C₁₋₅-Alkylsulfanyl-, C₁₋₅-Alkylsulfinyl-, C₁₋₅-Alkylsulfonyl-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl-, C₄₋₇-Cycloalkyleniminocarbonyl-, Aminosulfonyl-, C₁₋₅-Alkylaminosulfonyl-, Di-(C₁₋₅-alkyl)-aminosulfonyl-, C₄₋₇-Cycloalkyleniminosulfonyl-, Amino-, C₁₋₅-Alkylamino-, Di-(C₁₋₅-alkyl)-amino-, C₁₋₅-Alkylcarbonylamino-, C₁₋₅-Alkyl-sulfonylamino-, *N*-(C₁₋₅-Alkylsulfonyl)-C₁₋₅-alkylamino-, C₃₋₆-Cycloalkylcarbonylaminogruppe, oder einer Morpholinyl-, Thiomorpholinyl-, Pyrrolidinyl-, Piperidinyl-, Piperazinyl-, Tetrahydrofuranyl-, Tetrahydropyranyl-gruppe substituiert sein können, wobei die vorgenannten Carbo- und Hetero-cyclen im Ring jeweils durch 1-4 C₁₋₃-Alkyl- oder C₁₋₃-Alkylcarbonylgruppen oder jeweils durch 1-2 Oxogruppen substituiert sein können, und/oder
wobei die Wasserstoffatome der sp²-hybridisierten Kohlenstoffatome der geradkettigen oder verzweigten C₂₋₆-Alkenyl-gruppe gegebenenfalls ganz oder teilweise durch Fluoratome ersetzt sein können, oder
eine Nitril-, Carboxy-, Aminocarbonyl-, C₁₋₅-Alkylaminocarbonyl-, C₃₋₆-Cycloalkylaminocarbonyl-, Di-(C₁₋₅-alkyl)-aminocarbonyl-, C₁₋₅-Alkyloxycarbonyl- oder eine C₄₋₇-Cycloalkyleniminocarbonylgruppe in der gegebenenfalls eine Methylengruppe durch ein Sauerstoff-, Schwefel- oder C₀₋₃-Alkyl-substituiertes Stickstoffatom ersetzt sein kann, oder
eine Phenyl-, mono- oder bicyclische Heteroaryl-, Phenyl-C₁₋₅-alkyl- oder mono- oder bicyclische Heteroaryl-C₁₋₅-alkylgruppe,
die im Phenyl- oder Heteroarylteil gegebenenfalls ein- bis dreifach durch gleiche oder unterschiedliche Substituenten ausgewählt aus der Gruppe bestehend aus Fluor-, Chlor-, Brom- und Iodatomen, und C₁₋₅-Alkyl-, Trifluormethyl-, Amino-, C₁₋₅-alkyl-amino-, Di-(C₁₋₅-alkyl)-amino-, Hydroxy-, C₁₋₅-Alkyloxy-, Mono-, Di- oder Trifluormethoxy-, Carboxy- und C₁₋₅-Alkyloxycarbonylgruppe, substituiert sein kann,
bedeutet, und
wenn -L-E-G-J- eine -C-C-C-C-Gruppe bedeutet, kann R⁴ an E oder G auch ein Fluoratom oder eine Hydroxy-, C₂₋₅-Alenyloxy-, C₂₋₅-Alinyl-oxy-, C₁₋₅-Alkyl-oxy-, C₃₋₆-Cycloalkyl-oxy-, C₁₋₅-Alkylaminocarbonyloxy-, Di(C₁₋₅-alkyl)aminocarbonyloxy- oder C₄₋₇-Cycloalkyleniminocarbonyloxy-, Phenyl-C₀₋₃-alkyloxy-, Heteroaryl-C₀₋₃-alkyl-oxy-, Amino-, C₁₋₅-Alkylamino-, Di-(C₁₋₅-alkyl)-amino-, C₄₋₇-Cycloalkylenimino-, C₁₋₃-Acylamino-, (C₁₋₃-Acyl)C₁₋₃-alkylamino-, C₁₋₅-Alkyloxycarbonylamino-, C₁₅-Alkylaminocarbonylamino-, Di(C₁₋₅-alkyl)aminocarbonylamino- oder eine C₄₋₇-Cycloalkyleniminocarbonylamino-gruppe darstellen,
wobei die in den vorgenannten Alkyl- oder Cycloalkylresten vorhandenen Methyl oder Methylengruppen jeweils unabhängig voneinander durch einen Substituenten ausgewählt aus der Gruppe Morpholinyl-, Thiomorpholinyl-, Pyrrolidinyl-, Piperidinyl-, Piperazinyl-, Tetrahydrofuranyl-, Tetrahydropyranyl, Dimethylaminocarbonyl-, C₁₋₃Alkyloxycarbonyl-, Carboxy-, Methyl-, Hydroxy-, Methoxy- oder Amino- substituiert sein können,
   mit der Maßgabe, dass zwei Heteroatome aus der Gruppe Sauerstoff und Stickstoff durch genau eine gegebenenfalls substituierte -CH₂-Gruppe voneinander getrennt sind, und/oder
   dass zwei Atome eine -O-O- oder -S-O-Bindung bilden, ausgeschlossen ist, und
- R⁵: ein Wasserstoffatom oder eine C₁₋₅ Alkyl-, C₂₋₅ Alkenyl- oder C₂₋₅ Alkinylgruppe bedeutet, oder sofern R⁵ mit E oder G verknüpft ist auch eine Hydroxy- oder Methoxy-gruppe darstellen kann, oder
- R⁴ und R⁵: sofern sie an das selbe Kohlenstoffatom gebunden sind, eine-C(O)- Gruppe,oder eine -C(F₂)- Gruppe bilden können, oder
- R⁴ und R⁵: sofern sie an das selbe Kohlenstoffatom oder an zwei benachbarte Kohlenstoffatome gebunden sind,
eine C₃₋₇-Cycloalkyl- oder C₅₋₇-Cycloalkenylgruppe bilden können,
wobei eine der Methylengruppen dieser C₄₋₇-Cycloalkylgruppe durch ein Sauerstoff- oder Schwefelatom oder eine -NH-, -N(C₁₋₅-Alkyl)-, -N(C₁₋₄-Alkylcarbonyl)- oder eine Carbonyl-, Sulfinyl- oder Sulfonylgruppe ersetzt sein kann, und/oder
wobei zwei direkt benachbarte Methylengruppen dieser -C₄₋₇-Cycloalkylgruppe zusammen durch eine -C(O)NH-, - C(O)N(C₁₋₅-Alkyl)-, -S(O)₂NH-, oder -S(O)₂N(C₁₋₅-Alkyl)-Gruppe ersetzt sein können, und/oder
wobei 1 bis 3 Kohlenstoffatome einer C₃₋₇-Cycloalkylgruppe gegebenenfalls unabhängig voneinander durch jeweils ein oder zwei Fluoratome oder eine oder zwei C₁₋₅-Alkyl-gruppen oder eine Hydroxy-, C₁₋₅-Alkyloxy-, C₁₋₅-Alkylcarbonyloxy-, Amino-, C₁₋₅-Alkylamino-, Di-(C₁₋₅-alkyl)-amino-, C₄₋₇-Cycloalkylenimino-, C₁₋₅-Alkylcarbonylamino-, C₃₋₆-Cycloalkylcarbonylamino-, Nitril-, Carboxy-C₁₋₅-alkyl-, C₁₋₅-Alkyloxycarbonyl-C₁₋₅-alkyl-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl- oder C₄₋₇-Cycloalkyleniminocarbonylgruppe substituiert sein können,
mit der Maßgabe, dass eine solche, zusammen aus R⁴ und R⁵ gebildete C₃₋₇-Cycloalkylgruppe,
in der zwei Heteroatome im Cyclus aus der Gruppe Sauerstoff und Stickstoff durch genau eine gegebenenfalls substituierte -CH₂-Gruppe voneinander getrennt sind, und/oder
in der zwei Atome im Ring eine -O-O- oder -S-O-Bindung bilden,
ausgeschlossen ist,
- R¹³: ein Wasserstoffatom oder eine C₁₋₅Alkylgruppe bedeutet,
- M: einen gegebenenfalls durch R² und R⁶ substituierten Phenyl-, Thienyl- oder Pyridylring bedeutet, in dem
R² ein Fluor-, Chlor-, Brom- oder Jod-atom oder eine Methyl-, Ethyl-, Propyl-, Isopropyl-, Vinyl-, Methoxy-, Ethinyl-, Cyano- oder - C(O)NH₂-Gruppe darstellt, und
R⁶ ein Wasserstoff-, Fluor-, Chlor-, Brom- oder Jod-atom oder eine Hydroxy-, Methoxy-, Trifluormethoxy-, eine gegebenenfalls durch Fluoratome substituierte C₁₋₃-Alkyl-, Cyano-, Amino-, oder NH₂C(O)-Gruppe darstellt,
wobei, soweit nichts anderes erwähnt wurde, unter dem voranstehend in den Definitionen erwähnten Ausdruck "Heteroarylgruppe" eine monocyclische 5- oder 6-gliedrige Heteroarylgruppe zu verstehen ist, wobei
die 6-gliedrige Heteroarylgruppe ein, zwei oder drei Stickstoffatome, und
die 5-gliedrige Heteroarylgruppe eine gegebenenfalls durch eine C₁₋₃-Alkylgruppe substituierte Iminogruppe, ein Sauerstoff- oder Schwefelatom, oder
   eine gegebenenfalls durch eine C₁₋₃-Alkylgruppe substituierte Iminogruppe oder ein Sauerstoff- oder Schwefelatom und zusätzlich ein oder zwei Stickstoffatome, oder
   eine gegebenenfalls durch eine C₁₋₃-Alkylgruppe substituierte Iminogruppe und drei Stickstoffatome,
   enthält,
   und außerdem an die vorstehend erwähnten monocyclischen Heteroarylgruppen über zwei benachbarte Kohlenstoffatome ein gegebenenfalls durch ein Fluor-, Chlor- oder Bromatom, eine C₁₋₃-Alkyl-, Hydroxy-, C₁₋₃-Alkyloxygruppe, Amino-, C₁₋₃-Alkylamino-, Di-(C₁₋₃-alkyl)-amino- oder C₃₋₆-Cycloakyleniminogruppe substituierter Phenylring ankondensiert sein kann,
   und die Bindung über ein Stickstoffatom oder über ein Kohlenstoffatom des heterocyclischen Teils oder eines ankondensierten Phenylrings erfolgt,
und wobei, soweit nichts anderes erwähnt wurde, unter dem voranstehend in den Definitionen erwähnten Ausdruck "Halogenatom" ein Atom aus der Gruppe Fluor, Chlor, Brom und Iod zu verstehen ist,
und wobei die in den voranstehend erwähnten Definitionen enthaltenen Alkyl-, Alkenyl-, Alkinyl- und Alkyloxygruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen, sofern nichts anderes erwähnt, ganz oder teilweise durch Fluoratome ersetzt sein können,
deren Tautomere, deren Enantiomere, deren Diastereomere, deren Gemische und deren Salze.

Beispiele für monocyclische Heteroarylgruppen sind die Pyridyl-, *N*-Oxy-pyridyl-, Pyrazolyl-, Pyridazinyl-, Pyrimidinyl-, Pyrazinyl-, [1,2,3]Triazinyl-, [1,3,5]Triazinyl-, [1,2,4]Triazinyl-, Pyrrolyl-, Imidazolyl-, [1,2,4]Triazolyl-, [1,2,3]Triazolyl-, Tetrazolyl-, Furanyl-, Isoxazolyl-, Oxazolyl-, [1,2,3]Oxadiazolyl-, [1,2,4]Oxadiazolyl-, Furazanyl-, Thienyl-, Thiazolyl-, Isothiazolyl-, [1,2,3]Thiadiazolyl-, [1,2,4]Thiadiazolyl- oder [1,2,5]Thiadiazolyl-Gruppe.

Beispiele für bicyclische Heteroarylgruppen sind die Benzimidazolyl, Benzofuranyl-, Benzo[*c*]furanyl-, Benzothiophenyl-, Benzo[*c*]thiophenyl-, Benzothiazolyl-, Benzo[*c*]isothiazolyl-, Benzo[*d*]isothiazolyl-, Benzooxazolyl-, Benzo[*c*]isoxazolyl-, Benzo[*d*]isoxazolyl-, Benzo[1,2,5]oxadiazolyl-, Benzo[1,2,5]thiadiazolyl-, Benzo[1,2,3]thiadiazolyl-, Benzo[*d*][1,2,3]triazinyl-, Benzo[1,2,4]thazinyl-, Benzotriazolyl-, Cinnolinyl-, Chinolinyl-, *N*-Oxy-chinolinyl-, Isochinolinyl-, Chinazolinyl-, *N*-Oxy-chinazolinyl-, Chinoxalinyl-, Phthalazinyl-, Indolyl-, Isoindolyl- oder 1-Oxa-2,3-diaza-indenyl-Gruppe.

Beispiele für die voranstehend in den Definitionen erwähnten C₁₋₆-Alkylgruppen sind die Methyl-, Ethyl-, 1-Propyl-, 2-Propyl-, *n*-Butyl-, *sec*-Butyl-, *tert*-Butyl-, 1-Pentyl-, 2-Pentyl-, 3-Pentyl-, *neo*-Pentyl-, 3-Methyl-2-butyl-, 1-Hexyl-, 2-Hexyl-, 3-Hexyl, 3-Methyl-2-pentyl-, 4-Methyl-2-pentyl-, 3-Methyl-3-pentyl-, 2-Methyl-3-pentyl-, 2,2-Dimethyl-3-butyl- oder 2,3-Dimethyl-2-butyl-Gruppe.

Beispiele für die voranstehend in den Definitionen erwähnten C₁₋₅-Alkyloxygruppen sind die Methyloxy-, Ethyloxy-, 1-Propyloxy-, 2-Propyloxy-, *n*-Butyloxy-, *sec*-Butyloxy-, *tert*-Butyloxy-, 1-Pentyloxy-, 2-Pentyloxy-, 3-Pentyloxy- oder *neo*-Pentyloxy-Gruppe.

Beispiele für die voranstehend in den Definitionen erwähnten C₂₋₅-Alkenylgruppen sind die Ethenyl-, 1-Propen-1-yl-, 2-Propen-1-yl-, 1-Buten-1-yl-, 2-Buten-1-yl-, 3-Buten-1-yl-, 1-Penten-1-yl-, 2-Penten-1-yl-, 3-Penten-1-yl-, 4-Penten-1-yl-, 1-Hexen-1-yl-, 2-Hexen-1-yl-, 3-Hexen-1-yl-, 4-Hexen-1-yl-, 5-Hexen-1-yl-, But-1-en-2-yl-, But-2-en-2-yl-, But-1-en-3-yl-, 2-Methyl-prop-2-en-1-yl-, Pent-1-en-2-yl-, Pent-2-en-2-yl-, Pent-3-en-2-yl-, Pent-4-en-2-yl-, Pent-1-en-3-yl-, Pent-2-en-3-yl-, 2-Methyl-but-1-en-1-yl-, 2-Methyl-but-2-en-1-yl-, 2-Methyl-but-3-en-1-yl- oder 2-Ethyl-prop-2-en-1-yl -Gruppe,

Beispiele für die voranstehend in den Definitionen erwähnten C₂₋₅-Alkinylgruppen sind die Ethinyl-, 1-Propinyl-, 2-Propinyl-, 1-Butin-1-yl-, 1-Butin-3-yl-, 2-Butin-1-yl-, 3-Butin-1-yl-, 1-Pentin-1-yl-, 1-Pentin-3-yl-, 1-Pentin-4-yl-, 2-Pentin-1-yl-, 2-Pentin-3-yl-, 3-Pentin-1-yl-, 4-Pentin-1-yl-, 2-Methyl-1-butin-4-yl-, 3-Methyl-1-butin-1-yl- oder 3-Methyl-1-butin-3-yl-Gruppe.

Eine 2. Ausführungsform der vorliegenden Erfindung umfasst diejenigen Verbindungen der allgemeinen Formel (I), in denen E, G, J, L, M, R³-R⁵ und R¹³ wie in Ausführungsform 1 beschrieben definiert sind und
- D: ein substituiertes bicyclisches Ringsystem der Formel (II) 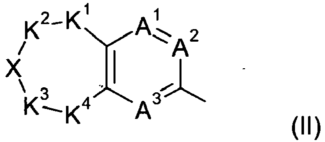 darstellt, in dem
K¹ und K⁴ jeweils unabhängig voneinander eine -CH₂-, -CHR^{7a}-, -CR^{7b}R^{7c}-, -C(=CH2)- oder eine -C(O)-Gruppe bedeuten, und wobei
R^{7a}/R^{7b}/R^{7c} jeweils unabhängig voneinander ein Fluoratom, eine Hydroxy-, C₁₅-Alkyloxy-, eine C₁₋₅-Alkylgruppe bedeutet,
wobei nicht gleichzeitig beide Reste R^{7b}/R^{7c} über ein Heteroatom an das Ringkohlenstoffatom gebunden sein können, außer - C(R^{7b}R^{7c})- entspricht einer -CF₂-Gruppe,
oder zwei Reste R^{7b}/R^{7c} zusammen mit dem Ringkohlenstoffatom einen Cyclopropylring bilden können,
K² und K³ jeweils unabhängig voneinander eine -CH₂-, -CHR^{8a}-, -CR^{8b}R^{8c}- oder eine -C(O)- Gruppe bedeuten, wobei

R^{8a}/R^{8b}/R^{8c}
jeweils unabhängig voneinander eine C₁₋₅-Alkylgruppe bedeutet, oder zwei Reste R^{8b}/R^{8c} zusammen mit dem Ringkohlenstoffatom einen Cyclopropylring bilden können, und
insgesamt in Formel (II) maximal vier Reste ausgewählt aus R^{7a}, R^{7b}, R^{7c}, R^{8a}, R^{8b} und R^{8c} vorhanden sein dürfen, und
X ein Sauerstoff- oder Schwefelatom, eine Sulfen-, Sulfon- oder eine NR¹-Gruppe bedeutet, in der
R¹ ein Wasserstoffatom oder eine Hydroxy-, C₁₋₃-Alkyloxy-, Amino-, C₁₋₃-Alkylamino-, Di-(C₁₋₃-alkyl)-amino-, eine C₁₋₅-Alkyl-, C₂₋₅-Alkenyl-CH₂-, C₂₋₅-Alkinyl-CH₂- oder eine C₃₋₆-Cycloalkylgruppe bedeutet, und in dem
A¹ entweder N oder CR¹⁰ bedeutet,
A² entweder N oder CR¹¹ bedeutet,
A³ entweder N oder CR¹² bedeutet, wobei R¹⁰, R¹¹ und R¹² jeweils unabhängig voneinander
ein Wasserstoff-, Fluor-, Chlor-, Brom- oder Iodatom, oder eine C₁₅-Alkyl-, CF₃-, eine Cyano-, Carboxy-, C₁₋₅-Alkyloxycarbonyl-, Hydroxy-, C₁₋₃-Alkyloxy-, CF₃O-, CHF₂O-, CH₂FO-, Amino-, C₁₋₅-Alkylamino-, Di-(C₁₋₅-Alkyl)-amino- oder C₄₋₇-Cycloalkylenimino-gruppe bedeuten.

Eine 3. Ausführungsform der vorliegenden Erfindung umfasst diejenigen Verbindungen der Ausführungsformen 1 oder 2, in denen
- X: eine NR¹-Gruppe bedeutet, in der
R¹ ein Wasserstoffatom oder eine C₁₋₅-Alkyl-, Allyl- oder Cyclopropylgruppe bedeutet, und
- A¹: CR¹⁰ bedeutet,
- A²: CR¹¹ bedeutet,
- A³: CR¹² bedeutet,
wobei R¹⁰, R¹¹ und R¹² jeweils unabhängig voneinander
ein Wasserstoff-, Fluor- oder Chloratom, oder eine Methyl-, CF₃-, Hydroxy-, Methoxy-, CF₃O-, CHF₂O-, CH₂FO-Gruppe bedeuten.

Eine 4. Ausführungsform der vorliegenden Erfindung umfasst diejenigen Verbindungen der allgemeinen Formel (I) in denen D, E, G, J, L, M, R³ und R¹³ wie in Ausführungsform 1, 2 oder 3 beschrieben definiert sind, und in der
- R⁴: ein Wasserstoffatom oder
eine geradkettige oder verzweigte C₁₋₆-Alkylgruppe bedeutet,
wobei die Wasserstoffatome der Methylen- und/oder Methylfragmente der geradkettigen oder verzweigten C₁₋₆-Alkylgruppe gegebenenfalls ganz oder teilweise durch Fluoratome ersetzt sein können, und/oder
wobei die Wasserstoffatome der Methylen- und/oder Methylfragmente der geradkettigen oder verzweigten C₁₋₆-Alkylgruppe gegebenenfalls jeweils unabhängig voneinander durch einen Substituenten ausgewählt aus einer Hydroxy-, C₁₋₅-Alkyloxy-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl-, C₄₋₇-Cycloalkyleniminocarbonyl-, Amino-, C₁₋₅-Alkylamino-, Di-(C₁₋₅-alkyl)-amino-, C₁₋₅-Alkylcarbonylamino-, C₁₋₅-Alkyl-sulfonylamino-, *N*-(C₁₋₅-Alkylsulfonyl)-C₁₋₅-alkylamino-, C₃₋₆-Cycloalkylcarbonylaminogruppe substituiert sein können, oder
eine Nitril-, Carboxy-, Aminocarbonyl-, C₁₋₅-Alkylaminocarbonyl-, C₃₋₆-Cycloalkylaminocarbonyl-, Di-(C₁₋₅-alkyl)-aminocarbonyl-, C₁₋₅-Alkyloxycarbonyl- oder eine C₄₋₇-Cycloalkyleniminocarbonylgruppe in der gegebenenfalls eine Methylengruppe.durch ein Sauerstoff-, Schwefel- oder C₀₋₃-Alkyl-substituiertes Stickstoffatom ersetzt sein kann, bedeutet, und
wenn -L-E-G-J- eine -C-C-C-C-Gruppe bedeutet, kann R⁴ an E oder G auch ein Fluoratom oder eine Hydroxy-, C₂₋₅-Alkenyl-oxy-, C₂₋₅-Alkinyl-oxy-, C₁₋₅-Alkyl-oxy-, C₃₋₆-Cycloalkyl-oxy-, C₁₋₅-Alkylaminocarbonyloxy-, Di(C₁₋₅-alkyl)aminocarbonyloxy- oder C₄₋₇-Cycloalkyleniminocarbonyloxy-, Phenyl-C₀₋₃-alkyloxy-, Amino-, C₁₋₅-Alkylamino-, Di-(C₁₋₅-alkyl)-amino-, C₄₋₇-Cycloalkylenimino-, C₁₋₃-Acylamino-, (C₁₋₃-Acyl)C₁₋₃-alkylamino-, C₁₅-Alkyloxycarbonylamino-, C₁₋₅-Alkylaminocarbonylamino-, Di(C₁₋₅-alkyl)aminocarbonylamino- oder eine C₄₋₇-Cycloalkyleniminocarbonylamino-gruppe darstellen,
wobei die in den vorgenannten Alkyl- oder Cycloalkylresten vorhandenen Methyl oder Methylengruppen jeweils unabhängig voneinander durch einen Substituenten ausgewählt aus der Gruppe Dimethylaminocarbonyl-, C₁₋₃Alkyloxycarbonyl-, Carboxy-, Methyl-, Hydroxy-, Methoxy- oder Amino- substituiert sein können,
   mit der Maßgabe, dass zwei Heteroatome aus der Gruppe Sauerstoff und Stickstoff durch genau eine gegebenenfalls substituierte -CH₂-Gruppe voneinander getrennt sind, und/oder
   dass zwei Atome eine -O-O- oder -S-O-Bindung bilden, ausgeschlossen ist, und
- R⁵: ein Wasserstoffatom, eine Allyl- oder eine C₁₋₅ Alkylgruppe bedeutet, oder sofern R⁵ mit E oder G verknüpft ist auch eine Hydroxy- oder Methoxy-gruppe darstellen kann oder
- R⁴ und R⁵: sofern sie an das selbe Kohlenstoffatom gebunden sind,
eine -C(O)- Gruppe,oder eine -C(F₂)- Gruppe bilden können, oder
- R⁴ und R⁵: sofern sie an das selbe Kohlenstoffatom oder an zwei benachbarte Kohlenstoffatome gebunden sind,
eine C₃₋₇-Cycloalkyl-gruppe bilden können,
wobei eine der Methylengruppen dieser C₄₋₇-Cycloalkylgruppe durch ein Sauerstoff- oder Schwefelatom oder eine -NH-, -N(C₁₋₅-Alkyl)-, -N(C₁₋₄-Alkylcarbonyl)- oder eine Carbonyl-, Sulfinyl- oder Sulfonylgruppe ersetzt sein kann, und/oder
wobei zwei direkt benachbarte Methylengruppen dieser C₄₋₇-Cycloalkylgruppe zusammen durch eine -C(O)NH-,-C(O)N(C₁₋₅-Alkyl)-, -S(O)₂NH-, oder -S(O)₂N(C₁₋₅-Alkyl)-Gruppe ersetzt sein können.

Eine 5. Ausführungsform der vorliegenden Erfindung umfasst diejenigen Verbindungen der Ausführungsformen 1, 2, 3 oder 4, in denen
- -L-E-G-J-: eine -C-C-C-C-Gruppe bedeutet, die durch R⁴ und R⁵, die wie oben in den Ausführungsformen 1, 2, 3 oder 4 definiert sind, substituiert sein kann.

Eine 6. Ausführungsform der vorliegenden Erfindung umfasst diejenigen Verbindungen der Ausführungsformen 1, 2, 3, 4 oder 5, in denen
- D: einen substituierten Benzazepinylrest der Formel (IIa) 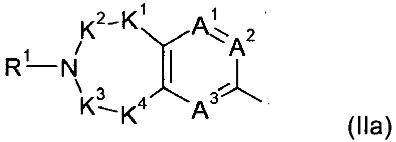 darstellt, in dem
K¹ und K⁴ jeweils unabhängig voneinander eine -CH₂-, -CHR^{7a}-, -CR^{7b}R^{7c}- oder eine -C(O)-Gruppe bedeutet, wobei
R^{7a} eine C₁₋₅-Alkyl-, Hydroxy- oder C₁₋₃-alkyloxy-gruppe bedeutet und
R^{7b}/R^{7c} jeweils unabhängig voneinander eine Hydroxy-, C₁₋₅-Alkyloxy- oder eine C₁₋₅-Alkylgruppe bedeutet, wobei nicht gleichzeitig beide Reste R^{7b}/R^{7c} über ein Sauerstoffatom an das Ringkohlenstoffatom gebunden sein können,
oder zwei Reste R^{7b}/R^{7c} zusammen mit dem Ringkohlenstoffatom einen Cyclopropylring bilden können, und
K² und K³ jeweils unabhängig voneinander eine -CH₂-, -CHR^{8a}- oder - CR^{8b}R^{ac}-Gruppe bedeuten, wobei
R^{8a}/R^{8b}/R^{8c} jeweils unabhängig voneinander eine C₁₋₃-Alkylgruppe bedeutet, und insgesamt in Formel (II) maximal vier Reste ausgewählt aus R^{7a}, R^{7b}, R^{7c}, R^{8a}, R^{8b} und R^{8c} vorhanden sein dürfen, und
R¹ ein Wasserstoffatom oder eine C₁₋₅-Alkyl-, Allyl- oder Cyclopropylgruppe bedeutet, und in denen
A¹ CR¹⁰ bedeutet,
A² CR¹¹ bedeutet,
A³ CR¹² bedeutet,
wobei R¹⁰, R¹¹ und R¹² jeweils unabhängig voneinander
ein Wasserstoff-, Fluor- oder Chloratom, oder eine Methyl-, CF₃-, Hydroxy-, Methoxy-, CF₃O-, CHF₂O-, CH₂FO-Gruppe bedeuten, und
- -L-E-G-J-: eine -C-C-C-C-Gruppe bedeutet, die durch R⁴ und R⁵ substituiert sein kann, und
- R³: ein Wasserstoffatom bedeutet, und
- R⁴: ein Wasserstoffatom oder
eine geradkettige oder verzweigte C₁₋₃-Alkylgruppe bedeutet,
wobei die Wasserstoffatome der Methylen- und/oder Methylfragmente der geradkettigen oder verzweigten C₁₋₆-Alkylgruppe gegebenenfalls unabhängig voneinander durch einen Substituenten ausgewählt aus einer Hydroxy-, C₁₋₅-Alkyloxy-, Carboxy-, C₁₋₅-Alkyloxycarbonyl-gruppe substituiert sein können, oder
eine CF3-, Nitril-, Carboxy-, Aminocarbonyl-, C₁₋₅-Alkylaminocarbonyl-, C₃₋₆-Cycloalkylaminocarbonyl-, Di-(C₁₋₅-alkyl)-aminocarbonyl-, C₁₋₅-Alkyloxycarbonyl- oder eine C₄₋₇-Cyloalkyleniminocarbonylgruppe bedeutet, oder
wenn R⁴ an E oder G angebunden ist auch ein Fluoratom oder eine Hydroxy-, Methoxy-, C₂₋₅-Alkenyl-oxy-, C₂₋₅-Alkyl-oxy-, Methoxyethoxy-, HOCH₂CH(OH)CH₂oxy-, C₃₋₆-Cycloalkyl-oxy-, C₁₋₅-Alkylaminocarbonyloxy-, Di(C₁₋₅-alkyl)aminocarbonyloxy- oder C₄₋₇-Cycloalkyleniminocarbonyloxy-gruppe darstellen kann,
- R⁵: ein Wasserstoffatom oder eine C₁₋₅ Alkylgruppe bedeutet, oder sofern R⁵ mit E oder G verknüpft ist auch eine Hydroxy- oder Methoxy-gruppe darstellen kann, oder
- R⁴ und R⁵: sofern sie an das selbe Kohlenstoffatom gebunden sind, eine C=O- oder eine -CF₂-Gruppe bedeuten können, und
- R¹³: ein Wasserstoffatom bedeutet,
- M: einen
substituierten Phenylring 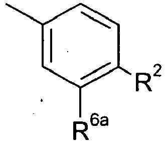 oder einen substituierten Pyridylring 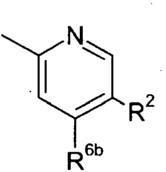
bedeutet, in dem
- R²: ein Fluor-, Chlor-, Bromatom, eine Methoxy- oder Ethinyl-Gruppe darstellt, und
- R^{6a}: ein Wasserstoff- oder Fluoratom darstellt und
- R^{6b}: ein Wasserstoffatom darstellt.

Erfindungsgemäß erhält man die Verbindungen der allgemeinen Formel (I) nach an sich bekannten Verfahren, beispielsweise nach folgenden Verfahren:
(a) Die Herstellung einer Verbindung der allgemeinen Formel (III) 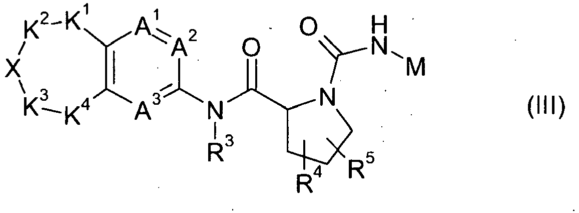 in der A¹ bis A³, K¹ bis K⁴, M und R¹ bis R⁶ wie in Ausführungsform 1 erwähnt definiert sind,
   und die gegebenenfalls an vorhandenen Amino-, Hydroxy-, Carboxy- oder Thiolgruppen durch gängige Schutzgruppen wie beispielsweise denen in T.W. Greene, P.G.M. Wuts in "Protective Groups in Organic Synthesis", Wiley, 1991 und 1999, beschriebenen geschützt sein kann, und deren Schutzgruppen in Literatur nach bekannter Methode abgespalten werden können,
   wird in den Ausführungsbeispielen beschrieben oder kann beispielsweise nach einem der folgenden Formelschemata 1 und 2 oder in Analogie zu den Syntheseverfahren die in WO2004/87695, WO2004/87646 oder in WO2003/45912 beschrieben sind durchgeführt werden. 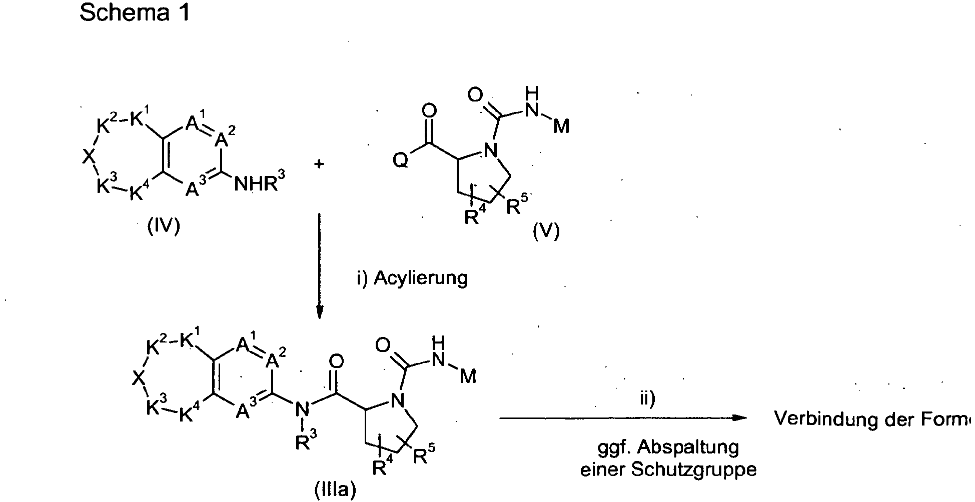 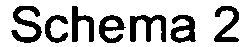 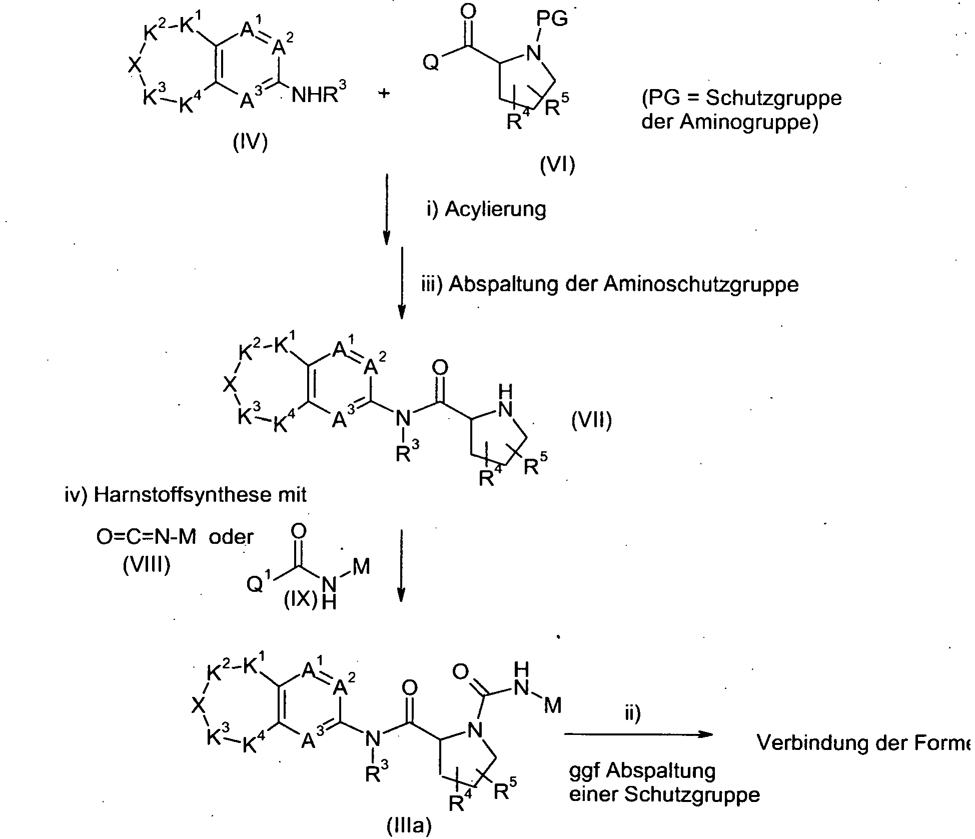 wobei
   Q/Q¹ eine Austrittsgruppe oder eine *in-situ* in eine Austrittsgruppe überführbare Gruppe wie beispielsweise ein Halogenatom, eine Hydroxy-, C₁₋₄-Alkyloxy-, Alkyloxycarbonyloxy-, 4-Nitrophenyloxy-, eine Trichlormethyl- oder Acyloxy-gruppe darstellt, und
   PG eine in Literatur bekannte Schutzgruppe der Aminofunktion wie beispielsweise eine tert.-Butoxycarbonyl-, Benzyloxycarbonyl- oder eine Trifluoracetyl-gruppe darstellt.

   Die in Schema 1 und 2 beschriebenen Reaktionsstufen i) -iv) können auf die in den Beispielen beschriebene Weise oder nach in Literatur bekannter Bedingungen beispielsweise wie folgt durchgeführt werden:
   i) Acylierung eines Amins (IV) mit einer gegebenenfalls aktivierten Carbonsäure (V) oder (VI) :
      Die Acylierung wird zweckmäßigerweise mit einem entsprechenden Halogenid oder Anhydrid in einem Lösungsmittel wie Methylenchlorid, Chloroform, Tetrachlorkohlenstoff, Ether, Tetrahydrofuran, Dioxan, Benzol, Toluol, Acetonitril, Dimethylformamid, Dimethylsulfoxid, Natronlauge oder Sulfolan gegebenenfalls in Gegenwart einer anorganischen oder organischen Base bei Temperaturen zwischen -20 und 200°C, vorzugsweise jedoch bei Temperaturen zwischen -10 und 100°C, durchgeführt.
      Die Acylierung kann jedoch auch mit der freien Säure gegebenenfalls in Gegenwart eines die Säure aktivierenden Mittels oder eines wasserentziehenden Mittels, beispielsweise in Gegenwart von Ethyl-1-ethoxy-1,2-dihydrochinolin-1-carboxylat, Chlorameisensäureisobutylester, Thionylchlorid, Trimethylchlorsilan, Chlorwasserstoff, Schwefelsäure, Methansulfonsäure, *p*-Toluolsulfonsäure, Phosphortrichlorid, Phosphorpentoxid, *N,N*'-Dicyclohexylcarbodiimid, *N,N*'-Dicyclohexylcarbodiimid/Camphersulfonsäure, *N,N*'-Dicyclohexyl-carbodiimid/*N*-Hydroxysuccinimid oder 1-Hydroxy-benztriazol, *N,N*'-Carbonyldiimidazol, O-(Benzotriazol-1-yl)-*N,N,N',N'*-tetramethyl-uroniumtetrafluorborat/*N*-Methylmorpholin, O-(Benzotriazol-1-yl)-*N,N,N',N*'-tetramethyl-uroniumtetrafluorborat/*N-*Ethyldiisopropylamin, *O*-Pentafluorophenyl-*N,N,N',N*'-tetramethyluronium-hexafluorophosphat/Triethylamin, *N,N'*-Thionyldiimidazol oder Triphenylphosphin/Tetrachlorkohlenstoff, gegebenenfalls unter Zusatz einer Hilfsbase wie Natronlauge, Cäsium-, Kalium- oder Natrium-carbonat oder -hydrogencarbonat oder einer Aminbase wie Pyridin, Triethylamin, N-Methylmorpholin oder Diisopropylethylamin bei Temperaturen zwischen -20 und 200°C, vorzugsweise jedoch bei Temperaturen zwischen -10 und 160°C, durchgeführt werden.
      Weitere Verfahren zur Amidkupplung sind beispielsweise in P.D. Bailey, I.D. Collier, K.M. Morgan in "Comprehensive Functional Group Interconversions", Vol. 5, Seite 257ff., Pergamon 1995, oder auch im Houben-Weyl Ergänzungsband 22, Thieme Verlag, 2003 und der dort zitierten Literatur beschrieben.
   ii) bzw. iii) Abspaltung einer Schutzgruppe
      Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid oder mittels Etherspaltung, z. B. in Gegenwart von lodtrimethylsilan, bei Temperaturen zwischen 0 und 100°C, vorzugsweise bei Temperaturen zwischen 10 und 50°C.
      Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Tetrahydrofuran, Methanol, Ethanol, Essigsäureethylester, Dimethylformamid, Dimethylformamid/Aceton oder Eisessig gegebenenfalls unter Zusatz einer Säure wie Salzsäure bei Temperaturen zwischen 0 und 50°C, vorzugsweise jedoch bei Raumtemperatur, und bei einem Wasserstoffdruck von 1 bis 7 bar, vorzugsweise jedoch von 1 bis 5 bar.
      Die Abspaltung ener Schutzgruppe kann aber auch nach den in T.W. Greene, P.G.M. Wuts in "Protective Groups in Organic Synthesis", Wiley, 1991 und 1999, beschriebenen Verfahren durchgeführt werden.
   iv) Synthese eines Harnstoffs
      Die Umsetzung eines Derivates VII mit einem Isocyanat VIII oder einer gegebenenfalls aktivierten Carbaminsäure IX erfolgt in einem Lösungsmittel wie beispielsweise Wasser, Methylenchlorid, Chloroform, Tetrachlorkohlenstoff, Ether, Tetrahydrofuran, Dioxan, Benzol, Toluol, Acetonitril, Dimethylformamid, Dimethylsulfoxid oder Sulfolan oder einem Gemisch der genannten Lösungsmittel gegebenenfalls unter Zusatz einer Hilfsbase wie Natronlauge, Cäsium-, Kalium- oder Natrium-carbonat oder -hydrogencarbonat oder einer Aminbase wie Pyridin, Triethylamin, N-Methylmorpholin oder Diisopropylethylamin bei Temperaturen zwischen -20 und 200°C, vorzugsweise jedoch bei Temperaturen zwischen -10 und 160°C, durchgeführt werden.
(b) Die Bausteine der allgemeinen Formel 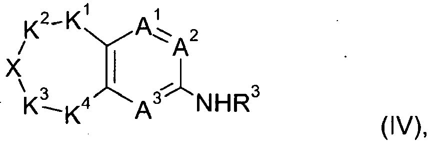 in denen A¹, A², A³, K¹, K², K³, K⁴, X und R³ wie in Ausführungsform 1 erwähnt definiert sind, und
   die gegebenenfalls an vorhandenen Amino-, Hydroxy-, Carboxy- oder Thiol-gruppen durch gängige Schutzgruppen, wie beispielsweise denen in T.W. Greene, P.G.M. Wuts in "Protective Groups in Organic Synthesis", Wiley, 1991 und 1999, beschriebenen geschützt sein können, und deren Schutzgruppen in Literatur nach bekannter Methode im Verlauf der Synthesesequenz zu Verbindungen der Formel (I) abgespalten werden können,
   sind aus der Literatur bekannt, oder deren Synthese wird in den Ausführungsbeispielen beschrieben, oder sie können beispielsweise nach in Literatur bekannter Syntheseverfahren oder in Analogie zu in Literatur bekannten Syntheseverfahren wie beispielsweise in WO2007/003536, DE4429079, US4490369, DE3515864, US5175157, DE1921861, WO85/00808 bzw. in G. Bobowski et al., J.Heterocyclic Chem. 16, 1525, 1979 oder in P.D. Johnson et al., Bioorg. Med. Chem. Lett 2003, 4197, beschrieben, hergestellt werden.
   Beispielsweise kann eine Verbindung der allgemeinen Formel (IV), in der R³ ein Wasserstoffatom bedeutet und A1, A2, A3, K1, K2, K3, K4 und X wie in Ausführungsform 1 erwähnt definiert sind durch Reduktion der Nitrogruppe einer Verbindungen derallgemeinen Formel (X) 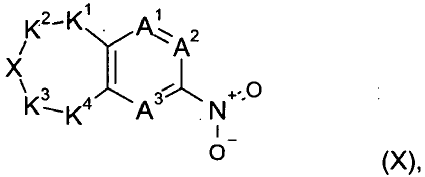 in der A1, A2, A3, K1, K2, K3, K4 und X wie in Ausführungsform 1 erwähnt definiert sind, wie folgt hergestellt werden.
   Die Reduktion der Nitrogruppe wird beispielsweise zweckmäßigerweise in einem Lösungsmittel oder Lösungsmittelgemisch wie Wasser, wäßriger Ammoniumchlorid-Lösung, Salzsäure, Schwefelsäure, Phosphorsäure, Ameisensäure, Essigsäure; Acetanhydrid mit Metallen wie Eisen, Zink, Zinn oder Schwefelverbindungen wie Ammoniumsulfid, Natriumsulfid oder Natriumdithionit oder durch katalytische Hydrierung mit Wasserstoff, beispielsweise unter einem Druck zwischen 0.5 und 100 bar, vorzugsweise jedoch zwischen 1 und 50 bar, oder mit Hydrazid als Reduktionsmittel, zweckmäßigerweise in Gegenwart eines Katalysators wie beispielsweise Raney-Nickel, Palladiumkohle, Platinoxid, Platin auf Mineralfaser oder Rhodium, oder mit komplexen Hydriden wie Lithiumaluminiumhydrid, Natriumborhydrid, Natriumcyanborhydrid, Diisobutylaluminiumhydrid, zweckmäßigerweise in einem Lösungsmittel oder Lösungsmittelgemisch wie Wasser, Methanol, Ethanol, Isopropanol, Pentan, Hexan, Cyclohexan, Heptan, Benzol, Toluol, Xylol, Ethylacetat, Methylpropionat, Glykol, Glykoldimethylether, Diethylenglykoldimethylether, Dioxan, Tetrahydrofuran, *N*-Methylpyrrolidinon, oder aber *N*-Ethyl-diisopropylamin, *N*-C₁₋₅-Alkylmorpholin, *N*-C₁₋₅-Alkylpiperidin, *N*-C₁₋₁₅-Alkylpyrrolidin, Triethylamin, Pyridin, beispielsweise bei Temperaturen zwischen -30 und 250°C, vorzugsweise jedoch zwischen 0 und 150°C, durchgeführt.
(c) Die Bausteine der allgemeinen Formel 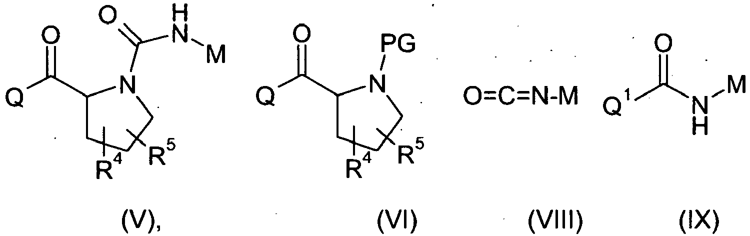 in denen R⁴, R⁵, R⁶ und R² wie in Ausführungsform 1 erwähnt definiert sind, und wobei
   Q/Q¹ beispielsweise eine Hydroxy- oder C₁₋₄-Alkyloxygruppe, ein Halogenatom, eine Alkyloxycarbonyloxy- oder Acyloxygruppe darstellt die gegebenenfalls an vorhandenen Amino-, Hydroxy-, Carboxy-oder Thiolgruppen durch gängige Schutzgruppen wie beispielsweise denen in T.W. Greene, P.G.M. Wuts in "Protective Groups in Organic Synthesis", Wiley, 1991 und 1999, beschriebenen geschützt sein können und deren Schutzgruppen in literaturbekannterweise im Verlauf der Synthesesequenz zu Verbindungen der Formel (I) abgespalten werden könnten,
   sind in der Literatur bekannt, oder deren Synthese wird in den Ausführungsbeispielen beschrieben, oder sie können beispielsweise nach in der Literatur bekannten Syntheseverfahren oder in Analogie zu in der Literatur bekannten Syntheseverfahren wie beispielsweise in WO2005/92849, WO2004/87646 oder WO2003/45912 beschrieben, hergestellt werden.

Bei den vorstehend beschriebenen Umsetzungen können gegebenenfalls vorhandene reaktive Gruppen wie Hydroxy-, Carboxy-, Amino-, Alkylamino-oder Iminogruppen während der Umsetzung durch übliche Schutzgruppen geschützt werden, welche nach der Umsetzung wieder abgespalten werden.

Beispielsweise kommt als Schutzrest für eine Hydroxygruppe die Methoxy-, Benzyloxy-, Trimethylsilyl-, Acetyl-, Benzoyl-, *tert*.-Butyl-, Trityl-, Benzyl- oder Tetrahydropyranyl-gruppe in Betracht.

Beispielsweise kommt als Schutzrest für eine Carboxylgruppe die Trimethylsilyl-, Methyl-, Ethyl-, *tert*.-Butyl-, Benzyl- oder Tetrahydropyranyl-gruppe in Betracht.

Beispielsweise kommt als Schutzrest für eine Amino-, Alkylamino- oder Iminogruppe die Acetyl-, Trifluoracetyl-, Benzoyl-, Ethoxycarbonyl-, *tert.-*Butoxycarbonyl-, Benzyloxycarbonyl-, Benzyl-, Methoxybenzyl- oder 2,4-Dimethoxybenzyl-gruppe, und für die Aminogruppe zusätzlich die Phthalylgruppe in Betracht.

Beispielsweise kommt als Schutzrest für eine Ethinylgruppe die Trimethylsilyl-, Diphenylmethylsilyl-, tert.Butyldimethylsilyl- oder eine 1-Hydroxy-1-methyl-ethylgruppe in Betracht.

Weitere Schutzgruppen die eingesetzt werden können und deren Abspaltung sind in T.W. Greene, P.G.M. Wuts, "Protective Groups in Organic Synthesis", Wiley, 1991 und 1999 beschrieben.

Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid oder mittels Etherspaltung, z. B. in Gegenwart von Iodtrimethylsilan, bei Temperaturen zwischen 0 und 100°C, vorzugsweise bei Temperaturen zwischen 10 und 50°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure bei Temperaturen zwischen 0 und 50°C, vorzugsweise jedoch bei Raumtemperatur, und bei einem Wasserstoffdruck von 1 bis 7 bar, vorzugsweise jedoch von 1 bis 5 bar.

Die Abspaltung einer Methoxybenzylgruppe kann auch in Gegenwart eines Oxidationsmittels wie Cer(IV)ammoniumnitrat in einem Lösungsmittel wie Methylenchlorid, Acetonitril oder Acetonitril/Wasser bei Temperaturen zwischen 0 und 50°C, vorzugsweise jedoch bei Raumtemperatur, erfolgen.

Die Abspaltung einer Methoxygruppe erfolgt zweckmäßigerweise in Gegenwart Bortribromid in einem Lösungsmittel wie Methylenchlorid bei Temperaturen zwischen -35 und -25°C.

Die Abspaltung eines 2,4-Dimethoxybenzylrestes erfolgt jedoch vorzugsweise in Trifluoressigsäure in Gegenwart von Anisol.

Die Abspaltung eines tert.-Butyl- oder tert.-Butoxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan oder Ether.

Die Abspaltung eines Phthalylrestes erfolgt vorzugsweise in Gegenwart von Hydrazin oder eines primären Amins wie Methylamin, Ethylamin oder *n-*Butylamin in einem Lösungsmittel wie Methanol, Ethanol, Isopropanol, Toluol/Wasser oder Dioxan bei Temperaturen zwischen 20 und 50°C.

Die Abspaltung eines Allyloxycarbonylrestes erfolgt durch Behandlung mit einer katalytischen Menge Tetrakis-(triphenylphosphin)-palladium(0) vorzugsweise in einem Lösungsmittel wie Tetrahydrofuran und vorzugsweise in Gegenwart eines Überschusses von einer Base wie Morpholin oder 1,3-Dimedon bei Temperaturen zwischen 0 und 100°C, vorzugsweise bei Raumtemperatur und unter Inertgas, oder durch Behandlung mit einer katalytischen Menge von Tris-(triphenylphosphin)-rhodium(I)chlorid in einem Lösungsmittel wie wässrigem Ethanol und gegebenenfalls in Gegenwart einer Base wie 1,4-Diazabicyclo[2.2.2]octan bei Temperaturen zwischen 20 und 70°C.

Ferner können die erhaltenen Verbindungen der allgemeinen Formel (I) in ihre Enantiomeren und/oder Diastereomeren aufgetrennt werden.

So lassen sich beispielsweise die erhaltenen Verbindungen der allgemeinen Formel (I), welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel (I) mit mindestes zwei asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch chromatographische Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren.und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Apfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise der (+)- oder (-)-Menthyloxycarbonylrest in Betracht.

Desweiteren können die erhaltenen Verwindungen der Formel (I) in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Methansulfonsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel (I), falls diese eine Carboxygruppe enthalten, gegebenenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Cyclohexylamin, Ethanolamin, Diethanolamin und Triethanolamin in Betracht.

Wie bereits eingangs erwähnt, weisen die Verbindungen der allgemeinen Formel (I) sowie deren Tautomere, deren Enantiomere, deren Diastereomere und deren physiologisch verträglichen Salze wertvolle pharmakologische Eigenschaften auf, insbesondere eine antithrombotische Wirkung, welche vorzugsweise auf einer Thrombin oder Faktor Xa beeinflussenden Wirkung beruht, beispielsweise auf einer thrombinhemmenden oder Faktor Xahemmenden Wirkung, auf einer die aPTT-Zeit verlängernden Wirkung und auf einer Hemmwirkung auf verwandte Serinproteasen wie z. B. Urokinase, Faktor VIIa, Faktor IX, Faktor XI und Faktor XII.

Die im Experimentellen Teil angeführten Verbindungen können auf ihre Wirkung auf die Hemmung des Faktors Xa wie folgt untersucht werden.

### Methodik

Enzymkinetische Messung mit chromogenem Substrat. Die durch humanen Faktor Xa aus dem farblosen chromogenen Substrat freigesetzte Menge an p-Nitroanilin (pNA) wird photometrisch bei 405 nm bestimmt. Sie ist proportional der Aktivität des eingesetzten Enzyms. Die Hemmung der Enzymaktivität durch die Testsubstanz (bezogen auf die Lösungsmittelkontrolle) wird bei verschiedenen Testsubstanz-Konzentrationen ermittelt und hieraus die IC₅₀ berechnet als diejenige Konzentration, die den eingesetzten Faktor Xa um 50 % hemmt.

### Material

Tris(hydroxymethyl)-aminomethan-Puffer (100 mMol) und Natriumchlorid (150 mMol), pH 8.0 plus 1 mg/ml Human Albumin Fraction V, Proteasefrei.

Faktor Xa (Calbiochem), Spez. Aktivität: 217 IU/mg, Endkonzentration: 7 IU/ml pro Reaktionsansatz.

Substrat S 2765 (Chromogenix), Endkonzentration: 0.3 mM/l (1 KM) pro Reaktionsansatz.

Testsubstanz: Endkonzentration 100, 30, 10, 3, 1, 0.3, 0.1, 0.03, 0.01, 0.003, 0.001 µMol/l.

### Durchführung

10 µl einer 23.5-fach konzentrierteren Ausgangslösung der Testsubstanz bzw. Lösungsmittel (Kontrolle), 175 µl TRIS/HSA-Puffer und 25 µl Faktor Xa-Gebrauchslösung von 65.8 U/L werden 10 Minuten bei 37°C inkubiert. Nach Zugabe von 25 µl S 2765-Gebrauchslösung (2.82 mMol/L) wird die Probe im Photometer (SpectraMax 250) bei 405 nm für 600 Sekunden bei 37°C gemessen.

### Auswertung

1. Ermittlung der maximalen Zunahme (deltaOD/Minuten) über 21 Messpunkte.
2. Ermittlung der %-Hemmung bezogen auf die Lösungsmittelkontrolle.
3. Erstellen einer Dosiswirkungskurve (%-Hemmung vs Substanzkonzentration).
4. Ermittlung der IC₅₀ durch Interpolation des X-Wertes (Substanzkonzentration) der Dosiswirkungskurve bei Y = 50 % Hemmung.

Alle getesteten Verbindungen zeigen IC₅₀-Werte, die kleiner als 100 µmol/L sind.

Die erfindungsgemäß hergestellten Verbindungen sind im allgemeinen gut verträglich.

Aufgrund ihrer pharmakologischen Eigenschaften eignen sich die neuen Verbindungen und deren physiologisch verträgliche Salze zur Vorbeugung und Behandlung venöser und arterieller thrombotischer Erkrankungen, wie zum Beispiel der Vorbeugung und Behandlung von tiefen Beinvenen-Thrombosen, Thrombophlebitis, der Verhinderung von Reokklusionen nach Bypass-Operationen oder Angioplastie (PT(C)A), sowie der Okklusion bei peripheren arteriellen Erkrankungen, sowie Vorbeugung und Behandlung von Lungenembolie, der disseminierten intravaskulären Gerinnung und der schweren Sepsis, der Verhinderung und Prophylaxe der DVT in Patienten mit Exacerbation der COPD, der Behandlung der ulzerativen Colitis, der Prophylaxe und Behandlung der Koronarthrombose, der Prophylaxe des Schlaganfalls und der Verhinderung der Okklusion von Shunts.

Zusätzlich sind die erfindungsgemäßen Verbindungen zur antithrombotischen Unterstützung bei einer thrombolytischen Behandlung, wie zum Beispiel mit Alteplase, Reteplase, Tenecteplase, Staphylokinase oder Streptokinase, zur Verhinderung der Langzeitrestenose nach PT(C)A, zur Prophylaxe und Behandlung von ischämischen Vorfällen in Patienten mit allen Formen der koronaren Herzerkrankung, zur Verhinderung der Metastasierung und des Wachstums von Tumoren und von Entzündungsprozessen, z.B. bei der Behandlung der pulmonalen Fibrose, zur Prophylaxe und Behandlung der rheumatoiden Arthritis, zur Verhütung oder Verhinderung von fibrin-abhängigen Gewebsadhäsionen und/oder Narbengewebebildung sowie zur Förderung von Wundheilungsprozessen geeignet.

Die genannten Verbindungen können auch als Antikoagulantien in Zusammenhang mit der Herstellung, Lagerung, Fraktionierung oder Verwendung von Vollblut oder bei invasiven Therapieverfahren, zum Beispiel zum Beschichten von Prothesen, künstlichen Herzklappen und Kathetern zur Reduktion des Thromboserisikos eingesetzt werden.

Aufgrund ihrer pharmakologischen Eigenschaften eignen sich außerdem die neuen Verbindungen und deren physiologisch verträgliche Salze zur Behandlung der Alzheimer- und Parkinson'schen Krankheit. Eine Rationale dafür ergibt sich zum Beispiel aus folgende Befunden, aus denen man schließen kann, dass Thrombinhemmer bzw. Faktor Xa Hemmer, durch Hemmung der Thrombinbildung bzw. -aktivität, wertvolle Medikamente in der Behandlung der Alzheimer- und Parkinson'schen Krankheit darstellen könnten. Klinische und experimentelle Studien legen nahe, dass neurotoxische Mechanismen, beispielsweise die mit der Aktivierung von Proteasen der Gerinnungskaskade einhergehende Entzündung, beteiligt ist am Absterben von Neuronen infolge von Himtraumata. Verschiedene Studien deuten auf eine Beteiligung von Thrombin bei neurodegenerativen Prozessen hin, beispielsweise infolge eines Schlaganfalls, wiederholter Bypassoperation oder traumatischen Hirnverletzungen. Eine erhöhte Thrombinaktivität konnte beispielsweise noch Tage nach peripherer Nervenverletzung nachgewiesen werden. Es konnte weiterhin gezeigt werden, dass Thrombin eine Neuritenretraktion, sowie Glia-Proliferation, und Apoptose in Primärkulturen von Neuronen und Neuroblastomzellen hervorruft (zur Übersicht siehe: Neurobiol. Aging, 2004, 25(6), 783-793). Darüberhinaus deuten verschiedene in vitro Studien an Gehirnen von Patienten mit Alzheimer-Krankheit darauf hin, dass Thrombin in der Pathogenese dieser Krankheit eine Rolle spielt (Neurosci. Lett., 1992, 146, 152-54). Eine Anreicherung immunreaktiven Thrombins konnte in Neuriten-Plaques in Gehirnen von Alzheimer-Patienten nachgewiesen werden. In vitro wurde gezeigt, dass Thrombin ebenfalls eine Rolle bei der Regulation und Stimulation der Produktion des "Amyloid Precursor Proteins" (APP) spielt sowie bei, der Spaltung des APP in Fragmente, welche in den Amyloid-Plaques im Gehirn von Alzheimer-Patienten nachgewiesen werden können. Weiterhin konnte gezeigt werden, dass die thrombin-induzierte mikrogliale Aktivierung in vivo zur Degeneration von nigralen dopaminergen Neuronen führt. Diese Befunde lassen den Schluss zu, dass mikrogliale Aktivierung -ausgelöst durch endogene Substanz(en) wie beispielsweise Thrombin- beteiligt sind am neuropathologischen Prozess des Zelltodes dopaminerger Neurone, wie er bei Patienten mit Parkinson'scher Krankheit vorkommt (J. Neurosci., 2003, 23, 5877-86).

Die neuen Verbindungen und ihre physiologisch verträglichen Salze sind auch für die Prophylaxe und Behandlug von ateriellen vaskulären Erkrankungen in der Kombinationstherapie mit lipidsenkenden Wirkstoffen wie HMG-CoA Reduktase Inhibitoren und Vasodilatatoren, insbesondere ACE-Inhibitoren, Angiotensin II-Antagonisten, Renin-Inhibitoren, ß-Rezeptor-Antagonisten, α-Rezeptor-Antagonisten, Diuretika, Ca-Kanalblockern, oder Stimulatoren der löslichen Guanylatzyklase einsetzbar.

Durch Erhöhung der antithrombotischen Wirkung sind die neuen Verbindungen und ihre physiologisch verträglichen Salze auch in der Kombinationstherapie mit anderen Antikoagulantien wie beispielsweise unfraktioniertem Heparin, niedermolekularem Heparin, Fondaparinux oder direkten Thrombinhemmern, zum Beispiel rekombinantem Hirudin oder "active-site"-Thrombinhemmern, einsetzbar

Die neuen Verbindungen und deren physiologisch verträgliche Salze können therapeutisch in Kombination mit Azetylsalizylsäure, mit Inhibitoren der Plättchen-Aggregation wie Fibrinogen-Rezeptorantagonisten (z.B. Abciximab, Eptifibatide, Tirofiban, Roxifiban), mit physiologischen Aktivatoren und Inhibitoren des Gerinnungssystems und deren rekombinanter Analoga (z.B. Protein C, TFPI, Antithrombin), mit Inhibitoren der ADP-induzierten Aggregation (z.B. Clopidogrel, Prasugrel, Ticlopidin), mit P₂T-Rezeptorantagonisten (z.B. Cangrelor) oder mit kombinierten Thromboxan Rezeptorantagonisten/Synthetaseinhibitoren (z.B. Terbogrel) eingesetzt werden.

Die zur Erzielung einer entsprechenden Wirkung erforderliche Dosierung beträgt zweckmäßigerweise bei intravenöser Gabe 0.01 bis 3 mg/kg, vorzugsweise 0.03 bis 1.0 mg/kg, und bei oraler Gabe 0.03 bis 30 mg/kg, vorzugsweise 0.1 bis 10 mg/kg, jeweils 1 bis 4 x täglich.

Hierzu lassen sich die erfindungsgemäß hergestellten Verbindungen der Formel (I), gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen oder Zäpfchen einarbeiten.

### Experimenteller Teil

Die nachfolgenden Beispiele sollen die Erfindung näher erläutern, ohne diese jedoch in ihrem Umfang zu beschränken.

Für die hergestellten Verbindungen liegen in der Regel Schmelzpunkte und/oder IR-, UV-, ¹H-NMR und/oder Massenspektren vor. Wenn nicht anders angegeben, wurden R_{f}-Werte unter Verwendung von DC-Fertigplatten Kieselgel 60 F₂₅₄ (E. Merck, Darmstadt, Artikel-Nr. 1.05714) ohne Kammersättigung bestimmt. Die unter der Bezeichnung Alox ermittelten R_{f}-Werte wurden unter Verwendung von DC-Fertigplatten Aluminiumoxid 60 F₂₅₄ (E. Merck, Darmstadt, Artikel-Nr. 1.05713) ohne Kammersättigung bestimmt. Die unter der Bezeichnung Reversed-Phase-8 (RP-8) ermittelten R_{f}-Werte wurden unter Verwendung von DC-Fertigplatten RP-8 F₂₅₄ₛ (E. Merck, Darmstadt, Artikel-Nr. 1.15684) ohne Kammersättigung bestimmt. Die bei den Fliessmitteln angegebenen Verhältnisse beziehen sich auf Volumeneinheiten der jeweiligen Lösungsmittel. Zu chromatographischen Reinigungen wurde Kieselgel der Firma Millipore (MATREX^{™}, 35-70 µm) verwendet. Falls nähere Angaben zur Konfiguration fehlen, bleibt offen, ob es sich um reine Stereoisomere oder um Enantiomeren-/Diastereomerengemische handelt.

Die HPLC-MS Daten wurden unter den folgenden Bedingungen erzeugt.

Waters Alliance 2690, Waters ZQ2000 Massen Spektrometer mit Diodenarraydetektor 996. Als mobile Phase wurde eingesetzt:
A: Wasser mit 0.10% TFA
B: Acetonitril mit 0.8% TFA

| Zeit in min | %A | %B | Flussrate in ml/min |
|---|---|---|---|
| 0.00 | 95 | 5 | 1.00 |
| 0.10 | 95 | 5 | 1.00 |
| 3.10 | 2 | 98 | 1.00 |
| 4.50 | 2 | 98 | 1.00 |
| 5.00 | 95 | 5 | 1.00 |

Als stationäre Phase diente eine Säule X-Terra MS C18, 2.5 µm, 4.6 mm x 30 mm.

Die Diodenarraydetektion erfolgte im Wellenlängenbereich 210-500 nm.

In den Versuchsbeschreibungen werden die folgenden Abkürzungen verwendet.

| | |
|---|---|
| DCM | Dichlormethan |
| DIPEA | *N*-Ethyl-diisopropylamin |
| DMF | *N,N*-Dimethylformamid |
| EtOH | Ethanol |
| ges. | gesättigt |
| h | Stunde(n) |
| HATU | *O*-(7-Azabenzotriazol-1-yl)-*N,N,N',N'*-tetramethyluronium-hexafluorphosphat |
| i. Vak. | im Vakuum |
| konz. | konzentriert |
| min | Minute(n) |
| NMM | *N*-Methyl-morpholin |
| R_{f} | Retentionsfaktor |
| Rₜ | Retentionszeit |
| TBTU | *O*-(Benzotriazol-1-yl)-*N,N,N',N'*-tetramethyluroniumtetrafluorborat |
| TEA | Triethylamin |
| TFA | Trifluoressigsäure |
| THF | Tetrahydrofuran |

### Beispiel 1

### (2R,4R)-4-Methoxy-pyrrolidin-1,2-dicarbonsäure-1-(4-chlor-phenyl)amid-2-(3-methyl-2,3,4,5-tetrahydro-1H-benzo[d]azepin-7-yl)amid

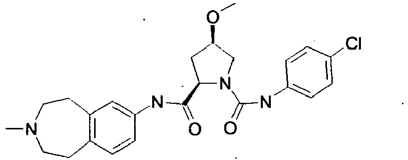

### (a) (2R,4R)-4-Methoxy-pyrrolidin-1,2-dicarbonsäure-1-tert.butoxy-ester-2-(3-methyl-2,3,4,5-tetrahydro-1H-benzo[d]azepin-7-yl)-amid

0.170 g (0.693 mmol) (2R,4R)-4-Methoxy-pyrrolidin-1,2-dicarbonsäure-1-tert.butoxy-ester werden in 5.0 ml THF gelöst, mit 0.19 ml (1.7 mmol) NMM und 0.234 g (0.728 mmol) TBTU verstetzt und 15 min gerührt. Anschließend werden 0.122 g (0.693 mmol) 7-Amino-3-methyl-2,3,4,5-tetrahydro-1*H*-benzo[*d*]azepin zugegeben und 16 h gerührt. Das Reaktionsgemisch wird i.Vak. konzentriert, mit 20 ml Essigester versetzt und sukzessiv mit ges. NaHCO₃-Lösung, ges. NaCl-Lösung und Wasser gewaschen und dann mir Na₂SO₄ und i. Vak. bis zur Trockene eingeengt.
Ausbeute: 0.210 g (75%)
R_{f}-Wert: 0.8 (Kieselgel; Dichlormethan/Ethanol/Ammoniak = 80:20:2) C₂₂H₃₃N₃O₄ (403.515)
Massenspektrum: (M+H)⁺ = 404

### (b) (2R,4R)-4-Methoxy-pyrrolidin-2-carbonsäure-(3-methyl-2,3,4,5-tetrahydro-1H-benzo[d]azepin-7-yl)-amid

Zu einer Lösung aus 0.210 g (0.520 mmol) (2R,4R)-4-Methoxy-pyrrolidin-1,2-dicarbonsäure-1-tert.butoxy-ester-2-(3-methyl-2,3,4,5-tetrahydro-1*H-*benzo[*d*]azepin-7-yl)-amid in 2.0 ml THF werden über 2 h 1.5 ml 6 M HCl zugegeben und insgesamt 18 h gerührt. Das Reaktionsgemisch wird i. Vak. eingeengt, mehrmals mit Methanol versetzt und erneut eingeengt.
Rohausbeute: 0.230 g (quantitativ)
R_{f}-Wert: 0.25 (Kieselgel; Dichlormethan/Ethanol/Ammoniak = 80:20:2) C₁₇H₂₅N₃O₂ (303.515) x 2 HCl
Massenspektrum: (M+H)⁺ = 304

### (c) (2R,4R)4-Methoxy-pyrrolidin-1,2-dicarbonsäure-1 -(4-chlor-phenyl)amid-2-(3-methyl-2,3,4,5-tetrahydro-1H-benzo[d]azepin-7-yl)amid

Zu einer Lösung aus 0.100 g (0.266 mmol) (2R,4R)-4-Methoxy-pyrrolidin-2-carbonsäure-(3-methyl-2,3,4,5-tetrahydro-1*H*-benzo[*d*]azepin-7-yl)-amid in 2.0 ml Dioxan und 2.0 ml DMF werden 40.8 mg (0.266 mmol) 4-Chlorphenylisocyanat zugegeben und 18 h gerührt. Das Reaktionsgemisch wird i.Vak. konzentriert und durch präp. HPLC gereinigt (Methode A)
Ausbeute: 15 mg (11%)
R_{f}-Wert: 0.4 (Kieselgel; Dichlormethan/Ethanol/Ammoniak = 80:20:2) C₂₄H₂₉ClN₄O₃ (456.974) x HCOOH
Massenspektrum: (M+H)⁺ = 457/459 (Chlorisotope)

Analog können die folgenden Verbindungen hergestellt werden:

| **Bsp.** | **Strukturformel** | **Ausbeute** | **Massenpeak(s)** | **DC/HPLC** |
|---|---|---|---|---|
| **2** | 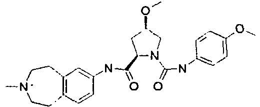 | 75% | (M+H)⁺ = 453 | R_{f}-Wert: 0.4 (Kieselgel; DCM/EtOH/NH3 = 80:20:2) |
| | (2R,4R)-4-Methoxy-pyrrolidin-1,2-dicarbonsäure-1-(4-methoxyphenyl)amid-2-(3-methyl-2,3,4,5-tetrahydro-1*H*-benzo[*d*]azepin-7-yl)amid | | | |
| **3** | 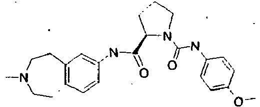 | 10% | (M+H)⁺ = 423 | R_{f}-Wert: 0.56 (RP-8; MeOH/5%NaCl-Lsg. = 6:4) |
| | | | (M-H)⁻ = 421 | |
| | (2R)-Pyrrolidin-1,2-dicarbonsäure-1-(4-methoxy-phenyl)amid-2-(3-methyl-2,3,4,5-tetrahydro-1*H*-benzo[*d*]azepin-7-yl)amid | | | |
| **4** | 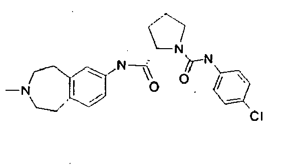 | 6% | (M+H)⁺ = 427/429 (Chlorisotope) | R_{f}-Wert: 0.44 (RP-8; MeOH/5%NaCl-Lsg. = 6:4) |
| | (2S)-Pyrrolidin-1,2-dicarbonsäure-1-(4-chlor-phenyl)amid-2-(3-methyl-2,3,4,5-tetrahydro-1*H*-benzo[*d*]azepin-7-yl)amid | | | |
| **5** | 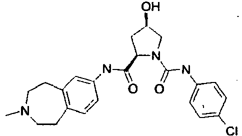 | 30% | (M+H)⁺ = 443/445 (Chlorisotope) | Rₜ-Zeit: 2.27 min (Methode A) |
| | (2R, 4R)-4-Hydroxy-pyrrolidin-1,2-dicarbonsäure-1-(4-chlor-phenyl]amid-2-(3-methyl-2,3,4,5-tetrahydro-1*H*-benzo[*d*]azepin-7-yl)amid | | | |
| **6** | 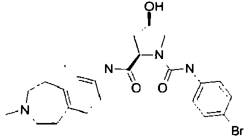 | 21% | (M+H)⁺ = 487/489 (Bromisotope) | Rₜ-Zeit: 2.28 min (Methode A) |
| | (2R,4R)-4-Hydroxy-pyrrolidin-1,2-dicarbonsäure-1-(4-bromphenyl)amid-2-(3-methyl-2,3,4,5-tetrahydro-1*H*-benzo[*d*]azepin-7-yl)amid | | | |
| **7** | 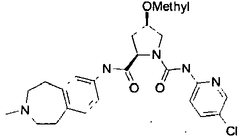 | 65% | (M+H)⁺ = 458/460 (Chlorisotope) | R_{f}-Wert: 0.3 (Kieselgel; DCM/EtOH/NH3 = 80:20:2) |
| | (2R,4R)-4-Methoxy-pyrrolidin-1,2-dicarbonsäure-1-(5-chlor-pyridin-2-yl)amid-2-(3-methyl-2,3,4,5-tetrahydro-1*H*-benzo[*d*]azepin-7-yl)amid | | | |
| **8** | 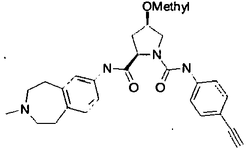 | 38% | (M+H)⁺ = 447 | R_{f}-Wert: 0.7 (Kieselgel; DCM/EtOH/NH3 = 80:20:2) |
| | (2R,4R)-4-Methoxy-pyrrolidin-1,2-dicarbonsäure-1-(4-ethinyl-phenyl)amid-2-(3-methyl-2,3,4,5-tetrahydro-1*H*-benzo[*d*]azepin-7-yl)-amid | | | |
| **9** | 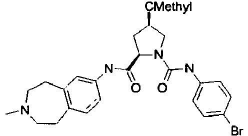 | 62% | (M+H)⁺ = 501/503 (Bromisotope) | Rₜ-Zeit: 4.17 min (Methode A) |
| | (2R,4R)-4-Methoxy-pyrrolidin-1,2-dicarbonsäure-1-(4-brom-phenyl)amid-2-(3-methyl-2,3,4,5-tetrahydro-1*H*-benzo[*d*]azepin-7-yl)amid | | | |
| **10** | 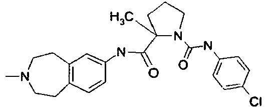 | 80% | (M+H)⁺ = 441/443 (Chlorisotope) | R_{f}-Wert: 0.6 (Kieselgel; DCM/EtOH/NH3 = 80:20:2) |
| | Pyrrolidin-2-methyl-1,2-dicarbonsäure-1-(4-chlor-phenyl)amid-2-(3-methyl-2,3,4,5-tetrahydro-1*H*-benzo[*d*]azepin-7-yl)amid | | | |

### Beispiel 11

### 2,5-Dihydro-pyrrole-1,2-dicarbonsäure-1-(4-chlor-phenyl)amid-2-[(3-methyl-2,3,4,5-tetrahydro-1H-benzo[d]azepin-7-yl)amid

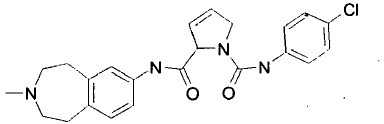

### (a) 1-(4-Chlor-phenylcarbamoyl)-2,5-dihydro-1H-pyrrol-2-carbonsäure

0.250 g (2.21 mmol) 3,4-Dehydro-DL-prolin werden in 15 ml 5%-NaHCO₃-Lösung gelöst, mit 0.678 ml (4.42 mmol) 4-Chlor-phenylisocyanat versetzt und 16 h bei 80°C gerührt. Anschließend wird abgekühlt, filtriert und der Rückstand mit Wasser gewaschen. Das Filtrat wird mit halbkonzentrierter HCl auf pH 1 gebracht, 2x mit Essigester extrahiert, mit Natriumsulfat getrocknet und i.Vak. konzentriert.
Ausbeute: 0.640 g (quantitativ, leicht unrein)
C₁₂H₁₁N₂O₃ (266.680)
Massenspektrum: (M+H)⁺ = 265/267 Chlorisotope

### (b) 2,5-Dihydro-pyrrole-1,2-dicarbonsäure-1-(4-chlor-phenyl)amid-2-(3-methyl-2,3,4,5-tetrahydro-1H-benzo[d]azepin-7-yl)amid

0.200 g (0.750 mmol) 1-(4-Chlor-phenylcarbamoyl)-2,5-dihydro-1*H*-pyrrol-2-carbonsäure werden analog Beispiel 1a mit 0.132 g (0.750 mmol) 7-Amino-3-methyl-2,3,4,5-tetrahydro-1*H*-benzo[*d*]azepin, NMM und TBTU zur Titelverbindung umgesetzt.
Ausbeute: 50 mg (14%)
R_{f}-Wert: 0.6 (Kieselgel; Dichlormethan/Ethanol/Ammoniak = 80:20:2) C₂₃H₂₅ClN₄O₂ (424.923)
Massenspektrum: (M+H)⁺ = 425/427 (Chlorisotope)

Analog kann die folgende Verbindung hergestellt werden:

| **Bsp.** | **Strukturformel** | **Massenpeak(s)** | **DC/HPLC** |
|---|---|---|---|
| **12** | 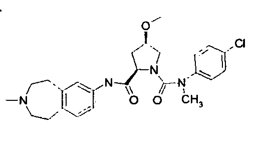 | (M+H)⁺ = 471/473 (Chlorisotope) | R_{f}-Wert: 0.3 (Kieselgel; Dichlormethan/Ethanol/A mmoniak = 80:20:2) |
| | (2R,4R)-4-Methoxy-pyrrolidin-1,2-dicarbonsäure-1-N-(4-chlorphenyl)-N-methyl-amid-2-(3-methyl-2,3,4,5-tetrahydro-1*H-*benzo[*d*]azepin-7-yl)amid | | |

### Beispiel 13

### (2R,4R)-4-Methoxy-pyrrolidin-1,2-dicarbonsäure-1-(4-chlor-phenyl)amid-2-((R)-1,3-dimethyl-2,3,4,5-tetrahydro-1H-benzo[d]azepin-8-yl)amid und (2R,4R)-4-Methoxy-pyrrolidin-1,2-dicarbonsäure-1-(4-chlor-phenyl)amid-2-((S)-1,3-dimethyl-2,3,4,5-tetrahydro-1H-benzo[d]azepin-8-yl)amid

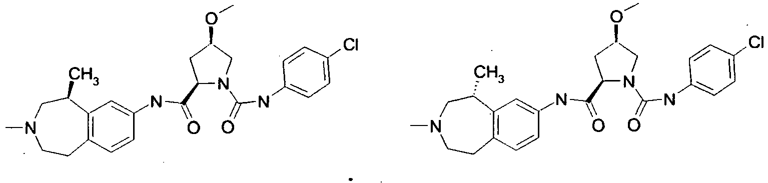

### (a) 1-Methyl-2-oxo-2,3,4,5-tetrahydro-1H-benzo[d]azepin

8.0 g (37 mmol) 2-Chlor-N-(2-phenylethyl)-propanamid und 15 g (112 mmol) Aluminiumtrichloride werden vorsichtig bei 90°C gemischt und 6 h auf 150°C erhitzt. Die Mischung wird mit Wasser und Methanol verdünnt und mit EtOAc extrahiert. Die vereinigten organischen Phasen werden mit Na₂SO₄ getrocknet, i.Vak. konzentriert und chromatographisch gereinigt.

### (b) 1-Methyl-2,3,4,5-tetrahydro-1H-benzo[d][azepin

2.7 g (15 mmol) 1-Methyl-2-oxo-2,3,4,5-tetrahydro-1*H*-benzo[*d*]azepin wird zu 46 ml 1M BH₃-THF Komplex-lösung gegeben und 16 h bei Raumtemperatur gerührt. 50 ml Methanol werden vorsichtig zugegeben, gefolgt von 30 ml 2M HCl. Die Mischung wird mit EtOAc extrahiert, die vereinigten organischen Phasen werden mit Na₂SO₄ getrocknet, i.Vak. konzentriert und chromatographisch gereinigt.

### (c) 1,3-Dimethyl-2,3,4,5-tetrahydro-1H-benzo[d]azepin

2.5 g (12 mmol) 1-Methyl-2,3,4,5-tetrahydro-1*H*-benzo[*d*][azepin werden in 4.5 ml Ameisensäure unter Rühren bei Raumtemperatur mit 3.6 ml Formalin-Lösung in Wasser (37%) versetzt und 3 h bei 70°C gerührt. Die Reaktionsmischung wird unter Eisbadkühlung mit NaOH-Lösung (50%) alkalisch gestellt und mit *tert.*-Butylmethylether extrahiert. Die organische Phase wird über Natriumsulfat getrocknet und i. Vak bis zur Trockene eingeengt.

### d) 1,3-Dimethyl-7-nitro-2,3,4,5-tetrahydro-1H-benzo[d]azepin und 1,3-Dimethyl-8-nitro-2,3,4,5-tetrahydro-1H-benzo[d]azepin

1.79 g (10 mmol) von 1,3-Dimethyl-2,3,4,5-tetrahydro-1*H*-benzo[*d*]azepin wird mit 3.7 ml konc. H₂SO₄ und 0.71 ml 65% HNO₃ bei -5°C gemischt und 1 h bei - 5°C bis 0°C gerührt. Die Mischung wird auf 100 ml Eiswasser gegeben und 10 ml NaOH werden zugesetzt. Die Mischung wird mit EtOAc extrahiert, die vereinigten organischen Phasen werden mit Na₂SO₄ getrocknet konzentriert und durch Chromatographie gereinigt (Eluens: Dichloromethan:95%ethanol/5% Ammoniak 99:1 to 95:5). Es wird eine Mischung der Titelverbindungen erhalten.

### (d) 1,3-Dimethyl-7-amino-2,3,4,5-tetrahydro-1H-benzo[d]azepin und 1,3-Dimethyl-8-amino-2,3,4,5-tetrahydro-1H-benzo[d]azepin

1.4 g (6.3 mmol) einer Mischung aus 1,3-Dimethyl-7-nitro-2,3,4,5-tetrahydro-1*H*-benzo[*d*]azepin und 1,3-Dimethyl-8-nitro-2,3,4,5-tetrahydro-1*H-*benzo[*d*]azepin,, 20 ml Methanol und 0.20 g 10% Palladium auf Kohle wird für 5.5 h unter einer Wasserstoff-Atmosphäre (50 psi) hydriert. Es wird filtriert, konzentriert und die Mischung wird chromatographisch mit Kieselgel gereinigt (Eluens: Dichloromethan:95%Ethanol/5% Ammoniak 99:1 to 80:20). Man erhält 0.45 g 1,3-Dimethyl-7-amino-2,3,4,5-tetrahydro-1*H*-benzo[*d*]azepin R_{f}-Wert: 0.75 (Kieselgel; Dichlormethan/Ethanol/Ammoniak = 80:20:2) C₁₂H₁₈N₂ (190.28)
Massenspektrum: (M+H)⁺ = 191
und 0.55 g 1,3-Dimethyl-8-amino-2,3,4,5-tetrahydro-1*H*-benzo[d]azepin. R_{f}-Wert: 0.70 (Kieselgel; Dichlormethan/Ethanol/Ammoniak = 80:20:2) C₁₂H₁₈N₂ (190.28)
Massenspektrum: (M+H)⁺ = 191.

### (e) (2R,4R)-4-Methoxy-pyrrolidin-1,2-dicarbonsäure-1-(4-chlor-phenyl)amid-2-((R)-1,3-dimethyl-2,3,4,5-tetrahydro-1H-benzo[d]azepin-8-yl)amid und (2R,4R)-4-Methoxy-pyrrolidin-1,2-dicarbonsäure-1-(4-chlor-phenyl)amid-2-((S)-1,3-dimethyl-2,3,4,5-tetrahydro-1H-benzo[d]azepin-8-yl)amid

Eine Mischung aus 5.0 ml DMF, 0.157 g (0.53 mmol) (2R,4R)-1-(4-Chlor-phenylcarbamoyl)-4-methoxy-pyrrolidin-2-carbonsäure, 0.20 ml NMM, 0.21 g (0.55 mmol) HATU und 0.10 g (0.53 mmol) 1,3-Dimethyl-8-amino-2,3,4,5-tetrahydro-1*H*-benzo[*d*]azepin werden über Nacht auf 70°C erwärmt. Das Reaktionsgemisch wird konzentriert, in EtOAc aufgenommen und mit ges. NaHCO₃-Lösung und Wasser und ges. NaCl-Lösung gewaschen. Die organische Phase wird mit NaSO₄ getrocknet, eingeengt und HPLC-MS gereinigt. Man erhält ein Gemisch der beiden Diastereomere.
R_{f}-Wert: 0.8 (Kieselgel; Dichlormethan/Ethanol/Ammoniak = 80:20:2)
C₂₅H₃₁ClN₄O₃ (470.99)
Massenspektrum: (M+H)⁺ = 471/473 (Chlorisotope)

### Beispiel 14

### (2R,4R)-4-Methoxy-pyrrolidin-1,2-dicarbonsäure-1-(4-chlor-phenyl)amid-2-((R)-1,3-dimethyl-2,3,4,5-tetrahydro-1H-benzo[d]azepin-7-yl)amid und (2R,4R)-4-Methoxy-pyrrolidin-1,2-dicarbonsäure-1-(4-chlor-phenyl)amid-2-((S)-1,3-dimethyl-2,3,4,5-tetrahydro-1H-benzo[d]azepin-7-yl)amid

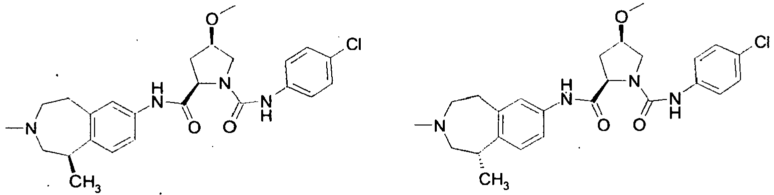

Eine Mischung der beiden Titelverbindungen wurde analog Beispiel 13 e aus (2R,4R)-1-(4-Chlor-phenylcarbamoyl)-4-methoxy-pyrrolidin-2-carbonsäure und 1,3-Dimethyl-7-amino-2,3,4,5-tetrahydro-1*H*-benzo[*d*]azepin hergestellt R_{f}-Wert: 0.8 (Kieselgel; Dichlormethan/Ethanol/Ammoniak = 80:20:2) C₂₅H₃₁ClN₄O₃ (470.99)
Massenspektrum: (M+H)⁺ = 471/473 (Chlorisotope)

Analog zu den oben beschriebenen Verfahren oder zu literaturbekannten Verfahren wie beispielsweise in WO2007/3536, WO2004/87646 oder WO2005/92849 beschrieben, können die folgenden Verbindungen hergestellt werden:

| **Bsp.** | **Strukturformel** | **Massenpeak(s)** | **DC/HPLC** |
|---|---|---|---|
| **15** | 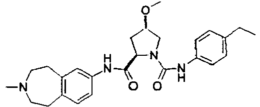 | (M+H)⁺ = 451 | |
| | (2R,4R)-4-Methoxy-pyrrolidin-1,2-dicarbonsäure-1-N-(4-ethylphenyl)amid-2-(3-methyl-2,3,4,5-tetrahydro-1*H*-benzo[*d*]azepin-7-yl)-amid | | |
| **16** | 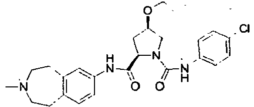 | (M+H)⁺ = 485/487 Chlorisotope | |
| | (2R,4R)-4-Propyloxy-pyrrolidin-1,2-dicarbonsäure-1-N-(4-chlorphenyl)amid-2-(3-methyl-2,3,4,5-tetrahydro-1*H*-benzo[*d*]azepin-7-yl)-amid | | |
| **17** | 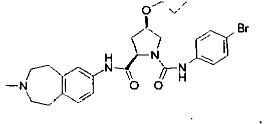 | (M+H)⁺ = 529/531 Bromisotope | |
| | (2R,4R)-4-Propyloxy-pyrrolidin-1,2-dicarbonsäure-1-N-(4-brom-phenyl)amid-2-(3-methyl-2,3,4,5-tetrahydro-1*H*-benzo[*d*]azepin-7-yl)-amid | | |
| **18** | 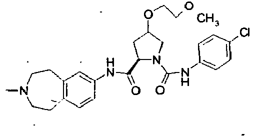 | (M+H)⁺ = 501/503 Chlorisotope | R_{f}-Wert: 0.8 (Kieselgel; Dichlormethan/Ethanol/Am moniak = 80:20:2) |
| | (2R)-4-Methoxyethoxy-pyrrolidin-1,2-dicarbonsäure-1-N-(4-chlorphenyl)amid-2-(3-methyl-2,3,4,5-tetrahydro-1*H*-benzo[*d*]azepin-7-yl)-amid -Mischung von Stereoisomeren | | |
| **19** | 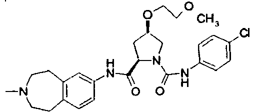 | (M+H)⁺ = 501/503 Chlorisotope | R_{f}-Wert: 0.8 (Kieselgel; Dichlormethan/Ethanol/Am moniak = 80:20:2) |
| | (2R,4R)-4-Methoxyethoxy-pyrrol idin-1,2-dicarbonsäure-1-N-(4-chlorphenyl)amid-2-(3-methyl-2,3,4,5-tetrahydro-1*H*-benzo[*d*]azepin-7-yl)-amid | | |
| **20** | 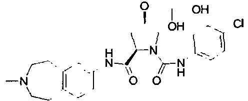 | (M+H)⁺ = 517/519 Chlorisotope | |
| | (2R,4R)-4-(2,3-Dihydroxy)propyl-1,2-dicarbonsäure-1-N-(4-chlor-phenyl)amid-2-(3-methyl-2,3,4,5-tetrahydro-1*H*-benzo[*d*]azepin-7-yl)-amid - Mischung von Stereoisomeren | | |
| **21** | 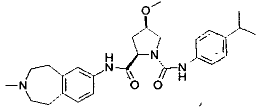 | (M+H)⁺ = 465 | |
| | (2R,4R)-4-Methoxy-pyrrolidin-1,2-dicarbonsäure-1-N-(4-isopropyl-phenyl)amid-2-(3-methyl-2,3,4,5-tetrahydro-1*H*-benzo[*d*]azepin-7-yl)-amid | | |
| **22** | 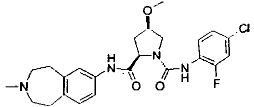 | (M+H)⁺ = 475/477 Chlorisotope | |
| | (2R,4R)-4-Methoxy-pyrrolidin-1,2-dicarbonsäure-1-N-(4-chlor-2-fluor-phenyl)-amid-2-(3-methyl-2,3,4,5-tetrahydro-1*H*-benzo[*d*]azepin-7-yl)-amid | | |
| **23** | 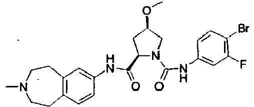 | (M+H)⁺ = 519/521 Bromisotope | |
| | (2R,4R)-4-Methoxy-pyrrolidin-1,2-dicarbonsäure-1-N-(4-brom-3-fluorphenyl)amid-2-(3-methyl-2,3,4,5-tetrahydro-1*H*-benzo[*d*]azepin-7-yl)-amid | | |
| **24** | 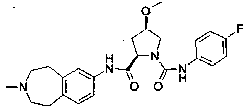 | (M+H)⁺ = 441 | R_{f}-Wert: 0.87 (Kieselgel; Dichlormethan/Ethanol/Am moniak = 80:20:2) |
| | (2R,4R)-4-Methoxy-pyrrolidin-1,2-dicarbonsäure-1-N-(4-fluorphenyl)amid-2-(3-methyl-2,3,4,5-tetrahydro-1*H*-benzo[*d*]azepin-7-yl)-amid | | |
| **25** | 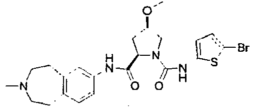 | (M+H)⁺ = 507/509 Bromisotope | |
| | (2R,4R)-4-Methoxy-pyrrolidin-1,2-dicarbonsäure-1-N-(5-brom-thien-2-yl)amid-2-(3-methyl-2,3,4,5-tetrahydro-1*H*-benzo[*d*]azepin-7-yl)-amid | | |
| **26** | 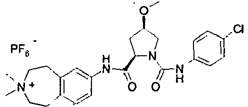 | (M+H)⁺ = 471/473 Chlorisotope | |
| | (2R,4R)-4-Methoxy-pyrrolidin-1,2-dicarbonsäure-1-N-(4-chlor-phenyl)amid-2-(3,3-dimethyl-2,3,4,5-tetrahydro-1*H*-benzo[*d*]azepin-7-ylium)-amid hexafluorophosphat | | |
| **27** | 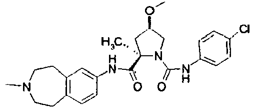 | (M+H)⁺ = 471/473 Chlorisotope | |
| | (2R,4R)-4-Methoxy-2-methyl-pyrrolidin-1,2-dicarbonsäure-1-N-(4-chlor-phenyl)amid-2-(3-methyl-2,3,4,5-tetrahydro-1*H*-benzo[*d*]azepin-7-yl)-amid | | |
| **28** | 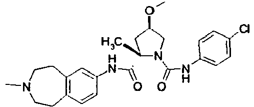 | (M+H)⁺ = 471/473 Chlorisotope | |
| | (2S,4R)-4-Methoxy-2-methyl-pyrrolidin-1,2-dicarbonsäure-1-N-(4-chlor-phenyl)amid-2-(3-methyl-2,3,4,5-tetrahydro-1*H*-benzo[*d*]azepin-7-yl)-amid | | |
| **29** | 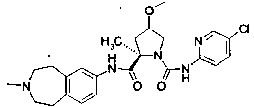 | (M+H)⁺ = 472/474 Chlorisotope | |
| | (2S,4R)-4-Methoxy-2-methyl-pyrrolidin-1,2-dicarbonsäure-1-N-(5-chlor-pyridin-2-yl)amid-2-(3-methyl-2,3,4,5-tetrahydro-1*H-*benzo[*d*]azepin-7-yl)-amid | | |
| **30** | 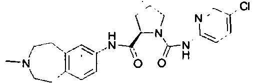 | (M+H)⁺ = 428/430 Chlorisotope | |
| | (2S)-Pyrrolidin-1,2-dicarbonsäure-1-N-(5-chlor-pyridin-2-yl)amid-2-(3-methyl-2,3,4,5-tetrahydro-1*H*-benzo[*d*]azepin-7-yl)-amid | | |
| **31** | 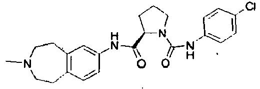 | (M+H)⁺ =427/429 Chlorisotope | |
| | (2S)-Pyrrolidin-1,2-dicarbonsäure-1-N-(4-chlor-phenyl)amid-2-(3-methyl-2,3,4,5-tetrahydro-1*H*-benzo[*d*]azepin-7-yl)-amid | | |
| **32** | 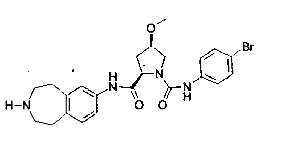 | (M+H)⁺ = 487/489 Bromisotope | |
| | (2R,4R)-4-Methyloxy-pyrrolidin-1,2-dicarbonsäure-1-N-(4-brom-phenyl)amid-2-(2,3,4,5-tetrahydro-1*H*-benzo[*d*]azepin-7-yl)-amid | | |
| **33** | 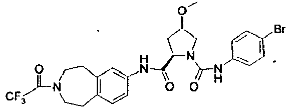 | (M-H)⁻ = 581/583 Bromisotope | |
| | (2R,4R)-4-Methyloxy-pyrrolidin-1,2-dicarbonsäure-1-N-(4-brom-phenyl)amid-2-(3-trifluoracetyl-2,3,4,5-tetrahydro-1*H*-benzo[*d*]azepin-7-yl)-amid | | |
| **34** | 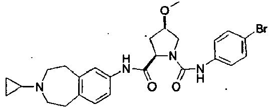 | (M-H)⁻ =527/529 Bromisotope | R_{f}-Wert: 0.63 (RP-8; Methanol/5%-NaCl-Lsg. = 6/4) |
| | (2R,4R)-4-Methyloxy-pyrrolidin-1,2-dicarbonsäure-1-N-(4-brom-phenyl)amid-2-(3-cyclopropyl-2,3,4,5-tetrahydro-1*H*-benzo[d]azepin-7-yl)-amid | | |
| **35** | 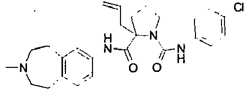 | (M+H)⁺ = 467/469 Chlorisotope | R_{f}-Wert: 0.41 (Alox; CH₂Cl₂/Ethanol = 19/1) |
| | (rac)-2-Allyl-pyrrolidin-1,2-dicarbonsäure-1-N-(4-chlor-phenyl)amid-2-(3-methyl-2,3,4,5-tetrahydro-1*H*-benzo[*d*]azepin-7-yl)-amid | | |
| **36** | 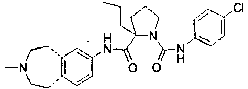 | (M+H)⁺ = 469/471 Chlorisotope | |
| | (rac)-2-Propyl-pyrrolidin-1,2-dicarbonsäure-1-N-(4-chlor-phenyl)amid-2-(3-methyl-2,3,4,5-tetrahydro-1*H*-benzo[*d*]azepin-7-yl)-amid | | |
| **37** | 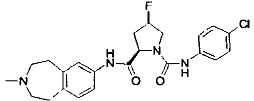 | (M+H)⁺ = 445/447 Chlorisotope | R_{f}-Wert: 0.7 (Kieselgel; Dichlormethan/Ethanol/Am moniak = 80:20:2) |
| | (2R)-4-Fluor-pyrrolidin-1,2-dicarbonsäure-1-N-(4-chlor-phenyl)amid-2-(3-methyl-2,3,4,5-tetrahydro-1*H*-benzo[*d*]azepin-7-yl)-amid (Stereoisomere) | | |
| **38** | 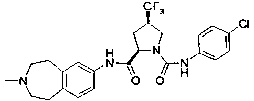 | (M+H)^{+ .}= 495/497 Chlorisotope | |
| | (2R,4R)-4-Trifluormethyl-pyrrolidin-1,2-dicarbonsäure-1-N-(4-chlor-phenyl)amid-2-(3-methyl-2,3,4,5-tetrahydro-1*H*-benzo[*d*]azepin-7-yl)-amid | | |
| **39** | 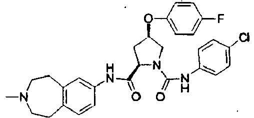 | (M+H)⁺ = 537/539 Chlorisotope | |
| | (2R,4R)-4-(4-Fluorphenyl)oxy-pyrrolidin-1,2-dicarbonsäure-1-N-(4-chlor-phenyl)amid-2-(3-methyl-2,3,4,5-tetrahydro-1*H*-benzo[*d*]azepin-7-yl)-amid | | |
| **40** | 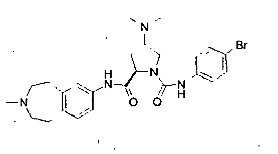 | (M+H)⁺ = 514/516 Bromisotope | R_{f}-Wert: 0.21 (Kieselgel; Dichlormethan/Ethanol/Am moniak = 80:20:2) |
| | (2R)-4-Dimethylamino-pyrrolidin-1,2-dicarbonsäure-1-N-(4-brom-phenyl)amid-2-(3-methyl-2,3,4,5-tetrahydro-1*H*-benzo[*d*]azepin-7-yl)-amid (2 Stereoisomere) | | |
| **41** | 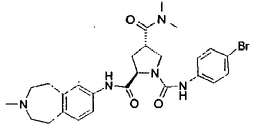 | (M+H)⁺ = 542/544 Bromisotope | R_{f}-Wert: 0.55 (Kieselgel; Dichlörmethan/Ethanol/Am moniak = 80:20:2) |
| | (2R,4S)-4-Dimethylaminocarbonyl-pyrrolidin-1,2-dicarbonsäure-1-N-(4-brom-phenyl)amid-2-(3-methyl-2,3,4,5-tetrahydro-1*H-*benzo[*d*]azepin-7-yl)-amid | | |
| **42** | 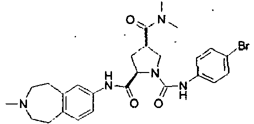 | (M+H)⁺ = 542/544 Bromisotope | R_{f}-Wert: 0.45 (Kieselgel; Dichlormethan/Ethanol/Am moniak = 80:20:2) |
| | (2R,4R)-4-Dimethylaminocarbonyl-pyrrolidin-1,2-dicarbonsäure-1*H-*(4-brom-phenyl)amid-2-(3-methyl-2,3,4,5-tetrahydro-1*H-*benzo[d]azepin-7-yl)-amid | | |
| **43** | 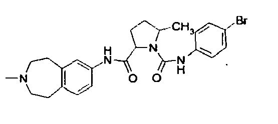 | (M+H)⁺ = 485/587 Bromisotope | |
| | 5-Methyl-pyrrolidin-1,2-dicarbonsäure-1-N-(4-brom-phenyl)amid-2-(3-methyl-2,3,4,5-tetrahydro-1*H*-benzo[*d*]azepin-7-yl)-amid (Stereoisomere) | | |
| **44** | 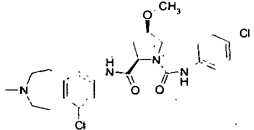 | (M+H)⁺ = 491/493/495 Chlorisotope | R_{f}-Wert: 0.33 (RP-8; Methanol/5%-NaCl-Lsg. = 6/4) |
| | (2R,4R)-4-Methyloxy-pyrrolidin-1,2-dicarbonsäure-1-N-(4-chlor-phenyl)amid-2-(9-chlor-3-methyl-2,3,4,5-tetrahydro-1*H-*benzo[*d*]azepin-7-yl)-amid | | |
| **45** | 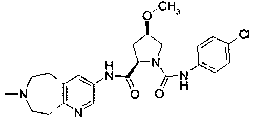 | (M+H)⁺ = 458/460 Chlorisotope | |
| **46** | 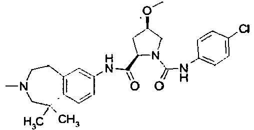 | (M+H)⁺ = 485/487 Chlorisotope | |
| | (2R,4R)-4-Methyloxy-pyrrolidin-1,2-dicarbonsäure-1-N-(4-chlor-phenyl)amid-2-(1,1,3-trimethyl-2,3,4,5-tetrahydro-1*H*-benzo[*d*]azepin-7-yl)-amid | | |

Die folgenden Verbindungen können analog hergestellt werden:

| | | | | | |
|---|---|---|---|---|---|
| **A** | 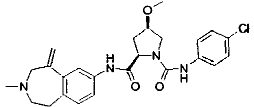 | **B** | 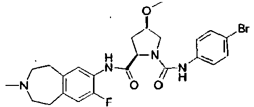 | **C** | 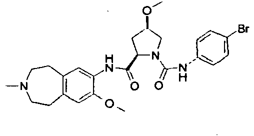 |
| **D** | 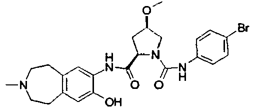 | **E** | 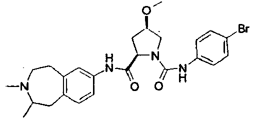 | **F** | 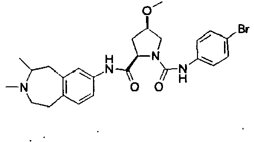 |
| **G** | 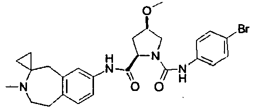 | **H** | 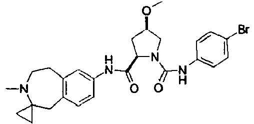 | **I** | 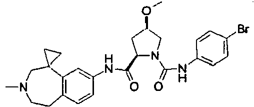 |
| **J** | 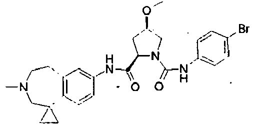 | **K** | 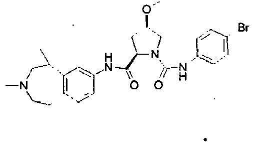 | **L** | 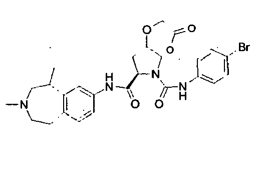 |
| **M** | 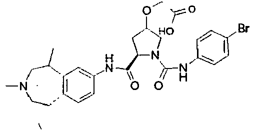 | **N** | 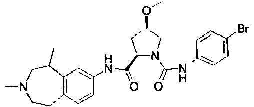 | **O** | 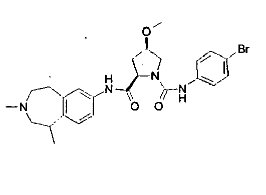 |

Die nachfolgenden Beispiele beschreiben die Herstellung pharmazeutischer Anwendungsformen, die als Wirkstoff eine beliebige Verbindung der allgemeinen Formel I enthalten.

### Beispiel A

### Trockenampulle mit 75 mg Wirkstoff pro 10 ml

### Zusammensetzung:

| | |
|---|---|
| Wirkstoff | 75.0 mg |
| Mannitol | 50.0 mg |
| Wasser für Injektionszwecke | ad 10.0 ml |

### Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

### Beispiel B

### Trockenampulle mit 35 mg Wirkstoff pro 2 ml

### Zusammensetzung:

| | |
|---|---|
| Wirkstoff | 35.0 mg |
| Mannitol | 100.0 mg |
| Wasser für Injektionszwecke | ad 2.0 ml |

### Herstellung:

Wirkstoff und Mannitol werden in Wasser gelöst. Nach Abfüllung wird gefriergetrocknet.
Die Auflösung zur gebrauchsfertigen Lösung erfolgt mit Wasser für Injektionszwecke.

### Beispiel C

### Tablette mit 50 mg Wirkstoff

### Zusammensetzung:

| | |
|---|---|
| (1) Wirkstoff | 50.0 mg |
| (2) Milchzucker | 98.0 mg |
| (3) Maisstärke | 50.0 mg |
| (4) Polyvinylpyrrolidon | 15.0 mg |
| (5) Magnesiumstearat | 2.0 mg |
| | 215.0 mg |

### Herstellung:

(1), (2) und (3) werden gemischt und mit einer wässrigen Lösung von (4) granuliert. Dem getrockneten Granulat wird (5) zugemischt. Aus dieser Mischung werden Tabletten gepresst, biplan mit beidseitiger Facette und einseitiger Teilkerbe.

Durchmesser der Tabletten: 9 mm.

### Beispiel D

### Tablette mit 350 mg Wirkstoff

### Zusammensetzung:

| | |
|---|---|
| (1) Wirkstoff | 350.0 mg |
| (2) Milchzucker | 136.0 mg |
| (3) Maisstärke | 80.0 mg |
| (4) Polyvinylpyrrolidon | 30.0 mg |
| (5) Magnesiumstearat | 4.0 mg |
| | 600.0 mg |

### Herstellung:

(1), (2) und (3) werden gemischt und mit einer wässrigen Lösung von (4) granuliert. Dem getrockneten Granulat wird (5) zugemischt. Aus dieser Mischung werden Tabletten gepresst, biplan mit beidseitiger Facette und einseitiger Teilkerbe.
Durchmesser der Tabletten: 12 mm.

### Beispiel E

### Kapseln mit 50 mg Wirkstoff

### Zusammensetzung:

| | |
|---|---|
| (1) Wirkstoff | 50.0 mg |
| (2) Maisstärke getrocknet | 58.0 mg |
| (3) Milchzucker pulverisiert | 50.0 mg |
| (4) Magnesiumstearat | 2.0 mg |
| | 160.0 mg |

### Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 3 abgefüllt.

### Beispiel F

### Kapseln mit 350 mg Wirkstoff

### Zusammensetzung:

| | |
|---|---|
| (1) Wirkstoff | 350.0 mg |
| (2) Maisstärke getrocknet | 46.0 mg |
| (3) Milchzucker pulverisiert | 30.0 mg |
| (4) Magnesiumstearat | 4.0 mg |
| | 430.0 mg |

### Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in HartgelatineSteckkapseln Größe 0 abgefüllt.

### Beispiel G

### Suppositorien mit 100 mg Wirkstoff

### 1 Zäpfchen enthält:

| | |
|---|---|
| Wirkstoff | 100.0 mg |
| Polyethylenglykol (M.G. 1500) | 600.0 mg |
| Polyethylenglykol (M.G. 6000) | 460.0 mg |
| Polyethylensorbitanmonostearat | 840.0 mg |
| | 2000.0 mg |

### Herstellung:

Das Polyethylenglykol wird zusammen mit Polyethylensorbitanmonostearat geschmolzen. Bei 40°C wird die gemahlene Wirksubstanz in der Schmelze homogen dispergiert. Es wird auf 38°C abgekühlt und in schwach vorgekühlte Suppositorienformen ausgegossen.

## Patentansprüche

1. Verbindungen der allgemeinen Formel (I) 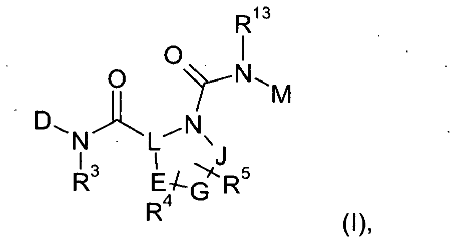 in denen
D ein substituiertes bicyclisches Ringsystem der Formel (II) 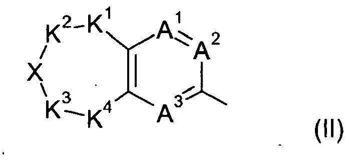 darstellt, in dem
K¹ und K⁴ jeweils unabhängig voneinander eine -CH₂-, -CHR^{7a}-, -CR^{7b}R^{7c}-, -C(=CH2)- oder eine -C(O)-Gruppe bedeuten, und wobei
R^{7a}/R^{7b}/R^{7c} jeweils unabhängig voneinander ein Fluoratom, eine Hydroxy-, C₁₅-Alkyloxy-, Amino-, C₁₋₅-Alkylamino-, Di-(C₁₋₅-alkyl)-amino-, C₃₋₅-Cycloalkylenimino-, C₁₋₅-Alkylcarbonylaminogruppe,
eine C₃₋₅-Cycloalkyl- oder eine C₁₋₅-Alkylgruppe, die durch 1-3 Fluoratome substituiert sein kann, eine Hydroxy-C₁₋₅-alkyl-, C₁₋₅-Alkyloxy-C₁₋₅-alkyl-, Amino-C₁₋₅-alkyl-, C₁₋₅-Alk-ylamino-C₁₋₅-alkyl-, Di-(C₁₋₅-alkyl)-amino-C₁₋₅-alkyl-, C₄₋₇-Cycloalkylenimino-C₁₋₅-alkyl-, Carboxy-C₀₋₅-alkyl-, C₁₋₅-Alkyloxycarbonyl-C₀₋₅-alkyl-, Aminocarbonyl-C₀₋₅-alkyl-, C₁₋₅-Alkylaminocarbonyl-C₀₋₅-alkyl-, Di-(C₁₋₅-alkyl)-aminocarbonyl-C₀₋₅-alkyl- oder eine C₄₋₇-Cycloalkyleniminocarbonyl-C₀₋₅-alkyl-gruppe bedeutet,
wobei nicht gleichzeitig beide Reste R^{7b}/R^{7c} über ein Heteroatom an das Ringkohlenstoffatom gebunden sein können, außer -C(R^{7b}R^{7c})- entspricht einer -CF₂-Gruppe, oder
R^{7a} eine durch Fluor-, Chlor- , Brom-, Methyl-, Methoxy-, Amino-oder Nitro-substituierte Phenyl- oder monocyclische Heteroarylgruppe bedeutet, oder
zwei Reste R^{7b}/R^{7c} zusammen mit dem Ringkohlenstoffatom einen 3-, 4-, 5-, 6- oder 7-gliedrigen gesättigten Carbocyclus oder einen Cyclopenten-, Cyclohexen-, Oxetan-, Azetidin-, Thietan-, Tetrahydrofuran-, Pyrrolidin-, Tetrahydrothiophen-, Tetrahydropyran-, Piperidin-, Pentamethylensulfid-, Hexamethylenimin-, 1,3-Dioxolan-, 1,4-Dioxan-, Hexahydropyridazin-, Piperazin-, Thiomorpholin-, Morpholin-, 2-Imidazolidinon-, 2-Oxazolidinon-, Tetrahydro-2(1H)-pyrimidinon-oder [1,3]Oxazinan-2-on-ring bilden können,
wobei dessen Methylengruppen durch 1-2 C₁₋₃-Alkyl- oder CF₃-gruppen substituiert sein können, und/oder dessen Methylengruppen, sofern sie nicht an ein Heteroatom gebunden sind, durch 1-2 Fluoratome substituiert sein können, und/oder
bei dem eine -CH₂-Gruppe neben einem N-Atom durch eine -CO-Gruppe ersetzt sein kann, und/oder
dessen Iminogruppen jeweils durch eine C₁₋₃-Alkyl- oder C₁₃-Alkylcarbonyl-gruppe substituiert sein können, und/oder bei dem das Schwefelatom zu einer Sulfoxid oder Sulfongruppe oxidiert sein kann,
K² und K³ jeweils unabhängig voneinander eine -CH₂-, -CHR^{8a}-, -CR^{8b}R^{8c}-oder eine -C(O)-Gruppe bedeuten, wobei
R^{8a}/R^{ab}/R^{8c} jeweils unabhängig voneinander eine C₃₋₅-Cycloalkyl- oder eine C₁₋₅-Alkylgruppe, die durch 1-3 Fluoratome substituiert sein kann, eine Hydroxy-C₁₋₅-alkyl-, C₁₋₅-Alkyloxy-C₁₋₅-alkyl-, Amino-C₁₋₅-alkyl-, C₁₋₅-Alkylamino-C₁₋₅-alkyl-, Di-(C₁₋₅-alkyl)-amino-C₁₋₅-alkyl-, 6₄₋₇-Cycloalkylenimino-C₁₋₅-alkyl-, Carboxy-C₀₋₅-alkyl-, C₁₋₅-Alkyloxycarbonyl-C₀₋₅-alkyl-, Aminocärbonyl-C₀₋₅-alkyl-, C₁₋₅-Alkylaminocarbonyl-C₀₋₅-alkyl-, Di-(C₁₋₅-alkyl)-aminocarbonyl-C₀₋₅-alkyl- oder eine C₄₋₇-Cycloalkyleniminocarbonyl-C₀₋₅-alkyl-gruppe bedeutet,
oder zwei Reste R^{8b}/R^{8c} zusammen mit dem Ringkohlenstoffatom einen 3-, 4-, 5-, 6- oder 7-gliedrigen gesättigten Carbocyclus oder einen Cyclopenten-, Cyclohexen-, Oxetan-, Azetidin-, Thietan-, Tetrahydrofuran-, Pyrrolidin-, Tetrahydrothiophen-, Tetrahydropyran-, Piperidin-, Pentamethylensulfid-, Hexamethylenimin-, Hexahydropyridazin-, Tetrahydro-2(1H)-pyrimidinon-, [1,3]Oxazinan-2-on-ring bilden können,
wobei dessen Methylengruppen durch 1-2 C₁₋₃-Alkyl- oder CF₃-gruppen substituiert sein können, und/oder dessen Methylengruppen, sofern sie nicht an ein Heteroatom gebunden sind, durch 1-2 Fluoratome substituiert sein können, und/oder
bei dem eine -CH₂-Gruppe neben einem Stickstoffatom durch eine -CO-Gruppe ersetzt sein kann, und/oder dessen Iminogruppen jeweils durch eine C₁₋₃-Alkyl- oder C₁-₃-Alkylcarbonyl-gruppe substituiert sein können, und/oder
bei dem das Schwefelatom zu einer Sulfoxid- oder Sulfongruppe oxidiert sein kann,
mit der Massgabe, dass ein durch R^{8b} oder R^{8c} eingebrachtes Heteroatom nicht durch nur ein Kohlenstoffatom von X in Formel (I) entfernt sein darf, und
insgesamt in Formel (II) maximal vier Reste ausgewählt aus R^{7a}, R^{7b}, R^{7c}, R^{8a}, R^{ab} und R^{8c} vorhanden sein dürfen, und
X ein Sauerstoff- oder Schwefelatom, eine CF₂-, Sulfen-, Sulfon- oder eine NR¹-Gruppe bedeutet, in der
R¹ ein Wasserstoffatom oder eine Hydroxy-, C₁₋₃-Alkyloxy-, Amino-, C₁₋₃-Alkylamino-, Di-(C₁₋₃-alkyl)-amino-, eine C₁₋₅-Alkyl-, C₂₋₅-Alkenyl-CH₂-, C₂₋₅-Alkinyl-CH₂-, C₃₋₆-Cycloalkyl-, C₄₋₆-Cycloalkenyl-, Oxetan-3-yl-, Tetrahydrofuran-3-yl-, Benzyl-, C₁₋₅-Alkyl-carbonyl-, Trifluormethylcarbonyl-, C₃₋₆-Cycloalkyl-carbonyl-, C₁₋₅-Alkyl-sulfonyl-, C₃₋₆-Cycloalkyl-sulfonyl-, Aminocarbonyl-, C₁₋₅-Alkylaminocarbonyl-, Di-(C₁₋₅-alkyl)-aminocarbonyl-, C₁₋₅-Alkyloxycarbonyl-, C₄₋₇-Cycloalkyleniminocarbonyl-gruppe bedeutet,
wobei die in den voranstehend genannten Gruppen befindlichen Methylen- und Methylgruppen zusätzlich durch eine C₁₋₃Alkyl-, Carboxy-, C₁₋₅-Alkoxycarbonyl-gruppe substituiert sein können, oder durch eine Hydroxy-, C₁₋₅-Alkyloxy-, Amino-, C₁₋₅-Alkylamino-, C₁₅-Dialkylamino- oder C₄₋₇Cycloalkyleniminogruppe substituiert sein können, sofern die Methylen- oder Methylgruppen nicht direkt an ein Heteroatom aus der Gruppe O, N oder S gebunden sind, und/oder ein bis drei Wasserstoffatome durch Fluoratome ersetzt sein können, sofern die Methylen- oder Methylgruppen nicht direkt an ein Heteroatom aus der Gruppe O, N oder S gebunden sind,
und in dem
A¹ entweder N oder CR¹⁰ bedeutet,
A² entweder N oder CR¹¹ bedeutet,
A³ entweder N oder CR¹² bedeutet,
wobei R¹⁰, R¹¹ und R¹² jeweils unabhängig voneinander
ein Wasserstoff-, Fluor-, Chlor-, Brom- oder lodatom, oder eine C₁₅-Alkyl-, CF₃-, C₂₋₅ -Alkenyl-, C₂₋₅-Alkinyl-, eine Cyano-, Carboxy-, C₁₋₅-Alkyloxycarbonyl-, Hydroxy-, C₁₋₃-Alkyloxy-, CF₃O-, CHF₂O-, CH₂FO-, Amino-, C₁₋₅-Alkylamino-, Di-(C₁₋₅-Alkyl)-amino- oder C₄₇-Cycloalkylenimino-gruppe bedeuten, oder
D eine der vier Gruppen (II-1), (II-2), (II-3), (II-4), (II-5) oder (II-6) darstellt 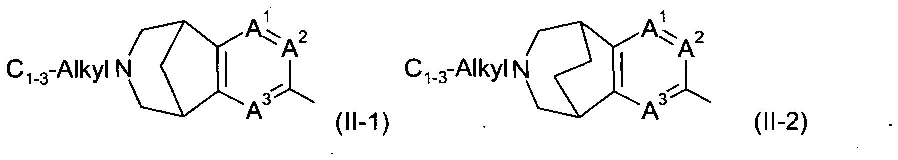 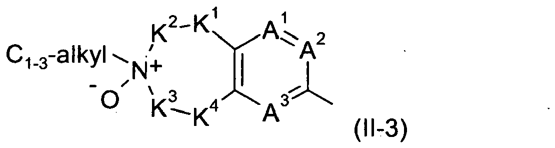 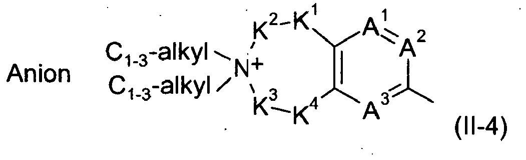 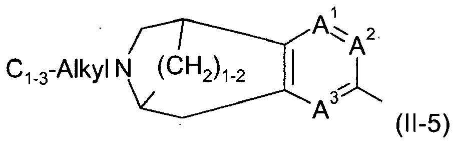 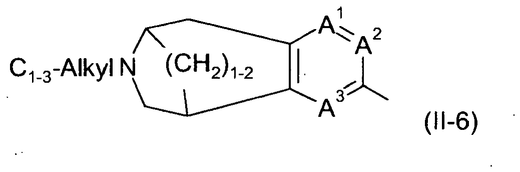 in der die Reste A1, A2, A3, K1, K2, K3, K4 wie oben definiert sind, und das Anion in (II-4) ein Fluorid, Chlorid, Bromid, Iodid, Sulfat, Hydrogensulfat, Phosphat, Hexafluorphosphat, Hydrogenphosphat, Benzoat, Salicylat, Succinat, Citrat oder Tartrat bedeutet,
R³ ein Wasserstoffatom oder eine C₁₋₃-Alkylgruppe bedeutet, und
-L-E-G-J- eine -C-C-C-C- oder -C-C=C-C-Gruppe bedeutet, und
R⁴ ein Wasserstoffatom oder
eine geradkettige oder verzweigte C₁₋₆-Alkyl-, C₂₋₆-Alkenyl- oder C₂₋₆-Alkinylgruppe bedeutet,
wobei die Wasserstoffatome der Methylen- und/oder Methylfragmente der geradkettigen oder verzweigten C₁₋₆-Alkyl-, C₂₋₆-Alkenyl- oder C₂₋₆-Alkinylgruppe gegebenenfalls ganz oder teilweise durch Fluoratome ersetzt sein können, und/oder
wobei die Wasserstoffatome der Methylen- und/oder Methylfragmente der geradkettigen oder verzweigten C₁₋₆-Alkyl-, C₂₋₆-Alkenyl- oder C₂₋₆-Alkinylgruppe gegebenenfalls jeweils unabhängig voneinander durch ein bis zwei Substituenten ausgewählt aus einer C₃₋₅-Cycloalkylgruppe, einer Nitril-, Hydroxy-oder C₁₋₅-Alkyloxygruppe, wobei die Wasserstoffatome der C₁₋₅-Alkyloxygruppe gegebenenfalls ganz oder teilweise durch Fluoratome ersetzt sein können, einer Allyloxy-, Propargyloxy-, Benzyloxy-, C₁₋₅-Alkylcarbonyloxy-, C₁₋₅-Alkyloxycarbonyloxy-, Carboxy-C₁₋₅-alkyloxy-, C₁₋₅-Alkyloxycarbonyl-C₁₋₅-alkyloxy-, Mercapto-, C₁₋₅-Alkylsulfanyl-, C₁₋₅-Alkylsulfinyl-, C₁₋₅-Alkylsulfonyl-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl-, C₄₋₇-Cycloalkyleniminocarbonyl-, Aminosulfonyl-, C₁₋₅-Alkylaminosulfonyl-, Di-(C₁₋₅-alkyl)-aminosulfonyl-, C₄₋₇-Cycloalkyleniminosulfonyl-, Amino-, C₁₋₅-Alkylamino-, Di-(C₁₋₅-alkyl)-amino-, C₁₋₅-Alkylcarbonylamino-, C₁₋₅-Alkyl-sulfonylamino-, N-(C₁₋₅-Alkylsulfonyl)-C₁₋₅-alkylamino-C₃₋₆-Cycloalkylcarbonylaminogruppe, oder einer Morpholinyl-, Thiomorpholinyl-, Pyrrolidinyl-, Piperidinyl-, Piperazinyl-, Tetrahydrofuranyl-, Tetrahydropyranyl-gruppe substituiert sein können, wobei die vorgenannten Carbo- und Hetero-cyclen im Ring jeweils durch 1-4 C₁₋₃-Alkyl- oder C₁₋₃-Alkylcarbonylgruppen oder jeweils durch 1-2 Oxogruppen substituiert sein können, und/oder
wobei die Wasserstoffatome der sp²-hybridisierten Kohlenstoffatome der geradkettigen oder verzweigten C₂₋₆-Alkenyl-gruppe gegebenenfalls ganz oder teilweise durch Fluoratome ersetzt sein können, oder
eine Nitril-, Carboxy-, Aminocarbonyl-, C₁₋₅-Alkylaminocarbonyl-, C₃₋₆-Cycloalkylaminocarbonyl-, Di-(C₁₋₅-alkyl)-aminocarbonyl-, C₁₋₅-Alkyloxycarbonyl- oder eine C₄₋₇-Cycloalkyleniminocarbonylgruppe in der gegebenenfalls eine Methylengruppe durch ein Sauerstoff-, Schwefel- oder C₀₋₃-Alkyl-substituiertes Stickstoffatom ersetzt sein kann, oder
eine Phenyl-, mono- oder bicyclische Heteroaryl-, Phenyl-C₁₋₅-alkyl- oder mono- oder bicyclische Heteroaryl-C₁₋₅-alkylgruppe,
die im Phenyl- oder Heteroarylteil gegebenenfalls ein- bis dreifach durch gleiche oder unterschiedliche Substituenten ausgewählt aus der Gruppe bestehend aus Fluor-, Chlor-, Brom- und lodatomen, und C₁₋₅-Alkyl-, Trifluormethyl-, Amino-, C₁₋₅-alkyl-amino-, Di-(C₁₋₅-alkyl)-amino-, Hydroxy-, C₁₋₅-Alkyloxy-Mono-, Di- oder Trifluormethoxy-, Carboxy- und C₁₋₅-Alkyloxycarbonylgruppe, substituiert sein kann,
bedeutet, und
wenn -L-E-G-J- eine -C-C-C-C-Gruppe bedeutet, kann R⁴ an E oder G auch ein Fluoratom oder eine Hydroxy-, C₂₋₅-Alkenyl-oxy-, C₂₋₅-Alkinyl-oxy-, C₁₋₅-Alkyl-oxy-, C₃₋₆-Cycloalkyl-oxy-, C₁₋₅-Alkylaminocarbonyloxy-, Di(C₁₋₅-alkyl)aminocarbonyloxy- oder C₄₋₇-Cycloalkyleniminocarbonyloxy-, Phenyl-C₀₋₃-alkyloxy-, Heteroaryl-C₀₋₃-alkyl-oxy-, Amino-, C₁₋₅-Alkylamino-, Di-(C₁₋₅-alkyl)-amino-, C₄₋₇-Cycloalkylenimino-, C₁₋₃-Acylamino-, (C₁₋₃-Acyl)C₁₋₃-alkylamino-, C₁₋₅-Alkyloxycarbonylamino-, C₁₅-Alkylaminocarbonylamino-, Di(C₁₋₅-alkyl)aminocarbonylamino- oder eine C₄₋₇-Cycloalkyleniminocarbonylamino-gruppe darstellen,
wobei die in den vorgenannten Alkyl- oder Cycloalkylresten vorhandenen Methyl oder Methylengruppen jeweils unabhängig voneinander durch einen Substituenten ausgewählt aus der Gruppe Morpholinyl-, Thiomorpholinyl-, Pyrrolidinyl-, Piperidinyl-, Piperazinyl-, Tetrahydrofuranyl-, Tetrahydropyranyl, Dimethylaminocarbonyl-, C₁₋₃Alkyloxycarbonyl-, Carboxy-, Methyl-, Hydroxy-, Methoxy- oder Amino- substituiert sein können,
zeit der Maßgabe, dass zwei Heteroatome aus der Gruppe Sauerstoff und Stickstoff durch genau eine gegebenenfalls substituierte -CH₂-Gruppe voneinander getrennt sind, und/oder
dass zwei Atome eine -O-O- oder -S-O-Bindung bilden, ausgeschlossen ist, und
R⁵ ein Wasserstoffatom oder eine C₁₋₅Alkyl-, C₂₋₅Alkenyl- oder C₂₋₅Alkinylgruppe bedeutet, oder sofern R⁵ mit E oder G verknüpft ist auch eine Hydroxy- oder Methoxy-gruppe darstellen kann, oder
R⁴ und R⁵ sofern sie an das selbe Kohlenstoffatom gebunden sind, eine-C(O)- Gruppe,oder eine -C(F₂)- Gruppe bilden können, oder
R⁴ und R⁵ sofern sie an das selbe Kohlenstoffatom oder an zwei benachbarte Kohlenstoffatome gebunden sind,
eine C₃₋₇-Cycloalkyl- oder C₅₋₇-Cycloalkenylgruppe bilden können,
wobei eine der Methylengruppen dieser C₄₋₇-Cycloalkylgruppe durch ein Sauerstoff- oder Schwefelatom oder eine -NH-, -N(C₁₋₅-Alkyl)-, -N(C₁₋₄-Alkylcarbonyl)- oder eine Carbonyl-, Sulfinyl- oder Sulfonylgruppe ersetzt sein kann, und/oder
wobei zwei direkt benachbarte Methylengruppen dieser C₄₋₇-Cycloalkylgruppe zusammen durch eine -C(O)NH-,-C(O)N(C₁₋₅-Alkyl)-, -S(O)₂NH-, oder -S(O)₂N(C₁₋₅-Alkyl)-Gruppe ersetzt sein können, und/oder
wobei 1 bis 3 Kohlenstoffatome einer C₃₋₇-Cycloalkylgruppe gegebenenfalls unabhängig voneinander durch jeweils ein oder zwei Fluoratome oder eine oder zwei C₁₋₅-Alkyl-gruppen oder eine Hydroxy-, C₁₋₅-Alkyloxy-, C₁₋₅-Alkylcarbonyloxy-, Amino-, C₁₋₅-Alkylamino-, Di-(C₁₋₅-alkyl)-amino-, C₄₋₇-Cycloalkylenimino-, C₁₋₅-Alkylcarbonylamino-, C₃₋₆-Cycloalkylcarbonylamino-, Nitril-, Carboxy-C₁₋₅-alkyl-, C₁₋₅-Alkyloxycarbonyl-C₁₋₅-alkyl-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl- oder
C₄₋₇-Cycloalkyleniminocarbonylgruppe substituiert sein können,
mit der Maßgabe, dass eine solche, zusammen aus R⁴ und R⁵ gebildete C₃₋₇-Cycloalkylgruppe,
in der zwei Heteroatome im Cyclus aus der Gruppe Sauerstoff und Stickstoff durch genau eine gegebenenfalls substituierte -CH₂-Gruppe voneinander getrennt sind, und/oder
in der zwei Atome im Ring eine -O-O- oder -S-O-Bindung bilden,
ausgeschlossen ist,
R¹³ ein Wasserstoffatom oder eine C₁₋₅-Alkylgruppe bedeutet,
M einen gegebenenfalls durch R² und R⁶ substituierten Phenyl-, Thienyl- oder Pyridylring bedeutet, in dem
R² ein Fluor-, Chlor-, Brom- oder Jod-atom oder eine Methyl-, Ethyl-, Propyl-, Isopropyl-, Vinyl-, Methoxy-, Ethinyl-, Cyano- oder -C(O)NH₂-Gruppe darstellt, und
R⁶ ein Wasserstoff-, Fluor-, Chlor-, Brom- oder Jod-atom oder eine Hydroxy-, Methoxy-, Trifluormethoxy-, eine gegebenenfalls durch Fluoratome substituierte C₁₋₃-Alkyl-, Cyano-, Amino-, oder NH₂C(O)-Gruppe darstellt,
wobei, soweit nichts anderes erwähnt wurde, unter dem voranstehend in den Definitionen erwähnten Ausdruck "Heteroarylgruppe" eine monocyclische 5- oder 6-gliedrige Heteroarylgruppe zu verstehen ist, wobei
die 6-gliedrige Heteroarylgruppe ein, zwei oder drei Stickstoffatome, und die 5-gliedrige Heteroarylgruppe eine gegebenenfalls durch eine C₁₋₃-Alkylgruppe substituierte Iminogruppe, ein Sauerstoff- oder Schwefelatom, oder
eine gegebenenfalls durch eine C₁₋₃-Alkylgruppe substituierte Iminogruppe oder ein Sauerstoff- oder Schwefelatom und zusätzlich ein oder zwei Stickstoffatome, oder
eine gegebenenfalls durch eine C₁₋₃-Alkylgruppe substituierte Iminogruppe und drei Stickstoffatome,
enthält,
und außerdem an die vorstehend erwähnten monocyclischen Heteroarylgruppen über zwei benachbarte Kohlenstoffatome ein gegebenenfalls durch ein Fluor-, Chlor- oder Bromatom, eine C₁₋₃-Alkyl-, Hydroxy-, C₁₋₃-Alkyloxygruppe, Amino-, C₁₋₃-Alkylamino-, Di-(C₁₋₃-alkyl)-amino- oder C₃₋₆-Cycloalkyleniminogruppe substituierter Phenylring ankondensiert sein kann,
und die Bindung über ein Stickstoffatom oder über ein Kohlenstoffatom des heterocyclischen Teils oder eines ankondensierten Phenylrings erfolgt,
und wobei, soweit nichts anderes erwähnt wurde, unter dem voranstehend in den Definitionen erwähnten Ausdruck "Halogenatom" ein Atom aus der Gruppe Fluor, Chlor, Brom und lod zu verstehen ist,
und wobei die in den voranstehend erwähnten Definitionen enthaltenen Alkyl-, Alkenyl-, Alkinyl- und Alkyloxygruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen, sofern nichts anderes erwähnt, ganz oder teilweise durch Fluoratome ersetzt sein können,
deren Tautomere, deren Enantiomere, deren Diastereomere, deren Gemische und deren Salze.

2. Verbindungen der allgemeinen Formel (I) nach Anspruch 1, in denen
D ein substituiertes bicyclisches Ringsystem der Formel (II) 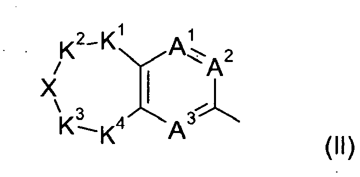 darstellt, in dem
K¹ und K⁴ jeweils unabhängig voneinander eine -CH₂-, -CHR^{7a}-, -CR^{7b}R^{7c}-, -C(=CH2)- oder eine -C(O)-Gruppe bedeuten, und wobei
R^{7a}/R^{7b}/R^{7c} jeweils unabhängig voneinander ein Fluoratom, eine Hydroxy-, C₁₅-Alkyloxy-, eine C₁₋₅-Alkylgruppe bedeutet,
wobei nicht gleichzeitig beide Reste R^{7b}/R^{7c} über ein Heteroatom an das Ringkohlenstoffatom gebunden sein können, außer-C(R^{7b}R^{7c})- entspricht einer -CF₂-Gruppe,
oder zwei Reste R^{7b}/R^{7c} zusammen mit dem Ringkohlenstoffatom einen Cyclopropylring bilden können,
K² und K³ jeweils unabhängig voneinander eine -CH₂-, -CHR^{8a}-, -CR^{8b}R^{ac}-oder eine -C(O)- Gruppe bedeuten, wobei
R^{8a}/R^{ab}/R^{8c} jeweils unabhängig voneinander eine C₁₋₅-Alkylgruppe bedeutet, oder zwei Reste R^{8b}/R^{8c} zusammen mit dem Ringkohlenstoffatom einen Cyclopropylring bilden können,
und
insgesamt in Formel (II) maximal vier Reste ausgewählt aus R^{7a}, R^{7b}, R^{7c}, R^{8a}, R^{8b} und R^{8c} vorhanden sein dürfen, und
X ein Sauerstoff- oder Schwefelatom, eine Surfen-, Sulfon- oder eine NR¹-Gruppe bedeutet, in der
R¹ ein Wasserstoffatom oder eine Hydroxy-, C₁₋₃-Alkyloxy-, Amino-, C₁₋₃-Alkylamino-, Di-(C₁₋₃-alkyl)-amino-, eine C₁₋₅-Alkyl-, C₂₋₅-Alkenyl-CH₂-, C₂₋₅-Alkinyl-CH₂- oder eine C₃₋₆-Cycloalkylgruppe bedeutet,
und in dem
A¹ entweder N oder CR¹⁰ bedeutet,
A² entweder N oder CR¹¹ bedeutet,
A³ entweder N oder CR¹² bedeutet,
wobei R¹⁰, R¹¹ und R¹² jeweils unabhängig voneinander
ein Wasserstoff-, Fluor-, Chlor-, Brom- oder Iodatom, oder eine C₁₅-Alkyl-, CF₃-, eine Cyano-, Carboxy-, C₁₋₅-Alkyloxycarbonyl-, Hydroxy-, C₁₋₃-Alkyloxy-, CF₃O-, CHF₂O-, CH₂FO-, Amino-, C₁₋₅-Alkylamino-, Di-(C₁₋₅-Alkyl)-amino- oder C₄₋₇-Cycloalkyleniminogruppe bedeuten.

3. Verbindungen der allgemeinen Formel (I) nach Anspruch 1 oder 2, in denen
X eine NR¹-Gruppe bedeutet, in der
R¹ ein Wasserstoffatom oder eine C₁₋₅-Alkyl-, Allyl- oder Cyclopropylgruppe bedeutet, und
A¹ CR¹⁰ bedeutet,
A² CR¹¹ bedeutet,
A³ CR¹² bedeutet,
wobei R¹⁰, R¹¹ und R¹² jeweils unabhängig voneinander
ein Wasserstoff-, Fluor- oder Chloratom, oder eine Methyl-, CF₃-, Hydroxy-, Methoxy-, CF₃O-, CHF₂O-, CH₂FO-Gruppe bedeuten.

4. Verbindungen der allgemeinen Formel (I) nach einem der Ansprüche 1, 2 oder 3, in denen
R⁴ ein Wasserstoffatom oder
eine geradkettige oder verzweigte C₁₋₆-Alkylgruppe bedeutet, wobei die Wasserstoffatome der Methylen- und/oder Methylfragmente der geradkettigen oder verzweigten C₁₋₆-Alkylgruppe gegebenenfalls ganz oder teilweise durch Fluoratome ersetzt sein können, und/oder
wobei die Wasserstoffatome der Methylen- und/oder Methylfragmente der geradkettigen oder verzweigten C₁₋₆-Alkylgruppe gegebenenfalls jeweils unabhängig voneinander durch einen Substituenten ausgewählt aus einer Hydroxy-, C₁₋₅-Alkyloxy-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl-, C₄₋₇-Cycloalkyleniminocarbonyl-, Amino-, C₁₋₅-Alkylamino-, Di-(C₁₋₅-alkyl)-amino-, C₁₋₅-Alkylcarbonylamino-, C₁₋₅-Alkylsulfonylamino-, N-(C₁₋₅-Alkylsulfonyl)-C₁₋₅-alkylamino-, C₃₋₆-Cycloalkylcarbonylaminogruppe substituiert sein können, oder
eine Nitril-, Carboxy-, Aminocarbonyl-, C₁₋₅-Alkylaminocarbonyl-, C₃₋₆-Cycloalkylaminocarbonyl-, Di-(C₁₋₅-alkyl)-aminocarbonyl-, C₁₋₅-Alkyloxycarbonyl- oder eine C₄₋₇-Cycloalkyleniminocarbonylgruppe in der gegebenenfalls eine Methylengruppe durch ein Sauerstoff-, Schwefel- oder C₀₋₃-Alkyl-substituiertes Stickstoffatom ersetzt sein kann, bedeutet, und
wenn -L-E-G-J- eine -C-C-C-C-Gruppe bedeutet, kann R⁴ an E oder G auch ein Fluoratom oder eine Hydroxy-, Methoxy-, C₂₋₅-Alkenyl-oxy-, C₂₅-Alkinyl-oxy-, C₁₋₅-Alkyl-oxy-, C₃₋₆-Cycloalkyl-oxy-, C₁₋₅-Alkylaminocarbonyloxy-, Di(C₁₋₅-alkyl)aminocarbonyloxy- oder C₄₋₇-Cycloalkyleniminocarbonyloxy-, Phenyl-C₀₋₃-alkyloxy-, Amino-, C₁₋₅-Alkylamino-, Di-(C₁₋₅-alkyl)-amino-, C₄₋₇-Cycloalkylenimino-, C₁₋₃-Acylamino-, (C₁₋₃-Acyl)C₁₋₃-alkylamino-, C₁₋₅-Alkyloxycarbonylamino-, C₁₅-Alkylaminocarbonylamino-, Di(C₁₋₅-alkyl)aminocarbonylamino- oder eine C₄₋₇-Cycloalkyleniminocarbonylamino-gruppe darstellen,
wobei die in den vorgenannten Alkyl- oder Cycloalkylresten vorhandenen Methyl oder Methylengruppen jeweils unabhängig voneinander durch einen Substituenten ausgewählt aus der Gruppe Dimethylaminocarbonyl-, C₁₋₃Alkyloxycarbonyl-, Carboxy-, Methyl-, Hydroxy-, Methoxy- oder Amino- substituiert sein können,
mit der Maßgabe, dass zwei Heteroatome aus der Gruppe Sauerstoff und Stickstoff durch genau eine gegebenenfalls substituierte -CH₂-Gruppe voneinander getrennt sind, und/oder
dass zwei Atome eine -O-O- oder -S-O-Bindung bilden, ausgeschlossen ist, und
R⁵ ein Wasserstoffatom, eine Allyl- oder eine C₁₋₅ Alkylgruppe bedeutet, oder sofern R⁵ mit E oder G verknüpft ist auch eine Hydroxy- oder Methoxy-gruppe darstellen kann, oder
R⁴ und R⁵ sofern sie an das selbe Kohlenstoffatom gebunden sind,
eine -C(O)- Gruppe,oder eine -C(F₂)- Gruppe bilden können, oder
R⁴ und R⁵ sofern sie an das selbe Kohlenstoffatom oder an zwei benachbarte Kohlenstoffatome gebunden sind,
eine C₃₋₇-Cycloalkyl-gruppe bilden können,
wobei eine der Methylengruppen dieser C₄₋₇-Cycloalkylgruppe durch ein Sauerstoff- oder Schwefelatom oder eine -NH-, -N(C₁₋₅-Alkyl)-, -N(C₁₋₄-Alkylcarbonyl)- oder eine Carbonyl-, Sulfinyl- oder Sulfonylgruppe ersetzt sein kann, und/oder
wobei zwei direkt benachbarte Methylengruppen dieser C₄₋₇-Cycloalkylgruppe zusammen durch eine -C(O)NH-,-C(O)N(C₁₋₅-Alkyl)-, -S(O)₂NH-, oder -S(O)₂N(C₁₋₅-Alkyl)-Gruppe ersetzt sein können.

5. Verbindungen der allgemeinen Formel (I) nach einem der Ansprüche 1, 2, 3 oder 4, in denen
-L-E-G-J-. eine -C-C-C-C-Gruppe bedeutet.

6. Verbindungen der allgemeinen Formel (I) nach einem der Ansprüche 1, 2, 3, 4 oder 5, in denen
D einen substituierten Benzazepinylrest der Formel (IIa) 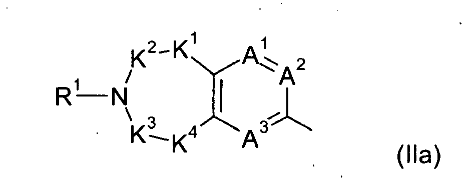 darstellt, in dem
K¹ und K⁴ jeweils unabhängig voneinander eine -CH₂-, -CHR^{7a}-, -CR^{7b}R^{7c}- oder eine -C(O)-Gruppe bedeutet, wobei
R^{7a} eine C₁₋₅-Alkyl-, Hydroxy- oder C₁₋₃-alkyloxy-gruppe Bedeutet, und
R^{7b}/R^{7c} jeweils unabhängig voneinander eine Hydroxy-, C₁₋₅-Alkyloxy- oder eine C₁₋₅-Alkylgruppe bedeutet, wobei nicht gleichzeitig beide Reste R^{7b}/R^{7c} über ein Sauerstoffatom an das Ringkohlenstoffatom gebunden sein können,
oder zwei Reste R^{7b}/R^{7c} zusammen mit dem Ringkohlenstoffatom einen Cyclopropylring bilden können, und
K² und K³ jeweils unabhängig voneinander eine -CH₂-, -CHR^{8a}- oder-CR^{8b}R^{8c}-Gruppe bedeuten, wobei
R^{8a}/R^{8b}/R^{8c} jeweils unabhängig voneinander eine C₁₋₃-Alkylgruppe bedeutet, und
insgesamt in Formel (II) maximal vier Reste ausgewählt aus R^{7a}, R^{7b}, R^{7c}, R^{8a}, R^{8b} und R^{8c} vorhanden sein dürfen, und
R¹ ein Wasserstoffatom oder eine C₁₋₅-Alkyl-, Allyl- oder Cyclopropylgruppe bedeutet, und
A¹ CR¹⁰ bedeutet,
A² CR¹¹ bedeutet,
A³ CR¹² bedeutet,
wobei R¹⁰, R¹¹ und R¹² jeweils unabhängig voneinander
ein Wasserstoff-, Fluor- oder Chloratom, oder eine Methyl-, CF₃-, Hydroxy-, Methoxy-, CF₃O-, CHF₂O-, CH₂FO-Gruppe bedeuten, und
-L-E-G-J- eine -C-C-C-C-Gruppe bedeutet, und
R³ ein Wasserstoffatom bedeutet, und
R⁴ ein Wasserstoffatom oder
eine geradkettige oder verzweigte C₁₋₃-Alkylgruppe bedeutet, wobei die Wasserstoffatome der Methylen- und/oder Methylfragmente der geradkettigen oder verzweigten C₁₋₆-Alkylgruppe gegebenenfalls unabhängig voneinander durch einen Substituenten ausgewählt aus einer Hydroxy-, C₁₋₅-Alkyloxy-, Carboxy-, C₁₋₅-Alkyloxycarbonyl-gruppe substituiert sein können, oder
eine CF3-, Nitril-, Carboxy-, Aminocarbonyl-, C₁₋₅-Alkylaminocarbonyl-, C₃₋₆-Cycloalkylaminocarbonyl-, Di-(C₁₋₅-alkyl)-aminocarbonyl-, C₁₋₅-Alkyloxycarbonyl- oder eine C₄₋₇-Cycloalkyleniminocarbonylgruppe bedeutet, oder
wenn R⁴ an E oder G angebunden ist auch ein Fluoratom oder eine Hydroxy-, Methoxy-, C₂₋₅-Alkenyl-oxy-, C₂₋₅-Alkyl-oxy-, Methoxyethoxy-, HOCH₂CH(OH)CH₂oxy-, C₃₋₆-Cycloalkyl-oxy-, C₁₋₅-Alkylaminocarbonyloxy-, Di(C₁₋₅-alkyl)aminocarbonyloxy- oder C₄₋₇-Cycloalkyleniminocarbonyloxy-gruppe darstellen kann,
R⁵ ein Wasserstoffatom oder eine C₁₋₅Alkylgruppe bedeutet, oder sofern R⁵ mit E oder G verknüpft ist auch eine Hydroxy- oder Methoxy-gruppe darstellen kann, oder
R⁴ und R⁵ sofern sie an das selbe Kohlenstoffatom gebunden sind, eine C=O- oder eine -CF₂-Gruppe bedeuten können, und
R¹³ ein Wasserstoffatom bedeutet,
M einen
substituierten Phenylring 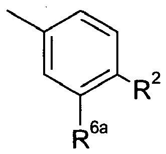 oder einen substituierten Pyridylring 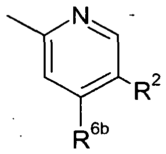
bedeutet, in dem
R² ein Fluor-, Chlor-, Bromatom, eine Methoxy- oder Ethinyl-Gruppe darstellt, und
R^{6a} ein Wasserstoff- oder Fluoratom darstellt und
R^{6b} ein Wasserstoffatom darstellt.

7. Physiologisch verträgliche Salze der Verbindungen gemäß einem der Ansprüche 1 bis 6.

8. Arzneimittel, enthaltend eine Verbindung nach mindestens einem der Ansprüche 1 bis 6 oder ein physiologisch verträgliches Salz gemäß Anspruch 7, neben gegebenenfalls einem oder mehreren inerten Trägerstoffen und/oder Verdünnungsmitteln.

9. Verwendung einer Verbindung nach mindestens einem der Ansprüche 1 bis 6 oder eines physiologisch verträglichen Salzes gemäß Anspruch 7 zur Herstellung eines Arzneimittels mit einem inhibitorischen Effekt auf Faktor Xa und/oder einem inhibitorischen Effekt auf verwandte Serinproteasen.

10. Verfahren zur Herstellung eines Arzneimittels gemäß Anspruch 8, **dadurch gekennzeichnet, dass** auf nichtchemischem Wege eine Verbindung nach mindestens einem der Ansprüche 1 bis 6 oder ein physiologisch verträgliches Salz gemäß Anspruch 7 in einen oder mehrere inerte Trägerstoffe und/oder Verdünnungsmittel eingearbeitet wird.

## Claims

1. Compounds of general formula (I) 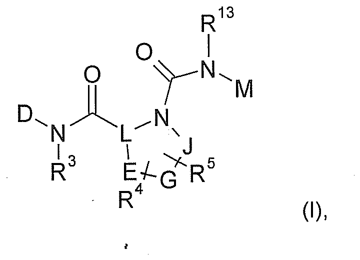 wherein
D denotes a substituted bicyclic ring system of formula (II) 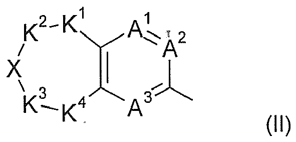 wherein
K¹ and K⁴ each independently of one another denote a -CH₂, -CHR^{7a}, -CR^{7b}R^{7c}, -C(=CH2) or a -C(O) group and wherein
R^{7a}/R^{7b}/R^{7c} each independently of one another denote a fluorine atom, a hydroxy, C₁₋₅-alkyloxy, amino, C₁₋₅-alkylamino, di-(C₁₋₅-alkyl)-amino, C₃₋₅-cycloalkyleneimino, C₁₋₅-alkylcarbonylamino group, a C₃₋₅-cycloalkyl or a C₁₋₅-alkyl group which may be substituted by 1-3 fluorine atoms, a hydroxy-C₁₋₅-alkyl, C₁₋₅-alkyloxy-C₁₋₅-alkyl, amino-C₁₋₅-alkyl, C₁₋₅-alkylamino-C₁₋₅-alkyl, di-(C₁₋₅-alkyl)-amino-C₁₋₅-alkyl, C₄₋₇-cycloalkyleneimino-C₁₋₅-alkyl, carboxy-C₁₋₅-alkyl, C₁₋₆-alkyloxycarbonyl-C₀₋₅-alkyl, aminocarbonyl-C₀₋₅-alkyl, C₁₋₅-alkylaminocarbonyl-C₀₋₅-alkyl, di-(C₁₋₅-alkyl)-aminocarbonyl-C₀₋₅-alkyl or a C₄₋₇-cycloalkyleneiminocarbonyl-C₀₋₅-alkyl group,
while the two groups R^{7b}/R^{7c} cannot both simultaneously be bound to the cyclic carbon atom via a heteroatom, except where -C(R^{7b}R^{7c})- corresponds to a -CF₂ group, or
R^{7a} denotes a fluorine-, chlorine-, bromine-, methyl-, methoxy-, amino- or nitro-substituted phenyl or monocyclic heteroaryl group, or
two groups R^{7b}/R^{7c} together with the cyclic carbon atom may form a 3-, 4-, 5-, 6- or 7-membered saturated carbocyclic group or a cyclopentene, cyclohexene, oxetane, azetidine, thietane, tetrahydrofuran, pyrrolidine, tetrahydrothiophene, tetrahydropyran, piperidine, pentamethylenesulphide, hexamethyleneimine, 1,3-dioxolane, 1,4-dioxane, hexahydropyridazine, piperazine, thiomorpholine, morpholine, 2-imidazolidinone, 2-oxazolidinone, tetrahydro-2(1H)-pyrimidinone or [1,3]oxazinan-2-one ring,
the methylene groups of which may be substituted by 1-2 C₁₋₃-alkyl or CF₃- groups, and/or
the methylene groups of which, if they are not bound to a heteroatom, may be substituted by 1-2 fluorine atoms, and/or wherein a -CH₂ group adjacent to an N atom may be replaced by a -CO group, and/or
the imino groups of which may in each case be substituted by a C₁₋₃-alkyl or C₁₋₃-alkylcarbonyl group, and/or
wherein the sulphur atom may be oxidised to a sulphoxide or sulphone group,
K² and K³ each independently of one another denote a -CH₂, -CHR^{8a}, -CR^{8b}R^{8c} or a -C(O) group, wherein
R^{8a}/R^{8b}/R^{8c} each independently of one another denote a C₃₋₅-cycloalkyl or a C₁₋₅-alkyl group which may be substituted by 1-3 fluorine atoms, a hydroxy-C₁₋₅-alkyl, C₁₋₅-alkyloxy-C₁₋₅-alkyl, amino-C₁₋₅-alkyl, C₁₋₅-alkylamino-C₁₋₅-alkyl, di-(C₁₋₅-alkyl)-amino-C₁₋₅-alkyl, C₄₋₇-cycloalkyleneimino-C₁₋₅-alkyl, carboxy-C₀₋₅-alkyl, C₁₋₅-alkyloxycarbonyl-C₀₋₅-alkyl, aminocarbonyl-C₀₋₅-alkyl, C₁₋₅-alkylaminocarbonyl-C₀₋₅-alkyl, di-(C₁₋₅-alkyl)-aminocarbonyl-C₀₋₅-alkyl or a C₄₋₇cycloalkyleneiminocarbonyl-C₀₋₅-alkyl group,
or two groups R^{8b}/R^{8c} together with the cyclic carbon atom may form a 3-, 4-, 5-, 6- or 7-membered saturated carbocyclic group or a cyclopentene, cyclohexene, oxetane, azetidine, thietane, tetrahydrofuran, pyrrolidine, tetrahydrothiophene, tetrahydropyran, piperidine, pentamethylenesulphide, hexamethyleneimine, hexahydropyridazine, tetrahydro-2(1H)-pyrimidinone, [1,3]oxazinan-2-one ring,
the methylene groups of which may be substituted by 1-2 C₁₋₃-alkyl or CF₃- groups, and/or
the methylene groups of which, if they are not bound to a heteroatom, may be substituted by 1-2 fluorine atoms, and/or wherein a -CH₂ group adjacent to a nitrogen atom may be replaced by a -CO group, and/or
the imino groups of which may in each case be substituted by a C₁₋₃-alkyl or C₁₋₃-alkylcarbonyl group, and/or
wherein the sulphur atom may be oxidised to a sulphoxide or sulphone group,
with the proviso that a heteroatom introduced by R^{8b} or R^{8c} must not be only one carbon atom away from X in formula (I), and
in total a maximum of four groups selected from R^{7a}, R^{7b}, R^{7c}, R^{8a}, R^{8b} and R^{8c} may be present in formula (II), and
X denotes an oxygen or sulphur atom, a CF₂, sulphene, sulphone or a NR¹ group, wherein
R¹ denotes a hydrogen atom or a hydroxy, C₁₋₃-alkyloxy, amino, C₁₋₃-alkylamino, di-(C₁₋₃-alkyl)-amino, a C₁₋₅-alkyl, C₂₋₅-alkenyl-CH₂, C₂₋₅-alkynyl-CH₂, C₃₋₆-cycloalkyl, C₄₋₆-cycloalkenyl, oxetan-3-yl, tetrahydrofuran-3-yl, benzyl, C₁₋₅-alkyl-carbonyl, trifluoromethylcarbonyl, C₃₋₆-cycloalkyl-carbonyl, C₁₋₅-alkyl-sulphonyl, C₃₋₆-cycloalkyl-sulphonyl, aminocarbonyl, C₁₋₅-alkylaminocarbonyl, di-(C₁₋₅-alkyl)-aminocarbonyl, C₁₋₅-alkyloxycarbonyl, C₄₋₇-cycloalkyleneiminocarbonyl group,
while the methylene and methyl groups present in the groups mentioned previously may additionally be substituted by a C₁₋₃alkyl, carboxy or C₁₋₅-alkoxycarbonyl group, or by a hydroxy, C₁₋₅-alkyloxy, amino, C₁₋₅-alkylamino, C₁₋₅-dialkylamino or C₄₋₇-cycloalkyleneimino group, provided that the methylene or methyl groups are not bound directly to a heteroatom selected from among O, N or S, and/or one to three hydrogen atoms may be replaced by fluorine atoms, provided that the methylene or methyl groups are not bound directly to a heteroatom selected from among O, N or S,
and wherein
A¹ denotes either N or CR¹⁰,
A² denotes either N or CR¹¹,
A³ denotes either N or CR¹²,
while R¹⁰, R¹¹ and R¹² each independently of one another denote
a hydrogen, fluorine, chlorine, bromine or iodine atom, or a C₁₋₅-alkyl, CF₃, C₂₋₅ -alkenyl, C₂₋₅-alkynyl, a cyano, carboxy, C₁₋₅-alkyloxycarbonyl, hydroxy, C₁₋₃-alkyloxy, CF₃O, CHF₂O CH₂FO, amino, C₁₋₅-alkylamino, di-(C₁₋₅-alkyl)-amino or C₄₋₇-cycloalkyleneimino group, or
D denotes one of the four groups (II-1), (II-2), (II-3), (II-4), (II-5) or (II-6) 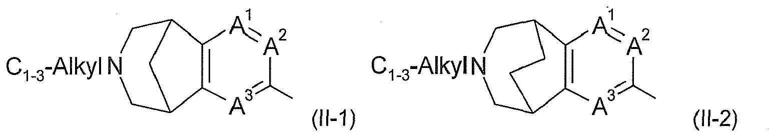 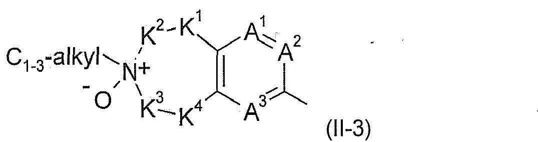 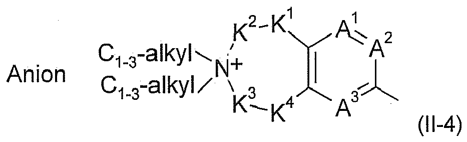 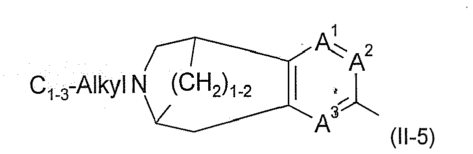 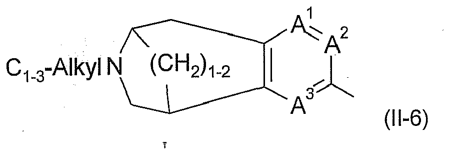 wherein the groups A1, A2, A3, K1, K2, K3, K4 are as hereinbefore defined, and the anion in (II-4) denotes a fluoride, chloride, bromide, iodide, sulphate, hydrogen sulphate, phosphate, hexafluorophosphate, hydrogen phosphate, benzoate, salicylate, succinate, citrate or tartrate,
R³ denotes a hydrogen atom or a C₁₋₃-alkyl group, and
-L-E-G-J- denotes a -C-C-C-C or -C-C=C-C group, and
R⁴ denotes a hydrogen atom or
a straight-chain or branched C₁₋₆-alkyl, C₂₋₆-alkenyl or C₂₋₆-alkynyl group, wherein the hydrogen atoms of the methylene and/or methyl fragments of the straight-chain or branched C₁₋₆-alkyl, C₂₋₆-alkenyl or C₂₋₆-alkynyl group may optionally be wholly or partly replaced by fluorine atoms, and/or
wherein the hydrogen atoms of the methylene and/or methyl fragments of the straight-chain or branched C₁₋₆-alkyl, C₂₋₆-alkenyl or C₂₋₆-alkynyl group may optionally each be substituted independently of one another by one to two substituents selected from a C₃₋₅-cycloalkyl group, a nitrile, hydroxy or C₁₋₅-alkyloxy group, wherein the hydrogen atoms of the C₁₋₅-alkyloxy group may optionally be wholly or partly replaced by fluorine atoms, an allyloxy, propargyloxy, benzyloxy, C₁₋₅-alkylcarbonyloxy, C₁₋₅-alkyloxycarbonyloxy, carboxy-C₁₋₅-alkyloxy, C₁₋₅-alkyloxycarbonyl-C₁₋₅-alkyloxy, mercapto, C₁₋₅-alkylsulphanyl, C₁₋₅-alkylsulphinyl, C₁₋₅-alkylsulphonyl, carboxy, C₁₋₅-alkyloxycarbonyl, aminocarbonyl, C₁₋₅-alkylaminocarbonyl, di-(C₁₋₅-alkyl)-aminocarbonyl, C₄₋₇-cycloalkyleneiminocarbonyl, aminosulphonyl, C₁₋₅-alkylaminosulphonyl, di-(C₁₋₅-alkyl)-aminosulphonyl, C₄₋₇cycloalkyleneiminosulphonyl, amino, C₁₋₅-alkylamino, di-(C₁₋₅-alkyl)-amino, C₁₋₅-alkylcarbonylamino, C₁₋₅-alkyl-sulphonylamino, N-(C₁₋₅-alkylsulphonyl)-C₁₋₅-alkylamino, C₃₋₆-cycloalkylcarbonylamino group, or a morpholinyl, thiomorpholinyl, pyrrolidinyl, piperidinyl, piperazinyl, tetrahydrofuranyl, tetrahydropyranyl group, while the above-mentioned carbocyclic and heterocyclic groups in the ring may each be substituted by 1-4 C₁₋₃-alkyl or C₁₋₃-alkylcarbonyl groups or by 1-2 oxo groups, and/or
wherein the hydrogen atoms of the sp²-hybridised carbon atoms of the straight-chain or branched C₂₋₆-alkenyl group may optionally be wholly or partly replaced by fluorine atoms, or
a nitrile, carboxy, aminocarbonyle, C₁₋₅-alkylaminocarbonyl, C₃₋₆-cycloalkylamino-carbonyl, di-(C₁₋₅-alkyl)-aminocarbonyl, C₁₋₅-alkyloxycarbonyl or a C₄₋₇-cycloalkyleneiminocarbonyl group wherein a methylene group may optionally be replaced by an oxygen, sulphur or C₀₋₃-alkyl-substituted nitrogen atom, or
a phenyl, mono- or bicyclic heteroaryl, phenyl-C₁₋₅-alkyl or mono- or bicyclic heteroaryl-C₁₋₅-alkyl group,
which may optionally be mono- to tri-substituted in the phenyl or heteroaryl moiety by identical or different substituents selected from among fluorine, chlorine, bromine and iodine atoms, and C₁₋₅-alkyl, trifluoromethyl, amino, C₁₋₅-alkyl-amino, di-(C₁₋₅-alkyl)-amino, hydroxy, C₁₋₅-alkyloxy, mono-, di- or trifluoromethoxy, carboxy and C₁₋₅-alkyloxycarbonyl group,
and
if -L-E-G-J- denotes a -C-C-C-C group, R⁴ at E or G may also denote a fluorine atom or a hydroxy, C₂₋₅-alkenyloxy, C₂₋₅-alkynyloxy, C₁₋₅-alkyl-oxy, C₃₋₆-cycloalkyloxy, C₁₋₅-alkylaminocarbonyloxy, di(C₁₋₅-alkyl)aminocarbonyloxy or C₄₋₇-cycloalkyleneiminocarbonyloxy, phenyl-C₀₋₃-alkyloxy, heteroaryl-C₀₋₃-alkyloxy, amino, C₁₋₅-alkylamino, di-(C₁₋₅-alkyl)-amino, C₄₋₇-cycloalkyleneimino, C₁₋₃-acylamino, (C₁₋₃-acyl)C₁₋₃-alkylamino, C₁₋₅-alkyloxycarbonylamino, C₁₋₅-alkylaminocarbonylamino, di(C₁₋₅-alkyl)aminocarbonylamino or a C₄₋₇-cycloalkyleneiminocarbonyl-amino group,
while the methyl or methylene groups present in the above-mentioned alkyl or cycloalkyl groups may each be substituted independently of one another by a substituent selected from among morpholinyl, thiomorpholinyl, pyrrolidinyl, piperidinyl, piperazinyl, tetrahydrofuranyl, tetrahydropyranyl, dimethylaminocarbonyl, C₁₋₃alkyloxycarbonyl, carboxy, methyl, hydroxy, methoxy or amino,
with the proviso that two heteroatoms selected from among oxygen and nitrogen are separated from one another by precisely one optionally substituted -CH₂ group, and/or
that two atoms form an -O-O or -S-O- bond, is excluded, and
R⁵ denotes a hydrogen atom or a C₁₋₅ alkyl, C₂₋₅ alkenyl or C₂₋₅ alkynyl group, or if R⁶ is linked to E or G it may also denote a hydroxy or methoxy group, or
R⁴ and R⁵ if they are bound to the same carbon atom, may form a -C(O)- group, or a -C(F₂)- group, or
R⁴ and R⁵ if they are bound to the same carbon atom or to two adjacent carbon atoms,
may form a C₃₋₇-cycloalkyl or C₅₋₇-cycloalkenyl group,
wherein one of the methylene groups of this C₄₋₇-cycloalkyl group may be replaced by an oxygen or sulphur atom or a -NH, -N(C₁₋₅-alkyl), -N(C₁₋₄-alkylcarbonyl) or a carbonyl, sulphinyl or sulphonyl group, and/or
wherein two directly adjacent methylene groups of this C₄₋₇-cycloalkyl group may together be replaced by a -C(O)NH, -C(O)N(C₁₋₅-alkyl), -S(O)₂NH, or -S(O)₂N(C₁₋₅-alkyl) group, and/or
wherein 1 to 3 carbon atoms of a C₃₋₇-cycloalkyl group may each optionally be substituted independently of one another by one or two fluorine atoms or one or two C₁₋₅-alkyl groups or a hydroxy, C₁₋₅-alkyloxy, C₁₋₅-alkylcarbonyloxy, amino, C₁₋₅-alkylamino, di-(C₁₋₅-alkyl)-amino, C₄₋₇-cycloalkyleneimino, C₁₋₅-alkylcarbonylamino, C₃₋₆-cycloalkylcarbonyl-amino, nitrile, carboxy-C₁₋₅-alkyl, C₁₋₅-alkyloxycarbonyl-C₁₋₅-alkyl, carboxy, C₁₋₅-alkyloxycarbonyl, aminocarbonyl, C₁₋₅-alkylaminocarbonyl, di-(C₁₋₅-alkyl)-aminocarbonyl or C₄₋₇cycloalkyleneiminocarbonyl group,
with the proviso that a C₃₋₇-cycloalkyl group of this kind, formed from R⁴ and R⁵ together,
wherein two heteroatoms in the cyclic group selected from among oxygen and nitrogen are separated from one another by precisely one optionally substituted.-CH₂ group, and/or
wherein two atoms in the ring form a -O-O or-S-O- bond,
is excluded,
R¹³ denotes a hydrogen atom or a C₁₋₅alkyl group,
M denotes a phenyl, thienyl or pyridyl ring optionally substituted by R² and R⁶, wherein
R² denotes a fluorine, chlorine, bromine or iodine atom or a methyl, ethyl, propyl, isopropyl, vinyl, methoxy, ethynyl, cyano or-C(O)NH₂ group, and
R⁶ denotes a hydrogen, fluorine, chlorine, bromine or iodine atom or a hydroxy, methoxy, trifluoromethoxy, an optionally fluorine-substituted C₁₋₃-alkyl, cyano, amino, or NH₂C(O) group,
while, unless otherwise stated, by the term "heteroaryl group" mentioned hereinbefore in the definitions is meant a monocyclic 5- or 6-membered heteroaryl group wherein
the 6-membered heteroaryl group contains one, two or three nitrogen atoms, and
the 5-membered heteroaryl group contains an imino group optionally substituted by a C₁₋₃-alkyl group, an oxygen or sulphur atom, or
an imino group optionally substituted by a C₁₋₃-alkyl group or an oxygen or sulphur atom and additionally one or two nitrogen atoms, or
an imino group optionally substituted by a C₁₋₃-alkyl group and three nitrogen atoms,
and additionally a phenyl ring optionally substituted by a fluorine, chlorine or bromine atom, a C₁₋₃-alkyl, hydroxy, C₁₋₃-alkyloxy group, amino, C₁₋₃-alkylamino, di-(C₁₋₃-alkyl)-amino or C₃₋₆-cycloalkyleneimino group may be fused to the above-mentioned monocyclic heteroaryl groups via two adjacent carbon atoms,
and the bond is effected via a nitrogen atom or a carbon atom of the heterocyclic moiety or a fused-on phenyl ring,
and wherein, unless otherwise stated, by the term "halogen atom" mentioned hereinbefore in the definitions is meant an atom selected from among fluorine, chlorine, bromine and iodine,
and wherein unless stated otherwise the alkyl, alkenyl, alkynyl and alkyloxy groups which have more than two carbon atoms, contained in the foregoing definitions, may be straight-chain or branched and the alkyl groups in the previously mentioned dialkylated groups, for example the dialkylamino groups, may be identical or different,
and the hydrogen atoms of the methyl or ethyl groups contained in the foregoing definitions, unless stated otherwise, may be wholly or partly replaced by fluorine atoms,
the tautomers, the enantiomers, the diastereomers, the mixtures thereof and the salts thereof.

2. Compounds of general formula (I) according to claim 1, wherein
D denotes a substituted bicyclic ring system of formula (II) 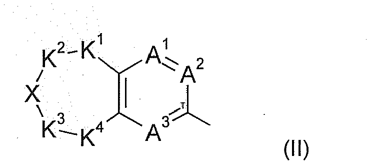 wherein
K¹ and K⁴ each independently of one another denote a -CH₂, -CHR^{7a}, -CR^{7b}R^{7c}, -C(=CH2) or a -C(O) group, and wherein
R^{7a}/R^{7b}/R^{7c} each independently of one another denote a fluorine atom, a hydroxy, C₁₋₅-alkyloxy, a C₁₋₅-alkyl group,
while the two groups R^{7b}/R^{7c} cannot both simultaneously be bound to the cyclic carbon atom via a heteroatom, except where -C(R^{7b}R^{7c})-corresponds to a -CF₂ group,
or two groups R^{7b}/R^{7c} together with the cyclic carbon atom may form a cyclopropyl ring,
K² and K³ each independently of one another denote a -CH₂, -CHR^{8a}, -CR^{8b}R^{8c} or a -C(O)- group, while
R^{8a}/R^{8b}/R^{8c} each independently of one another denote a C₁₋₅-alkyl group, or two groups R^{8b}/R^{8c} together with the cyclic carbon atom may form a cyclopropyl ring,
and
in total a maximum of four groups selected from R^{7a}, R^{7b}, R^{7c}, R^{8a}, R^{8b} and R^{8c} may be present in formula (II), and
X denotes an oxygen or sulphur atom, a sulphene, sulphone or an NR¹ group, wherein
R¹ denotes a hydrogen atom or a hydroxy, C₁₋₃-alkyloxy, amino, C₁₋₃-alkylamino, di-(C₁₋₃-alkyl)-amino, a C₁₋₅-alkyl, C₂₋₅-alkenyl-CH₂, C₂₋₅-alkynyl-CH₂ or a C₃₋₆-cycloalkyl group,
and wherein
A¹ denotes either N or CR¹⁰,
A² denotes either N or CR¹¹,
A³ denotes either N or CR¹²,
while R¹⁰, R¹¹ and R¹² each independently of one another denote
a hydrogen, fluorine, chlorine, bromine or iodine atom, or a C₁₋₅-alkyl, CF₃, a cyano, carboxy, C₁₋₆-alkyloxycarbonyl, hydroxy, C₁₋₃-alkyloxy, CF₃O, CHF₂O, CH₂FO, amino, C₁₋₅-alkylamino, di-(C₁₋₅-alkyl)-amino or C₄₋₇-cycloalkyleneimino group.

3. Compounds of general formula (I) according to claim 1 or 2, wherein
X denotes a NR¹ group, wherein
R¹ denotes a hydrogen atom or a C₁₋₅-alkyl, allyl or cyclopropyl group, and
A¹ denotes CR¹⁰,
A² denotes CR¹¹,
A³ denotes CR¹²,
while R¹⁰, R¹¹ and R¹² each independently of one another represent
a hydrogen, fluorine or chlorine atom, or a methyl, CF₃, hydroxy, methoxy, CF₃O, CHF₂O, CH₂FO group.

4. Compounds of general formula (I) according to one of Claims 1, 2 or 3, wherein
R⁴ denotes a hydrogen atom or
a straight-chain or branched C₁₋₆-alkyl group,
wherein the hydrogen atoms of the methylene and/or methyl fragments of the straight-chain or branched C₁₋₆-alkyl group may optionally be wholly or partly replaced by fluorine atoms, and/or
wherein the hydrogen atoms of the methylene and/or methyl fragments of the straight-chain or branched C₁₋₆-alkyl group may optionally each be substituted independently of one another by a substituent selected from a hydroxy, C₁₋₅-alkyloxy, carboxy, C₁₋₅-alkyloxycarbonyl, aminocarbonyl, C₁₋₅-alkylaminocarbonyl, di-(C₁₋₅-alkyl)-aminocarbonyl, C₄₋₇-cycloalkyleneiminocarbonyl, amino, C₁₋₅-alkylamino, di-(C₁₋₅-alkyl)-amino, C₁₋₅-alkylcarbonylamino, C₁₋₅-alkylsulphonylamino, N-(C₁₋₅-alkylsulphonyl)-C₁₋₅-alkylamino, C₃₋₆-cycloalkylcarbonylamino group, or
a nitrile, carboxy, aminocarbonyl, C₁₋₅-alkylaminocarbonyl, C₃₋₆-cycloalkylamino-carbonyl, di-(C₁₋₆-alkyl)-aminocarbonyl, C₁₋₅-alkyloxycarbonyl or a C₄₋₇-cycloalkyleneiminocarbonyl group wherein a methylene group may optionally be replaced by an oxygen, sulphur or C₀₋₃-alkyl-substituted nitrogen atom, and
if -L-E-G-J- denotes a -C-C-C-C group, R⁴ at E or G may also denote a fluorine atom or a hydroxy, methoxy, C₂₋₅-alkenyloxy, C₂₋₅-alkynyloxy, C₁₋₅-alkyl-oxy, C₃₋₆-cycloalkyloxy, C₁₋₅-alkylaminocarbonyloxy, di(C₁₋₅-alkyl)aminocarbonyloxy or C₄₋₇-cycloalkyleneiminocarbonyloxy, phenyl-C₀₋₃-alkyloxy, amino, C₁₋₅-alkylamino, di-(C₁₋₅-alkyl)-amino, C₄₋₇cycloalkyleneimino, C₁₋₃-acylamino, (C₁₋₃-acyl)C₁₋₃-alkylamino, C₁₋₅-alkyloxycarbonylamino, C₁₋₅-alkylaminocarbonylamino, di(C₁₋₅-alkyl)aminocarbonylamino or a C₄₋₇-cycloalkyleneiminocarbonylamino- group,
while the methyl or methylene groups present in the above-mentioned alkyl or cycloalkyl groups may each be substituted independently of one another by a substituent selected from among dimethylaminocarbonyl, C₁₋₃alkyloxycarbonyl, carboxy, methyl, hydroxy, methoxy or amino,
with the proviso that two heteroatoms selected from among oxygen and nitrogen are separated from one another by precisely one optionally substituted -CH₂ group, and/or
that two atoms form an -O-O or -S-O- bond, is excluded, and
R⁵ denotes a hydrogen atom, an allyl or a C₁₋₅alkyl group, or if R⁵ is linked to E or G it may also denote a hydroxy or methoxy group or
R⁴ and R⁵, if they are bound to the same carbon atom,
may form a -C(O)- group,or a -C(F₂)- group, or
R⁴ and R⁵ if they are bound to the same carbon atom or to two adjacent carbon atoms,
may form a C₃₋₇-cycloalkyl group,
wherein one of the methylene groups of this C₄₋₇-cycloalkyl group may be replaced by an oxygen or sulphur atom or an -NH, -N(C₁₋₅-alkyl), -N(C₁₋₄-alkylcarbonyl) or a carbonyl, sulphinyl or sulphonyl group, and/or
wherein two directly adjacent methylene groups of this C₄₋₇-cycloalkyl group may together be replaced by a -C(O)NH, -C(O)N(C₁₋₅-alkyl), -S(O)₂NH or -S(O)₂N(C₁₋₅-alkyl) group.

5. Compounds of general formula (I) according to one of Claims 1, 2, 3 or 4, wherein
-L-E-G-J- denotes a -C-C-C-C group.

6. Compounds of general formula (I) according to one of Claims 1, 2, 3, 4 or 5, wherein
D denotes a substituted benzazepinyl group of formula (IIa) 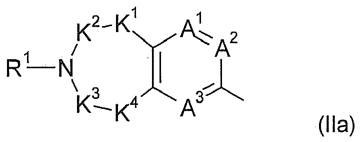 wherein
K¹ and K⁴ each independently of one another denote a -CH₂, -CHR^{7a}, -CR^{7b}R^{7c} or a -C(O) group, wherein
R^{7a} denotes a C₁₋₅-alkyl, hydroxy or C₁₋₃-alkyloxy group and
R^{7b}/R^{7c} each independently of one another denote a hydroxy, C₁₋₅-alkyloxy or a C₁₋₅-alkyl group,
while the two groups R^{7b}/R^{7c} cannot both simultaneously be bound to the cyclic carbon atom via an oxygen atom, or two groups R^{7b}/R^{7c} together with the cyclic carbon atom may form a cyclopropyl ring, and
K² and K³ each independently of one another denote a -CH₂, -CHR^{8a} or -CR^{8b}R^{8c} group, wherein
R^{8a}/R^{8b}/R^{8c} each independently of one another denote a C₁₋₃-alkyl group,
and
in total a maximum of four groups selected from R^{7a}, R^{7b}, R^{7c}, R^{8a}, R^{8b} and R^{8c} may be present in formula (II), and
R¹ denotes a hydrogen atom or a C₁₋₅-alkyl, allyl or cyclopropyl group, and
A¹ denotes CR¹⁰,
A² denotes CR¹¹,
A³ denotes CR¹²,
while R¹⁰, R¹¹ and R¹² each independently of one another denote
a hydrogen, fluorine or chlorine atom, or a methyl, CF₃, hydroxy, methoxy, CF₃O, CHF₂O, CH₂FO group,
and
-L-E-G-J- denotes a -C-C-C-C group, and
R³ denotes a hydrogen atom, and
R⁴ denotes a hydrogen atom or
a straight-chain or branched C₁₋₃-alkyl group,
wherein the hydrogen atoms of the methylene and/or methyl fragments of the straight-chain or branched C₁₋₆-alkyl group may optionally be substituted independently of one another by a substituent selected from among a hydroxy, C₁₋₅-alkyloxy, carboxy, C₁₋₅-alkyloxycarbonyl group, or
a CF₃, nitrile, carboxy, aminocarbonyl, C₁₋₅-alkylaminocarbonyl, C₃₋₆-cycloalkylaminocarbonyl, di-(C₁₋₅-alkyl)-aminocarbonyl, C₁₋₅-alkyloxycarbonyl or a C₄₋₇-cycloalkyleneiminocarbonyl group, or
if R⁴ is bound to E or G it may also denote a fluorine atom or a hydroxy, methoxy, C₂₋₅-alkenyl-oxy, C₂₋₅-alkyl-oxy, methoxyethoxy, HOCH₂CH(OH)CH₂oxy, C₃₋₆-cycloalkyloxy, C₁₋₅-alkylaminocarbonyloxy, di(C₁₋₆-alkyl)aminocarbonyloxy or C₄₋₇-cycloalkyleneiminocarbonyloxy- group,
R⁵ denotes a hydrogen atom or a C₁₋₅ alkyl group, or if R⁵ is linked to E or G it may also denote a hydroxy or methoxy group, or
R⁴ and R⁵, if they are bound to the same carbon atom, may denote a C=O or a -CF₂ group, and
R¹³ denotes a hydrogen atom,
M denotes a substituted phenyl ring 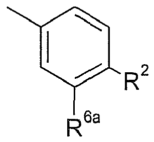 or a substituted pyridyl ring 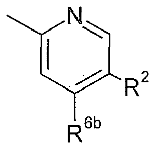 wherein
R² denotes a fluorine, chlorine, bromine atom, a methoxy or ethynyl group, and
R^{6a} denotes a hydrogen or fluorine atom and
R^{6b} denotes a hydrogen atom.

7. Physiologically acceptable salts of the compounds according to one of claims 1 to 6.

8. Medicaments, containing a compound according to at least one of claims 1 to 6 or a physiologically acceptable salt according to claim 7, optionally together with one or more inert carriers and/or diluents.

9. Use of a compound according to at least one of claims 1 to 6 or a physiologically acceptable salt according to claim 7 for preparing a medicament with an inhibitory effect on factor Xa and/or an inhibitory effect on related serine proteases.

10. Process for preparing a medicament according to claim 8, **characterised in that** a compound according to at least one of claims 1 to 6 or a physiologically acceptable salt according to claim 7 is incorporated in one or more inert carriers and/or diluents by a non-chemical method.

## Revendications

1. Composés de formule générale (I) 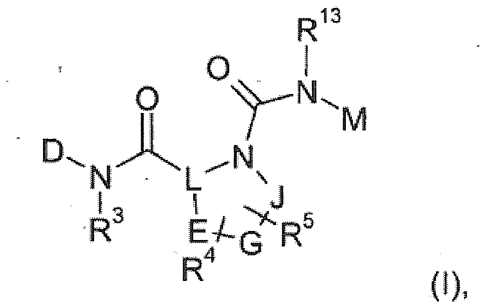 dans lesquels
D représente un système cyclique bicyclique substitué de formule (II) 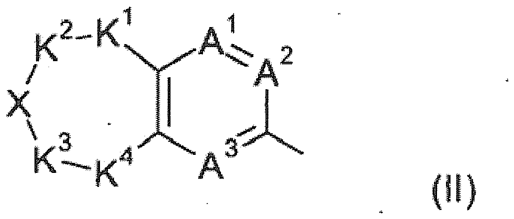 dans lequel
K¹ et K⁴ sont respectivement, indépendamment l'un de l'autre, un groupe -CH₂, -CHR^{7a}, -CR^{7b}R^{7c},-C(=CH2) ou -C(O), et dans lequel
R^{7a}/R^{7b}/R^{7c} sont respectivement, indépendamment les uns des autres, un atome de fluor, un groupe hydroxy, alkyloxy en C1 à C₅, amino, alkylamino en C₁ à C₅, di- (alkyle en C₁ à C₅) -amino, cycloalkylène-imino en C₃ à C₅, alkylcarbonylamino en C₁ à C₅, cycloalkyle en C₃ à C₅ ou alkyle en C₁ à C₅, qui peut être substitué par 1 à 3 atomes de fluor, un groupe hydroxy-alkyle en C₁ à C₅, alkyloxy en C₁ à C₅-alkyle en C₁ à C₅, amino-alkyle en C₁ à C₅, alkylamino en C₁ à C₅-alkyle en C₁ à C₅, di-(alkyle en C₁ à C₅)-amino-alkyle en C₁ à C₅, cycloalkylène-imino en C₄ à C₇-alkyle en C₁ à C₅, carboxy-alkyle en C₀ à C₅, alkyloxyocarbonyle en C₁ à C₅-alkyle en C₀ à C₅, aminocarbonyl-alkyle en C₀ à C₅, alkylaminocarbonyle en C₁ à C₅-alkyle en C₀ à C₅, di- (alkyle en C₁ à C₅)-aminocarbonyl-alkyle en C₀ à C₅ ou cycloalkylène-iminocarbonyle en C₄ à C₇-alkyle en C₀ à C₅,
dans lequel les deux radicaux R^{7b}/R^{7c} ne peuvent pas être liés en même temps par un hétéroatome à l'atome de carbone cyclique, sauf si -C(R^{7b}R^{7c}) correspond à un groupe -CF₂, ou
R^{7a} est un groupe phényle ou hétéroaryle monocyclique substitué par un atome de fluor, de chlore, de brome, un groupe méthyle, méthoxy, amino ou nitro, ou
deux radicaux R^{7b}/R^{7c} conjointement avec l'atome de carbone cyclique peuvent former un carbocycle saturé de 3, 4, 5, 6 ou 7 chaînons ou un cycle cyclopentène, cyclohexène, oxétane, azétidine, thiétane, tétrahydrofurane, pyrrolidine, tétrahydro-thiophène, tétrahydropyrane, pipéridine, pentaméthylènesulfure, hexaméthylène-imine, 1,3-dioxolane, 1,4-dioxane, hexahydro-pyridazine, pipérazine, thiomorpholine, morpholine, 2-imidazolidinone, 2-oxazolidinone, tétrahydro-2(1H)-pyrimidinone ou [1,3]oxazinan-2-one,
dans lequel leurs groupes méthylène peuvent être substitués par 1 à 2 groupes alkyle en C₁ à C₃ ou CF₃, et/ou leurs groupes méthylènes, dans la mesure où ils ne sont pas liés à un hétéroatome, peuvent être substitués par 1 à 2 atomes de fluor, et/ou
dans lequel un groupe -CH₂ peut être remplacé, en plus d'un atome de N, par un groupe -CO, et/ou
leurs groupes imino peuvent être substitués respectivement par un groupe alkyle en C₁ à C₃ ou alkylcarbonyle en C₁. à C₃, et/ou dans lequel l'atome de soufre peut être oxydé en un sulfoxyde ou un groupe sulfone,
K² et K³ sont respectivement, indépendamment l'un de l'autre, un groupe -CH₂, -CHR^{8a}, -CR^{8b}R^{8c} ou un groupe -C(O), dans lequel
R^{8a}/R^{8b}/R^{8c} sont respectivement, indépendamment les uns des autres, un groupe cycloalkyle en C₃ à C₅ ou alkyle en C₁ à C₅ qui peut être substitué par 1 à 3 atomes de fluor, un groupe hydroxy-alkyle en C₁ à C₅, alkyloxy en C₁ à C₅-alkyle en C₁ à C₅, amino-alkyle en C₁ à C₅, alkylamino en C₁ à C₅-alkyle en C₁ à C₅, di-(alkyle en C₁ à C₅)-amino-alkyle en C₁ à C₅, cycloalkylène-imino en C₄ à C₇-alkyle en C₁ à C₅, carboxy-alkyle en C₀ à C₅, alkyloxycarbonyle en C₁ à C₅-alkyle en C₀ à C₅, aminocarbonyl-alkyle en C₀ à C₅, alkylaminocarbonyle en C₁ à C₅-alkyle en C₀ à C₅, di-(alkyle en C₁ à C₅)-aminocarbonyl-alkyle en C₀ à C₅ ou cycloalkylène-iminocarbonyle en C₄ à C₇-alkyle en C₀ à C₅,
ou deux radicaux R^{8b}/R^{8c} peuvent former conjointement avec l'atome de carbone cyclique, un carbocycle saturé de 3, 4, 5, 6 ou 7 chaînons ou un cycle cyclopentène, cyclohexène, oxétane, azétidine, thiétane, tétrahydrofurane, pyrrolidine, tétrahydro-thiophène, tétrahydropyrane, pipéridine, pentaméthylènesulfure, hexaméthylène-imine, hexahydropyridazine, tétrahydro-2(1H)-pyrimidinone, [1,3]oxazinan-2-one,
dans lequel leurs groupes méthylène peuvent être substitués par 1 à 2 groupes alkyle en C₁ à C₃ ou CF₃, et/ou leurs groupes méthylène, dans la mesure où ils ne sont pas liés à un hétéroatome, peuvent être substitués par 1 à 2 atomes de fluor, et/ou
dans lequel un groupe -CH₂ peut être remplacé, outre un atome d'azote, par un groupe -CO, et/ou
leurs groupes imino peuvent être substitués respectivement par un groupe alkyle en C₁ à C₃ ou alkylcarbonyle en C₁ à C₃, et/ou
dans lequel l'atome de soufre peut être oxydé en un groupe sulfoxyde ou sulfone, à condition qu'un hétéroatome apporté par R^{8b} ou R^{8c} ne puisse pas être éliminé par un atome de carbone de X dans la formule (I), et
au total, dans la formule (II) au maximum, quatre radicaux choisis parmi R^{7a}, R^{7b}, R^{7c}, R^{8a}, R^{8b} et R^{8c} peuvent être présents, et
X est un atome d'oxygène ou un atome de soufre, un groupe CF₂, sulfène, sulfone ou NR¹, dans lequel
R¹ est un atome d'hydrogène ou un groupe hydroxy, alkyloxy en C₁ à C₃, amino, alkylamino en C₁ à C₃, di-(alkyle en C₁ à C₃) -amino, un groupe alkyle en C₁ à C₅, alcényle en C₂ à C₅-CH₂, alcinyle en C₂ à C₅-CH₂, cycloalkyle en C₃ à C₆, cycloalcényle en C₄ à C₆, oxétan-3-yle, tétrahydrofuran-3-yle, benzyle, alkyle en C₁ à C₅-carbonyle, trifluoro-méthylcarbonyle, cycloalkyle en C₃ à C₆-carbonyle, alkyle en C₁ à C₅-sulfonyle, cycloalkyle en C₃ à C₆-sulfonyle, aminocarbonyle, alkylaminocarbonyle en C₁ à C₅, di-(alkyle en C₁ à C₅)-aminocarbonyle, alkyloxycarbonyle en C₁ à C₅, cycloalkylène-iminocarbonyle en C₄ à C₇,
dans lequel les groupes méthylène et méthyle se trouvant dans les groupes mentionnés précédemment peuvent en outre être substitués par un groupe alkyle en C₁ à C₃, carboxy, alcoxycarbonyle en C₁ à C₅ ou par un groupe hydroxy, alkyloxy en C₁ à C₅, amino, alkylamino en C₁ à C₅, dialkylamino en C₁ à C₅ ou cycloalkylène-imino en C₄ à C₇, dans la mesure où les groupes méthylène ou méthyle ne sont pas liés directement à un hétéroatome du groupe O, N ou S, et/ou un à trois atomes d'hydrogène peuvent être remplacés par des atomes de fluor, dans la mesure où les groupes méthylène ou méthyle ne sont pas directement liés à un hétéroatome du groupe 0, N ou S,
et dans lequel
A¹ est soit N soit CR¹⁰,
A² est soit N, soit CR¹¹,
A³ est soit N, soit CR¹²,
dans lequel R¹⁰, R¹¹ et R¹² sont chacun indépendamment les uns des autres
un atome d'hydrogène, de fluor, de chlore, de brome ou d'iode ou un groupe alkyle en C₁ à C₅, CF₃, alcényle en C₂ à C₅, alcinyle en C₂ à C₅, un groupe cyano, carboxy, alkyloxycarbonyle en C₁ à C₅, hydroxy, alkyloxy en C₁ à C₃, CF₃O, CHF₂O, CH₂FO, amino, alkylamino en C₁ à C₅, di-(alkyle en C₁ à C₅)-amino ou cycloalkylène-imino en C₄ à C₇, ou
D représente l'un des quatre groupes (II-1), (II-2), (II-3), (II-4), (II-5) ou (II-6) 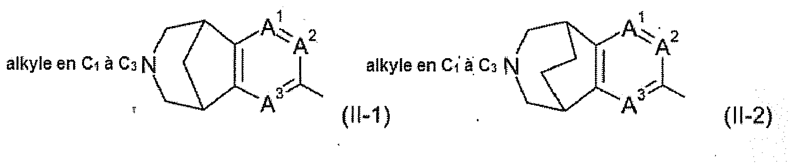 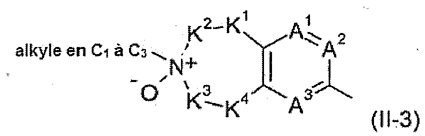 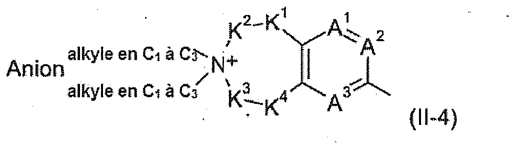 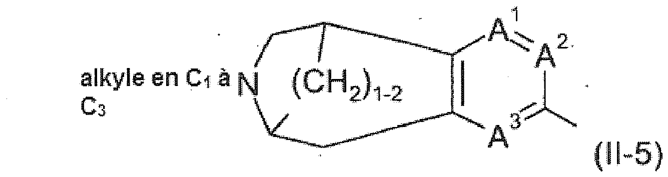 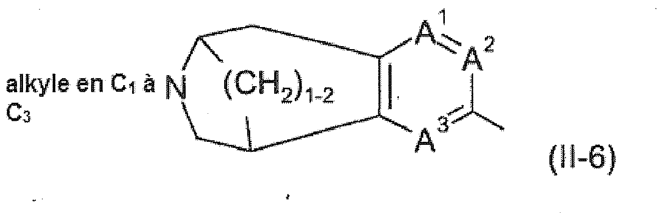 dans lequel les radicaux A¹, A², A³, K¹, K², K³, K⁴ sont tels que définis ci-dessus, et l'anion dans (II-4) est un fluorure, un chlorure, un bromure, un iodure, un sulfate, un hydrogénosulfate, un phosphate, un hexafluorophosphate, un hydrogénophosphate, un benzoate, un salicylate, un succinate, un citrate ou un tartrate,
R³ est un atome d'hydrogène ou un groupe alkyle en C₁ à C₃, et
-L-E-G-J est un groupe -C-C-C-C ou -C-C=C-C et
R⁴ est un atome d'hydrogène ou
un groupe alkyle en C₁ à C₆, alcényle en C₂ à C₆ ou alcinyle en C₂ à C₆, linéaire ou ramifié,
dans lequel les atomes d'hydrogène des fragments méthylène et/ou méthyle du groupe alkyle en C₁ à C₆, alcényle en C₂ à C₆ ou alcinyle en C₂ à C₆ linéaire ou ramifié peuvent être remplacés éventuellement totalement ou partiellement par des atomes de fluor, et/ou
dans lequel les atomes d'hydrogène des fragments méthylène et/ou méthyle du groupe alkyle en C₁ à C₆, alcényle en C₂ à C₆ ou alcinyle en C₂ à C₆ linéaires ou ramifiés peuvent être chacun substitués éventuellement, indépendamment les uns des autres, par un à deux substituants choisis parmi un groupe cycloalkyle en C₃ à C₅, un groupe nitrile, hydroxy ou alkyloxy en C₁ à C₅, dans lequel les atomes d'hydrogène du groupe alkyloxy en C₁ à C₅ peuvent être remplacés éventuellement totalement ou partiellement par des atomes de fluor, un groupe allyloxy, propargyloxy, benzyloxy, alkylcarbonyloxy en C₁ à C₅, alkyloxycarbonyloxy en C₁ à C₅, carboxy-alkyloxy en C₁ à C₅, alkyloxycarbonyle en C₁ à C₅-alkyloxy en C₁ à C₅, mercapto, alkylsulfanyle en C₁ à C₅, alkylsulfinyle en C₁ à C₅, alkylsulfonyle en C₁ à C₅, carboxy, alkyloxycarbonyle en C₁ à C₅, aminocarbonyle, alkylaminocarbonyle en C₁ à C₅, di-(alkyle en C₁ à C₅)-aminocarbonyle, cycloalkylène-iminocarbonyle en C₄ à C₇, aminosulfonyle, alkylaminosulfonyle en C₁ à C₅, di-(alkyle en C₁ à C₅)-aminosulfonyle, cycloalkylène-iminosulfonyle en C₄ à C₇, amino, alkylamino en C₁ à C₅, di- (alkyle en C₁ à C₅)-amino, alkylcarbonylamino en C₁ à C₅, alkyle en C₁ à C₅-sulfonylamino, N-(alkylsulfonyle en C₁ à C₅)-alkylamino en C₁ à C₅, cycloalkylcarbonylamino en C₃ à C₆ ou un groupe morpholinyle, thiomorpholinyle, pyrrolidinyle, pipéridinyle, pipérazinyle, tétrahydrofuranyle, tétrahydropyranyle, dans lequel les carbo- et hétérocycles mentionnés précédemment peuvent être substitués dans le cycle respectivement par 1 à 4 groupes alkyle en C₁ à C₃ ou alkylcarbonyle en C₁ à C₃ ou respectivement par 1 à 2 groupes oxo, et/ou
dans lequel les atomes d'hydrogène des atomes de carbone hybridés sp² du groupe alcényle en C₂ à C₆ à chaîne linéaire ou ramifié peuvent être remplacés éventuellement totalement ou partiellement par des atomes de fluor, ou
un groupe nitrile, carboxy, aminocarbonyle, alkylaminocarbonyle en C₁ à C₅, cycloalkyl-aminocarbonyle en C₃ à C₆, di-(alkyle en C₁ à C₅)-aminocarbonyle, alkyloxycarbonyle en C₁ à C₅ ou un groupe cycloalkylène-iminocarbonyle en C₄ à C₇ dans lequel éventuellement un groupe méthylène peut être remplacé par un atome d'oxygène, de soufre ou d'azote substitué par un groupe alkyle en C₀ à C₃,
ou
un groupe phényle, hétéroaryle mono- ou bicyclique, phényl-alkyle en C₁ à C₅ ou hétéroaryl-alkyle en C₁ à C₅ mono- ou bicyclique,
qui peut être substitué dans la partie phényle ou hétéroaryle éventuellement une à trois fois par des substituants identiques ou différents choisis dans le groupe comprenant des atomes de fluor, de chlore, de brome et d'iode et un groupe alkyle en C₁ à C₅, trifluorométhyle, amino, alkyle en C₁ à C₅-amino, di-(alkyle en C₁ à C₅)-amino, hydroxy, alkyloxy en C₁ à C₅, mono-, di- ou trifluorométhoxy, carboxy et alkyoxycarbonyle en C₁ à C₅, et
dans lequel
si -L-E-G-J est un groupe -C-C-C-C, R⁴ peut représenter sur E ou G, également un atome de fluor ou un groupe hydroxy, alcényle en C₂ à C₅-oxy, alcinyle en C₂ à C₅-oxy, alkyle en C₁ à C₅-oxy, cycloalkyle en C₃ à C₆-oxy, alkylaminocarbonyloxy en C₁ à C₅, di (alkyle en C₁ à C₅)aminocarbonyloxy ou cycloalkylène-iminocarbonyloxy en C₄ à C₇, phényl-alkyloxy en C₀ à C₃, hétéroaryl-alkyle en C₀ à C₃-oxy, amino, alkylamino en C₁ à C₅, di-(alkyle en C₁ à C₅)-amino, cycloalkylène-imino en C₄ à C₇, acylamino en C₁ à C₃, (acyle en C₁ à C₃) alkylamino en C₁ à C₃, alkyloxycarbonylamino en C₁ à C₅, alkylaminocarbonylamino en C₁ à C₅, di(alkyle en C₁ à C₅)aminocarbonylamino ou un groupe cycloalkylène-iminocarbonylamino en C₄ à C₇,
dans lequel les groupes méthyle ou méthylène présents dans les radicaux alkyle ou cycloalkyle mentionnés précédemment peuvent être substitués respectivement, indépendamment les uns des autres, par un substituant choisi dans le groupe morpholinyle; thiomorpholinyle, pyrrolidinyle, pipéridinyle, pipérazinyle, tétrahydro-furanyle, tétrahydropyranyle, diméthylamino-carbonyle, alkyloxycarbonyle en C₁ à C₃, carboxy, méthyle, hydroxy, méthoxy ou amino,
à condition qu'il soit exclu que deux hétéroatomes du groupe comprenant l'oxygène et l'azote soient séparés l'un de l'autre par exactement un groupe -CH₂ éventuellement substitué, et/ou
que deux atomes forment une liaison -O-O ou -S-O,
R⁵ est un atome d'hydrogène ou un groupe alkyle en C₁ à C₅, alcényle en C₂ à C₅ ou alcinyle en C₂ à C₅ ou dans la mesure où R⁵ est relié à E ou G, peut également représenter un groupe hydroxy ou méthoxy, ou
R⁴ et R⁵, dans la mesure où ils sont liés au même atome de carbone, peuvent former un groupe -C(O) ou un groupe -C(F₂), ou
R⁴ et R⁵, dans la mesure où ils sont liés au même atome de carbone ou à deux atomes de carbone voisin,
peuvent former un groupe cycloalkyle en C₃ à C₇ ou cycloalcényle en C₅ à C₇,
dans lequel l'un des groupes méthylène de ce groupe cycloalkyle en C₄ à C₇ peut être remplacé par un atome d'oxygène ou de soufre ou un groupe-NH, -N(alkyle en C₁ à C₅), -N(alkylcarbonyle en C₁ à C₄) ou carbonyle, sulfinyle ou sulfonyle, et/ou
dans lequel deux groupes méthylène directement voisins de ce groupe cycloalkyle en C₄ à C₇ peuvent être remplacés conjointement par un groupe -C(O)NH, -C(O)N(alkyle en C₁ à C₅), -S(O)₂NH ou-S(O)₂N(alkyle en C₁ à C₅), et/ou
dans lequel 1 à 3 atomes de carbone d'un groupe cycloalkyle en C₃ à C₇ peuvent être substitués éventuellement, indépendamment les uns des autres, par respectivement un ou deux atomes de fluor ou un ou deux groupes alkyle en C₁ à C₅ ou un groupe hydroxy, alkyloxy en C₁ à C₅, alkylcarbonyloxy en C₁ à C₅, amino, alkylamino en C₁ à C₅, di- (alkyle en C₁ à C₅) amino, cycloalkylène-imino en C₄ à C₇, alkylcarbonylamino en C₁ à C₅, cyclo-alkylcarbonylamino en C₃ à C₆, nitrile, carboxy-alkyle en C₁ à C₅, alkyloxycarbonyle en C₁ à C₅-alkyle en C₁ à C₅, carboxy, alkyloxycarbonyle en C₁ à C₅, aminocarbonyle, alkylaminocarbonyle en C₁ à C₅, di-(alkyle en C₁ à C₅)-aminocarbonyle, ou cycloalkylène-iminocarbonyle en C₄ à C₇,
à condition que ce groupe cycloalkyle en C₃ à C₇ formé conjointement de R⁴ et R⁵,
dans lequel deux hétéroatomes dans le cycle du groupe oxygène et azote sont séparés l'un de l'autre par exactement un groupe -CH₂ éventuellement substitué, et/ou
dans lequel deux atomes dans le cycle forment une liaison -O-O ou -S-O,
soit exclu,
R¹³ est un atome d'hydrogène ou un groupe alkyle en C₁ à C₅,
M est un cycle phényle, thiényle ou pyridyle éventuellement substitué par R² et R⁶, dans lequel
R² représente un atome de fluor, de chlore, de brome ou d'iode ou un groupe méthyle, éthyle, propyle, isopropyle, vinyle, méthoxy, éthinyle, cyano ou -C(O)NH₂, et
R⁶ représente un atome d'hydrogène, de fluor, de chlore, de brome ou d'iode ou un groupe hydroxy, méthoxy, trifluorométhoxy, un groupe alkyle en C₁ à C₃ éventuellement substitué par des atomes de fluor, cyano, amino ou NH₂C(O),
dans lequel, sauf indication contraire, l'expression "groupe hétéroaryle" mentionnée précédemment dans les définitions, désigne un groupe hétéroaryle monocyclique de 5 ou 6 chaînons, dans lequel
le groupe hétéroaryle à 6 chaînons contient, un, deux ou trois atomes d'azote, et
le groupe hétéroaryle à 5 chaînons contient un groupe imino substitué éventuellement par un groupe alkyle en C₁ à C₃, un atome d'oxygène ou de soufre, ou
un groupe imino substitué éventuellement par un groupe alkyle en C₁ à C₃ ou un atome d'oxygène ou de soufre et en outre un ou deux atomes d'azote, ou
un groupe imino substitué éventuellement par un groupe alkyle en C₁ à C₃ et trois atomes d'azote,
et en outre aux groupes hétéroaryle monocycliques mentionnés précédemment, par deux atomes de carbone voisins, peut être condensé un cycle phényle substitué éventuellement par un atome de fluor, de chlore ou de brome, un groupe alkyle en C₁ à C₃, hydroxy, alkyloxy en C₁ à C₃, amino, alkylamino en C₁ à C₃, di-(alkyle en C₁ à C₃) -amino ou cycloalkylène-imino en C₃ à C₆,
et la liaison s'effectue par un atome d'azote ou par un atome de carbone de la partie hétérocyclique ou d'un cycle phényle condensé,
et dans lequel, sauf indication contraire, l'expression "atome d'halogène" mentionnée précédemment dans les définitions désigne un atome du groupe comprenant le fluor, le chlore, le brome et l'iode,
et dans lequel, les groupes alkyle, alcényle, alcinyle et alkyloxy contenus dans les définitions mentionnés précédemment, qui présentent plus de deux atomes de carbone, peuvent être, sauf indication contraire, à chaîne linéaire ou ramifiés et les groupes alkyle dans les radicaux dialkylés mentionnés précédemment, par exemple les groupes dialkylamino, peuvent être identiques ou différents,
et dans lequel les atomes d'hydrogène des groupes méthyle ou éthyle contenus dans les définitions mentionnées précédemment, sauf indication contraire, peuvent être remplacés totalement ou partiellement par des atomes de fluor,
leurs tautomères, leurs énantiomères, leurs diastéréomères, leurs mélanges et leurs sels.

2. Composés de formule générale (I) selon la revendication 1, dans lesquels
D représente un système cyclique bicyclique substitué de formule (II) 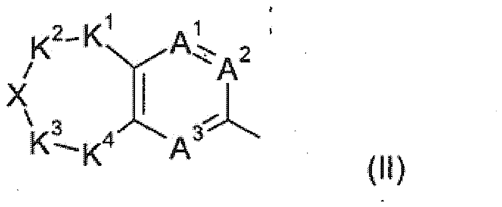 dans lequel
K¹ et K⁴ sont respectivement, indépendamment l'un de l'autre, un groupe -CH₂, -CHR^{7a}, -CR^{7b}R^{7c},-C(=CH2) ou -C(O), et dans lequel
R^{7a}/R^{7b}/R^{7c} sont respectivement, indépendamment les uns des autres, un atome de fluor, un groupe hydroxy, alkyloxy en C₁ à C₅, alkyle en C₁ à C₅,
dans lequel les deux radicaux R^{7b}/^{R7c} ne peuvent pas être liés en même temps par un hétéroatome à l'atome de carbone cyclique, sauf si -C(R^{7b}R^{7c}) correspond à un groupe-CF₂,
ou deux radicaux R^{7b}/R^{7c} conjointement avec l'atome de carbone cyclique peuvent former un cycle cyclopropyle,
K² et K³ sont respectivement, indépendamment l'un de l'autre, un groupe -CH₂, -CHR^{8a}, -CR^{8b}R^{8c} ou un groupe -C(O), dans lequel
R^{8a}/R^{8b}/R^{8c} sont respectivement, indépendamment les uns des autres, un groupe alkyle en C₁ à C₅ ou deux radicaux R^{8b}/R^{8c} peuvent former, conjointement avec l'atome de carbone cyclique, un cycle cyclopropyle,
et
au total, dans la formule (II) au maximum, quatre radicaux choisis parmi R^{7a}, R^{7b}, R^{7c}, R^{8a}, R^{8b} et R^{8c} peuvent être présents, et
X est un atome d'oxygène ou un atome de soufre, un groupe sulfène, sulfone ou NR¹, dans lequel
R¹ est un atome d'hydrogène ou un groupe hydroxy, alkyloxy en C₁ à C₃, amino, alkylamino en C₁ à C₃, di-(alkyle en C₁ à C₃)-amino, un groupe alkyle en C₁ à C₅, alcényle en C₂ à C₅-CH₂, alcinyle en C₂ à C₅-CH₂ ou cycloalkyle en C₃ à C₆,
et dans lequel
A¹ est soit N soit CR¹⁰,
A² est soit N, soit CR¹¹,
A³ est soit N, soit CR¹²,
dans lequel R¹⁰, R¹¹ et R¹² sont chacun indépendamment les uns des autres
un atome d'hydrogène, de fluor, de chlore, de brome ou d'iode ou un groupe alkyle en C₁ à C₅, CF₃, un groupe cyano, carboxy, alkyloxycarbonyle en C₁ à C₅, hydroxy, alkyloxy en C₁ à C₃, CF₃O, CHF₂O, CH₂FO, amino, alkylamino en C₁ à C₅, di-(alkyle en C₁ à C₅)-amino ou cycloalkylène-imino en C₄ à C₇.

3. Composés de formule générale (I) selon la revendication 1 ou 2, dans lesquels
X est un groupe NR¹, dans lequel
R¹ est un atome d'hydrogène ou un groupe alkyle en C₁ à C₅, allyle ou cyclopropyle, et
A¹ est CR¹⁰,
A² est CR¹¹,
A³ est CR¹²,
dans lequel R¹⁰, R¹¹ et R¹² sont chacun indépendamment les uns des autres
un atome d'hydrogène, de fluor ou de chlore ou un groupe méthyle, CF₃, hydroxy, méthoxy, CF₃O, CHF₂O, CH₂FO.

4. Composés de formule générale (I) selon l'une des revendications 1, 2 ou 3, dans lesquels
R⁴ est un atome d'hydrogène ou
un groupe alkyle en C₁ à C₆ à chaîne linéaire ou ramifié,
dans lequel les atomes d'hydrogène des fragments méthylène et/ou méthyle du groupe alkyle en C₁ à C₆ linéaire ou ramifié peuvent être remplacés totalement ou partiellement par des atomes de fluor, et/ou
dans lequel les atomes d'hydrogène des fragments méthylène et/ou méthyle du groupe alkyle en C₁ à C₆ à chaîne linéaire ou ramifié peuvent être substitués éventuellement, indépendamment les uns des autres, par un substituant choisi parmi un groupe hydroxy, alkyloxy en C₁ à C₅, carboxy, alkyloxycarbonyle en C₁ à C₅, aminocarbonyle, alkylaminocarbonyle en C₁ à C₅, di-(alkyle en C₁ à C₅)-aminocarbonyle, cycloalkylène-iminocarbonyle en C₄ à C₇, amino, alkylamino en C₁ à C₅, di-(alkyle en C₁ à C₅) amino, alkylcarbonylamino en C₁ à C₅, alkyle en C₁ à C₅-sulfonylamino, N-(alkylsulfonyle en C₁ à C₅)-alkylamino en C₁ à C₅, cycloalkylcarbonylamino en C₃ à C₆, ou
un groupe nitrile, carboxy, aminocarbonyle, alkylaminocarbonyle en C₁ à C₅, cycloalkylaminocarbonyle en C₃ à C₆, di-(alkyle en C₁ à C₅)-aminocarbonyle, alkyloxycarbonyle en C₁ à C₅ ou un groupe cycloalkylène-iminocarbonyle en C₄ à C₇ dans lequel éventuellement un groupe méthylène peut être remplacé par un atome d'oxygène, de soufre ou d'azote substitué par un groupe alkyle en C₀ à C₃, et
si -L-E-G-J est un groupe -C-C-C-C, R⁴ peut représenter sur E ou G, également un atome de fluor ou un groupe hydroxy, méthoxy, alcényle en C₂ à C₅-oxy, alcinyle en C₂ à C₅-oxy, alkyle en C₁ à C₅-oxy, cycloalkyle en C₃ à C₆-oxy, alkylaminocarbonyloxy en C₁ à C₅, di(alkyle en C₁ à C₅)aminocarbonyloxy ou cycloalkylène-iminocarbonyloxy en C₄ à C₇, phényl-alkyloxy en C₀ à C₃, amino, alkylamino en C₁ à C₅, di-(alkyle en C₁ à C₅)-amino, cycloalkylène-imino en C₄ à C₇, acylamino en C₁ à C₃, (acyle en C₁ à C₃)alkylamino en C₁ à C₃, alkyloxycarbonylamino en C₁ à C₅, alkylaminocarbonylamino en C₁ à C₅, di(alkyle en C₁ à C₅)aminocarbonylamino ou un groupe cycloalkylène-iminocarbonylamino en C₄ à C₇,
dans lequel les groupes méthyle ou méthylène présents dans les radicaux alkyle ou cycloalkyle mentionnés précédemment peuvent être substitués respectivement, indépendamment les uns des autres, par un substituant choisi dans le groupe diméthylamino-carbonyle, alkyloxycarbonyle en C₁ à C₃, carboxy, méthyle, hydroxy, méthoxy ou amino,
à condition qu'il soit exclu que deux hétéroatomes du groupe comprenant l'oxygène et l'azote soient séparés l'un de l'autre par exactement un groupe -CH₂ éventuellement substitué, et/ou
que deux atomes forment une liaison -O-O ou -S-O, et
R⁵ est un atome d'hydrogène ou un groupe allyle ou alkyle en C₁ à C₅, ou dans la mesure où R⁵ est relié à E ou G, peut également représenter un groupe hydroxy ou méthoxy, ou
R⁴ et R⁵, dans la mesure où ils sont liés au même atome de carbone,
peuvent former un groupe -C(O) ou un groupe -C(F₂), ou
R⁴ et R⁵, dans la mesure où ils sont liés au même atome de carbone ou à deux atomes de carbone voisins,
peuvent former un groupe cycloalkyle en C₃ à C₇
dans lequel l'un des groupes méthylène de ce groupe cycloalkyle en C₄ à C₇ peut être remplacé par un atome d'oxygène ou de soufre ou un groupe-NH, -N(alkyle en C₁ à C₅), -N(alkylcarbonyle en C₁ à C₄) ou carbonyle, sulfinyle ou sulfonyle, et/ou
dans lequel deux groupes méthylène directement voisins de ce groupe cycloalkyle en C₄ à C₇ peuvent être remplacés conjointement par un groupe -C(O)NH, C(O)N(alkyle en C₁ à C₅), -S(O)₂NH ou-S(O)₂N(alkyle en C₁ à C₅).

5. Composés de formule générale (I) selon l'une des revendications 1, 2, 3 ou 4, dans lesquels
-L-E-G-J- est un groupe -C-C-C-C-.

6. Composés de formule générale (I) selon l'une des revendications 1, 2, 3, 4 ou 5, dans lesquels
D représente un radical benzazépinyle substitué de formule (IIa) 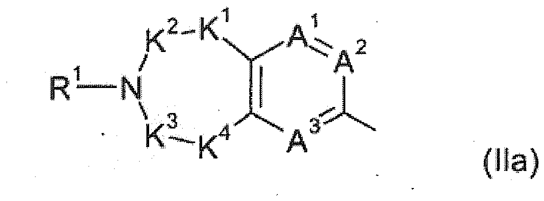
K¹ et K⁴ sont respectivement, indépendamment l'un de l'autre, un groupe -CH₂, -CHR^{7a}, -CR^{7b}R^{7c} ou-C(O), dans lequel
R^{7a} est un groupe alkyle en C₁ à C₅, hydroxy ou alkyloxy en C₁ à C₃, et
R^{7b}/R^{7c} sont respectivement, indépendamment les uns des autres, un groupe hydroxy, alkyloxy en C₁ à C₅ ou alkyl en C₁ à C₅, dans lequel les deux radicaux R^{7b}/^{R7c} ne peuvent pas être liés en même temps par un atome d'oxygène à l'atome de carbone cyclique,
ou deux radicaux R^{7b}/R^{7c} peuvent former, conjointement avec l'atome de carbone cyclique, un cycle cyclopropyle, et
K² et K³ sont respectivement, indépendamment l'un de l'autre, un groupe -CH₂, -CHR^{8a}, ou -CR^{8b}R^{8c}, dans lequel
R^{8a}/R^{8b}/R^{8c} sont respectivement, indépendamment les uns des autres, un groupe alkyle en C₁ à C₅,
et
au total dans la formule (II), au maximum quatre radicaux choisis parmi R^{7a}, R^{7b}, R^{7c}, R^{8a}, R^{8b} et R^{8c} peuvent être présents, et
R¹ est un atome d'hydrogène ou un groupe alkyle en C₁ à C₅, allyle ou cyclopropyle, et
A¹ est CR¹⁰,
A² est CR¹¹,
A³ est CR¹²,
dans lequel R¹⁰, R¹¹ et R¹² sont chacun indépendamment les uns des autres
un atome d'hydrogène, de fluor ou de chlore ou un groupe méthyle, CF₃, hydroxy, méthoxy, CF₃O, CHF₂O, CH₂FO, et
-L-E-G-J est un groupe -C-C-C-C, et
R³ est un atome d'hydrogène, et
R⁴ est un atome d'hydrogène, ou
est un groupe alkyle en C₁ à C₃, à chaîne linéaire ou ramifié,
dans lequel les atomes d'hydrogène des fragments méthylène et/ou méthyle du groupe alkyle en C₁ à C₆ à chaîne linéaire ou ramifié peuvent être substitués éventuellement, indépendamment les uns des autres, par un substituant choisi parmi un groupe hydroxy alkyloxy en C₁ à C₅, carboxy, alkyloxycarbonyle en C₁ à C₅, ou
un groupe CF₃, nitrile, carboxy, aminocarbonyle, alkylaminocarbonyle en C₁ à C₅, cycloalkyl-aminocarbonyle en C₃ à C₆, di-(alkyle en C₁ à C₅)-aminocarbonyle, alkyloxycarbonyle en C₁ à C₅ ou cycloalkylène-iminocarbonyle en C₄ à C₇, ou
si R⁴ est lié à E ou G, peut représenter également un atome de fluor ou un groupe hydroxy, méthoxy, alcényle en C₂ à C₅-oxy, alkyle en C₂ à C₅-oxy, méthoxyéthoxy, HOCH₂CH(OH)CH₂oxy, cycloalkyle en C₃ à C₆-oxy, alkylaminocarbonyloxy en C₁ à C₅, di(alkyle en C₁ à C₅)aminocarbonyloxy ou cycloalkylène-iminocarbonyloxy en C₄ à C₇,
R⁵ est un atome d'hydrogène ou un groupe alkyle en C₁ à C₅, ou dans la mesure où R⁵ est lié à E ou G, peut représenter également un groupe hydroxy ou méthoxy, ou
R⁴ et R⁵, dans la mesure où ils sont liés au même atome de carbone, peuvent être un groupe C=O ou -CF₂, et
R¹³ est un atome d'hydrogène,
M est un cycle phényle substitué 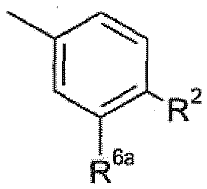 ou un cycle pyridyle substitué 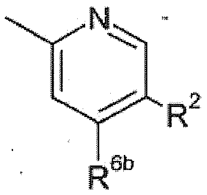 dans lequel
R² représente un atome de fluor, de chlore, de brome, un groupe méthoxy ou éthinyle, et
R^{6a} représente un atome d'hydrogène ou de fluor et
R^{6b} représente un atome d'hydrogène.

7. Sels physiologiquement acceptables des composés selon l'une des revendications 1 à 6.

8. Médicament, contenant un composé selon au moins l'une des revendications 1 à 6 ou un sel physiologiquement acceptable selon la revendication 7, outre éventuellement un ou plusieurs véhicules et/ou diluants inertes.

9. Utilisation d'un composé selon au moins l'une des revendications 1 à 6 ou d'un sel physiologiquement acceptable selon la revendication 7, pour la production d'un médicament présentant un effet inhibiteur sur le facteur Xa et/ou un effet inhibiteur sur des sérine-protéases apparentées.

10. Procédé de production d'un médicament selon la revendication 8, **caractérisé en ce que**, de façon non chimique, un composé selon au moins l'une des revendications 1 à 6 ou un sel physiologiquement acceptable selon la revendication 7 est intégré dans un ou plusieurs véhicules et/ou diluants inertes.
